# EUROPEAN PATENT SPECIFICATION

(11) **EP 1 042 293 B1**
(45) Date of publication and mention of the grant of the patent: **19.03.2008**
(21) Application number: 98965447.0
(22) Date of filing: 21.12.1998
(51) Int. Cl.: C07D 213/74, C07D 409/14, C07D 401/14, C07D 401/04, C07D 213/64, C07D 405/14, C07D 403/04, C07D 403/14, A61K 31/44, A61K 31/50

(54) **SUBSTITUTED PYRIDINE AND PYRIDAZINE COMPOUNDS AND THEIR PHARMACEUTICAL USE**
SUBSTITUIERTE PYRIDIN- UND PYRIDAZINDERIVATE UND IHRE PHARMAZEUTISCHE VERWENDUNG
COMPOSE DE PYRIDINE ET DE PYRIDAZINE SUBSTITUEES ET LEURS UTILISATIONS PHARMACEUTIQUES

(30) Priority: 19.12.1997 US 68199 P; 18.12.1998 US 215426
(43) Date of publication of application: 11.10.2000
(73) Proprietor: AMGEN INC., Thousand Oaks, CA 91320-1799 (US)
(72) Inventor: MANTLO, Nathan, B., Lafayette, CO 80026 (US); HWANG, Chan, Kou, Boulder, CO 80304 (US); SPOHR, Ulrike, D., Boulder, CO 80301 (US)
(74) Representative: Richardson, Kate
(86) International application number: PCT/US1998/027298
(87) International publication number: WO 1999/032448

(56) References cited:
- WO-A-92/02513
- WO-A-96/24584
- WO-A-97/05877
- WO-A-97/05878
- WO-A-98/03484
- GB-A- 1 238 959
- US-A- 5 461 053
- DATABASE WPI Section Ch, Week 8947 Derwent Publications Ltd., London, GB; Class B03, AN 89-345165 XP002101123 & JP 01 258671 A (MORISHITA PHARM CO LTD) , 16 October 1989
- CHEMICAL ABSTRACTS, vol. 108, no. 19, 9 May 1988 Columbus, Ohio, US; abstract no. 166870g, EICHINGER, KARL ET AL: "Spectroscopic studies of new uncommon oxidation-stable cation radicals of quaternary pyridinium compounds" page 591; XP002101120 -& CHEMICAL ABSTRACTS 12TH COLLECTIVE INDEX: FORMULA INDEX, page 18102F XP002101116 COLUMBUS US & SPECTROCHIM. ACTA, PART A (1987), 43A(6), 731-3 CODEN: SAMCAS;ISSN: 0584-8539,
- CHEMICAL ABSTRACTS, vol. 99, no. 7, 15 August 1983 Columbus, Ohio, US; abstract no. 53532a, ISMAIL, M. F. ET AL: "Synthesis of some indole derivatives of expected biological activity" page 538; XP002101121 -& CHEMICAL ABSTRACTS 11TH COLLECTIVE INDEX: FORMULA INDEX, page 16921F XP002101117 COLUMBUS US & EGYPT. J. CHEM. (1982), VOLUME DATE 1981, 24(4-6), 375-9 CODEN: EGJCA3;ISSN: 0367-0422,
- CHEMICAL ABSTRACTS, vol. 97, no. 7, 16 August 1982 Columbus, Ohio, US; abstract no. 55786u, BERGER, ULRICH ET AL: "Diels-Alder reactions with cyclic imines. III. Cycloaddition of imines to tetrazines" page 649; XP002101122 -& CHEMICAL ABSTRACTS 11TH COLLECTIVE INDEX: FORMULA INDEX, page 17994F XP002101118 COLUMBUS US & ARCH. PHARM. (WEINHEIM, GER.) (1982), 315(5), 428-37 CODEN: ARPMAS;ISSN: 0365-6233,
- CASE, FRANCIS H.: "The preparation of substituted pyridyltetrazines and pyridazines containing the ferroin group" J. HETEROCYCL. CHEM. (1968), 5(3), 431-2 CODEN: JHTCAD, XP002101119

## Description

### BACKGROUND OF THE INVENTION

The present invention comprises a new class of substituted pyridine and pyridazine compounds useful in treating diseases, such as TNF-α, IL-1β, IL-6 and/or IL-8 mediated diseases and other maladies, such as pain, cancer, and diabetes. In particular, the compounds of the invention are useful for the prophylaxis and treatment of diseases or conditions involving inflammation. This invention also relates to intermediates and processes useful in the preparation of such compounds.

Interleukin-1 (IL-1) and Tumor Necrosis Factor α (TNF-α) are pro-inflammatory cytokines secreted by a variety of cells, including monocytes and macrophages, in response to many inflammatory stimuli (e.g., lipopolysaccharide - LPS) or external cellular stress (*e.g.*, osmotic shock and peroxide).

Elevated levels of TNF-α and/or IL-1 over basal levels have been implicated in mediating or exacerbating a number of disease states including rheumatoid arthritis; Pagets disease; osteophorosis; multiple myeloma; uveititis; acute and chronic myelogenous leukemia; pancreatic ß cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; Reiter's syndrome; type I and type II diabetes; bone resorption diseases; graft vs. host reaction; ischemia reperfusion injury; atherosclerosis; brain trauma; multiple sclerosis; cerebral malaria; sepsis; septic shock; toxic shock syndrome; fever, and myalgias due to infection. HIV-1, HIV-2, HIV-3, cytomegalovirus (CMV), influenza, adenovirus, the herpes viruses (including HSV-1, HSV-2), and herpes zoster are also exacerbated by TNF-α

It has been reported that TNF-α plays a role in head trauma, stroke, and ischemia. For instance, in animal models of head trauma (rat), TNF-α levels increased in the contused hemisphere (Shohami et al., J. Cereb. Blood Flow Metab. 14, 615 (1994)). In a rat model of ischemia wherein the middle cerebral artery was occluded, the levels of TNF-α mRNA of TNF-α increased (Feurstein et al., Neurosci. Lett. 164, 125 (1993)). Administration of TNF-α into the rat cortex has been reported to result in significant neutrophil accumulation in capillaries and adherence in small blood vessels. TNF-α promotes the infiltration of other cytokines (IL-1β, IL-6) and also chemokines, which promote neutrophil infiltration into the infarct area (Feurstein, Stroke 25, 1481 (1994)). TNF-α has also been implicated to play a role in type II diabetes (Endocrinol. 130, 43-52, 1994; and Endocrinol. 136, 1474-1481, 1995).

TNF-α appears to play a role in promoting certain viral life cycles and disease states associated with them. For instance, TNF-α secreted by monocytes induced elevated levels of HIV expression in a chronically infected T cell clone (Clouse et al., J. Immunol. 142, 431 (1989)). Lahdevirta et al., (Am. J. Med. 85, 289 (1988)) discussed the role of TNF-α in the HIV associated states of cachexia and muscle degradation.

TNF-α is upstream in the cytokine cascade of inflammation. As a result, elevated levels of TNF-α may lead to elevated levels of other inflammatory and proinflammatory cytokines, such as IL-1, IL-6, and IL-8.

Elevated levels of IL-1 over basal levels have been implicated in mediating or exacerbating a number of disease states including rheumatoid arthritis; osteoarthritis; rheumatoid spondylitis; gouty arthritis; inflammatory bowel disease; adult respiratory distress syndrome (ARDS); psoriasis; Crohn's disease; ulcerative colitis; anaphylaxis; muscle degeneration; cachexia; Reiter's syndrome; type I and type II diabetes; bone resorption diseases; ischemia reperfusion injury; atherosclerosis; brain trauma; multiple sclerosis; sepsis; septic shock; and toxic shock syndrome. Viruses sensitive to TNF-α inhibition, e.g., HIV-1, HIV-2, HIV-3, are also affected by IL-1.

TNF-α and IL-1 appear to play a role in pancreatic β cell destruction and diabetes. Pancreatic β cells produce insulin which helps mediate blood glucose homeostasis. Deterioration of pancreatic β cells often accompanies type I diabetes. Pancreatic β cell functional abnormalities may occur in patients with type II diabetes. Type II diabetes is characterized by a functional resistance to insulin. Further, type II diabetes is also often accompanied by elevated levels of plasma glucagon and increased rates of hepatic glucose production. Glucagon is a regulatory hormone that attenuates liver gluconeogenesis inhibition by insulin. Glucagon receptors have been found in the liver, kidney and adipose tissue. Thus glucagon antagonists are useful for attenuating plasma glucose levels (WO 97/16442, incorporated herein by reference in its entirety). By antagonizing the glucagon receptors, it is thought that insulin responsiveness in the liver will improve, thereby decreasing gluconeogenesis and lowering the rate of hepatic glucose production.

In rheumatoid arthritis models in animals, multiple intra-articular injections of IL-1 have led to an acute and destructive form of arthritis (Chandrasekhar et al., Clinical Immunol Immunopathol. 55, 382 (1990)). In studies using cultured rheumatoid synovial cells, IL-1 is a more potent inducer of stromelysin than is TNF-α (Firestein, Am. J. Pathol. 140, 1309 (1992)). At sites of local injection, neutrophil, lymphocyte, and monocyte emigration has been observed. The emigration is attributed to the induction of chemokines (*e.g.,* IL-8), and the up-regulation of adhesion molecules (Dinarello, Eur. Cytokine Netw. 5, 517-531 (1994)).

IL-1 also appears to play a role in promoting certain viral life cycles. For example, cytokine-induced increase of HIV expression in a chronically infected macrophage line has been associated with a concomitant and selective increase in IL-1 production (Folks et al., J. Immunol. 136, 40 (1986)). Beutler et al. (J. Immunol. 135, 3969 (1985)) discussed the role of IL-1 in cachexia. Baracos et al. (New Eng. J. Med. 308, 553 (1983)) discussed the role of IL-1 in muscle degeneration.

In rheumatoid arthritis, both IL-1 and TNF-α induce synoviocytes and chondrocytes to produce collagenase and neutral proteases, which leads to tissue destruction within the arthritic joints. In a model of arthritis (collagen-induced arthritis (CIA) in rats and mice), intra-articular administration of TNF-α either prior to or after the induction of CIA led to an accelerated onset of arthritis and a more severe course of the disease (Brahn et al., Lymphokine Cytokine Res. 11, 253 (1992); and Cooper, Clin. Exp. Immunol. 898, 244 (1992)).

IL-8 has been implicated in exacerbating and/or causing many disease states in which massive neutrophil infiltration into sites of inflammation or injury (e.g., ischemia) is mediated by the chemotactic nature of IL-8, including, but not limited to, the following: asthma, inflammatory bowel disease, psoriasis, adult respiratory distress syndrome, cardiac and renal reperfusion injury, thrombosis and glomerulonephritis. In addition to the chemotaxis effect on neutrophils, IL-8 also has the ability to activate neutrophils. Thus, reduction in IL-8 levels may lead to diminished neutrophil infiltration.

Several approaches have been taken to block the effect of TNF-α. One approach involves using soluble receptors for TNF-α (e.g., TNFR-55 or TNFR-75), which have demonstrated efficacy in animal models of TNF-α-mediated disease states. A second approach to neutralizing TNF-α using a monoclonal antibody specific to TNF-α, cA2, has demonstrated improvement in swollen joint count in a Phase II human trial of rheumatoid arthritis (Feldmann et al., Immunological Reviews, pp. 195-223 (1995)). These approaches block the effects of TNF-α and IL-1 by either protein sequestration or receptor antagonism.

GB 2,306,108, which is incorporated herein by reference in its entirety, describes imidazole derivatives which are Raf kinase antagonists useful in the treatment of cancer which is mediated by Raf and Raf-inducable proteins. Raf proteins are kinases activated in response to extracellular mitogenic stimuli such as PDGF, EGF, acidic FGF, thrombin, insulin or endothelin, and also in response to oncoproteins such as v-src, v-sis, and v-fms. Raf functions downstream of ras in signal transduction from the cellular membrane to the nucleus. Compounds may be oncolytics through the antagonism of Raf kinase. It has been reported that antisense constructs which reduce cellular levels of c-Raf and hence Raf activity inhibit the growth of rodent fibroblasts in soft agar, while exhibiting little or no general cytotoxicity. This inhibition of growth in soft agar is highly predictive of tumor responsiveness in whole animals. Moreover, Raf antisense constructs have shown efficacy in reducing tumor burden in animals. Examples of cancers where Raf kinase is implicated by overexpression include cancers of the brain, larynx, lung, lymphatic system, urinary tract and stomach, including hystocytic lymphoma, lung adenocarcinoma and small cell lung cancers. Other examples include cancers involving overexpression of upstream activators of Raf or Raf-activating oncogenes, including pancreatic and breast carcinoma.

GB 1,238,959 describes 3- or 4-(hetero)aryl substituted pyridine and pyridone compounds useful in the treatment of inflammation.

WO 98/03484 describes 2-(substituted phenyl or pyridinyl)-3-(4-(methylsulfonyl, aminosulfonyl, trifluorocarbonylaminosulfonyl or methylaminosulfonyl) phenyl-pyridine compounds useful in the treatment of COX-2 mediated diseases.

WO 96/24584 describes 2,3-di(hetero)aryl substituted pyridine compounds, wherein one of such (hetero)aryl substitutents is a phenyl radical substituted with an alkylsulfonyl, aminosulfonyl or haloalkylsulfonyl radical, useful as anti-inflammatory, analgesic and antipyretic agents.

US 5,461,053 discloses (aryl)(amino)pyridazines as ligands of cholinergic receptors.

WO 92/02513 discloses heterocyclyl-diaryl pyrazines and heterocyclyl-diaryl pyrimidines having anti-thrombotic, vasodilating and anti-inflammatory activities.

WO 97/05877 discloses 2-aryl-3-heterocyclyl pyrroles for the treatment of cytokine mediated diseases.

WO 97/05878 discloses 2,5-diaryl-3-heterocyclyl pyrroles for the treatment of diseases characterized by excessive IL-8 activity.

JP 870304986 discloses imidazolyl-pyridazinones having anti-thrombotic and anti-fungal activity.

Spectrochim.Acta, Part A 1987, 43A(6), 731-3 discloses spectroscopic studies on terpyridimium and quaterpyridimium salts.

Egypt.J.Chain.1981, 24 (4-6), 375-9 discloses the synthesis of pyridazone substituted indoles.

Arch.pharm. 1982, 315(5), 428-37 discloses the cycloaddition of imines to tetrazines to give pyridazines and other heterocycles.

J. Heterocycl. Chem. 1968, 5(3), 431-2 discloses the synthesis of substituted pyridyl tetrazines and pyridazines as metal ion chelators.

### BRIEF DESCRIPTION OF THE INVENTION

The present invention comprises a new class of compounds useful in the prophylaxis and treatment of diseases, such as TNF-α, IL-1β, IL-6 and/or IL-8 mediated diseases and other maladies, such as pain, cancer and diabetes. In particular, the compounds of the invention are useful for the prophylaxis and treatment of diseases or conditions involving inflammation. Accordingly, the invention also comprises pharmaceutical compositions comprising the compounds, methods for the prophylaxis and treatment of TNF-α, IL-1β, IL-6 and/or IL-8 mediated diseases, such as inflammatory, pain and diabetes diseases, using the compounds and compositions of the invention, and intermediates and processes useful for the preparation of the compounds of the invention.

The compounds of the invention are represented by the following general structure: 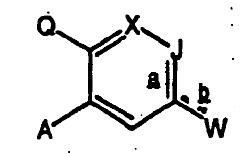 wherein A, Q, X, J, W, a, and b are defined below.

The foregoing merely summarizes certain aspects of the invention and is not intended, nor should it be construed, as limiting the invention in any way. All patents and other publications recited herein are hereby incorporated by reference in their entirety.

### DETAILED DESCRIPTION OF THE INVENTION

According to the present invention, there is provided a compound of formula. 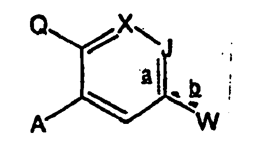 or pharmaceutically acceptable salt thereof, wherein
W is R₁, R₂ or O
A is R₁₁ and Q is R₁₂ or vice versa;
X is N or C-H;
J is N-R₃, N, C-R₁ or C-R₂, provided at least one of X or J is N or N-R₃; and
when W is R₁, then a is a double bond, b is a single bond and J is other than N-R₃ or C-R₁; when W is R₂, a is a double bond, b is a single bond and J is other than N-R₃ or C-R₂; and when W is O, then a is a single bond, b is a double bond and J is N-R₃;
R₁ is -Z-Y or -Y; and each R₃ is independently a hydrogen radical or -Z-Y; provided that the total number of aryl, heteroaryl, cycloalkyl and heterocyclyl radicals in R₁, R₂ and R₃ is 0-2;
R₂ is (1) a hydrogen, halo, trifluoromethyl or cyano radical; or
(2) C₁-C₄ alkyl radical optionally substituted by (a) 1-2 radicals of amino, C₁-C₄ alkylamino or di-(C₁-C₄ alkyl)amino;
each Z is independently a
(1) C₁-C₄ alkyl radical optionally substituted by (a) 1-2 radicals of amino, di-(C₁-C₂ alkyl)amino, hydroxy, C₁-C₂ alkoxy or C₁-C₂ alkylthio, and (b) an aryl radical; or
(2) a heterocyclyl radical optionally substituted by 1-2 radicals of C₁-C₂ alkyl or aryl-C₁-C₂ alkyl radicals; wherein the aryl radicals are optionally substituted by 1-2 radicals of amino, di-(C₁-C₂ alkyl)amino, hydroxy, C₁-C₂ alkoxy, C₁-C₂ alkylthio, cyano, halo, C₁-C₂ alkyl or trifluoromethyl radicals;
each Y is independently a
(1) hydrogen radical;
(2) -C(O)-R₂₀ or -C(O)-NR₅R₂₁ radical;
(3) -OR₂₁, -SR₂₁, -S(O)-R₂₀, -S(O)₂-R₂₀ or -S(O)₂-NR₅R₂₁ radical; or
(4) -NR₅R₂₁ or -NR₂₂-C(O)-R₂₁ radical;
each R₅ is independently
(1) hydrogen radical;
(2) C₁-C₄ alkyl radical optionally substituted by 1-3 radicals of amino, di-(C₁-C₂-alkyl)amino, hydroxy, C₁-C₂ alkoxy, C₁-C₂ alkylthio or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₂-alkyl)amino, hydroxy, C₁-C₂ alkoxy, C₁-C₂ alkylthio, methoxy, methylthio, C₁-C₄ alkyl or trifluoromethyl radicals;
each R₂₀ is independently
(1) C₁-C₈ alkyl radicals optionally substituted by 1-3 radicals of amino, C₁-C₄ alkylamino, di-(C₁-C₄ alkyl)amino, C₁-C₅ alkanoylamino, (C₁-C₄ alkoxy) carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or C₃-C₆ cycloalkyl, heterocyclyl, aryl or heteroaryl radicals optionally substituted by 1-2 radicals of amino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of hydroxy, C₁-C₄ alkoxy, C₁-C₄ alkylthio or C₁-C₄ alkyl'; or
(3) aryl or heteroaryl radicals optionally substituted by 1-2 radicals of (C₁-C₄ alkoxy)carbonyl, amino, C₁-C₄ alkylamino, di-(C₁-C₄ alkyl)amino, hydroxy, C₁-C₄ alkoxy, C₁-C₄ alkylthio, cyano, halo, azido, C₁-C₄ alkyl or trifluoromethyl radicals;
each R₂₁ is independently hydrogen radical or R₂₀;
each R₂₂ is independently hydrogen or C₁-C₄ alkyl radical;
R₁₁ is an aryl or heteroaryl radical other than an "N"-heteroaryl radical, and R₁₂ is a "N"-heteroaryl radical, wherein the aryl, heteroayyl and "N"-heteroaryl radicals are optionally substituted by 1-2 radicals of
(1) R₃₀;
(2) halo or cyano radicals; or
(3) -C(O)-NR₃₁R₃₂, -OR₂₉, -SR₂₉, -S(O)-R₃₀, -S(O)₂-R₃₀, S(O)₂-NR₃₁R₃₂, -NR₃₁R₃₂ or -NR₃₃-C(O)-R₂₉ radicals;
each R₃₀ is independently
(1) C₁-C₄ alkyl radical optionally substituted by a phenyl or heteroaryl radical optionally substituted by 1-3 radicals of amino, dimethylamino, acetamido, hydroxy, halo, methoxy, methyl or trifluoromethyl radicals;
(2) trifluoromethyl' radical; or
(3) aryl or heteroaryl radicals optionally substituted by 1-3 radicals of amino, dimethylamino, acetamido, hydroxy, halo, methoxy, methyl or trifluoromethyl, radicals;
each R₂₉ is indepdently hydrogen radical or R₃₀; and
each R₃₁ is independently
(1) hydrogen radicals; or
(2) C₁-C₄ alkyl radical optionally substituted by an phenyl or heteroaryl radical optionally substituted by 1-3 radicals of amino, C₁-C₄ alkylamino, di-(C₁-C₄ alkyl) amino, C₁-C₅ alkanoylamino, (C₁-C₄ alkoxy), carbonyl amino, hydroxy, C₁-C₄ alkoxy, C₁-C₄ alkylthio, cyano, C₁-C₄ alkyl or trifluoromethyl radicals;
wherein heterocyclyl is a radical of a monocyclic saturated heterocyclic ring system having 5-6 ring members, wherein 1-3 ring members are oxygen, sulfur or nitrogen heteroatoms, which is optionally benzo-fused and optionally substituted by 1-2 oxo or thioxo radicals; aryl is a phenyl or naphthyl radical; and heteroaryl is radical of a monocyclic aromatic heterocyclic ring system having 5-6 ring members, wherein 1-3 ring members are oxygen, sulfur or nitrogen heteroatoms, which is optionally benzo-fused or saturated C₃-C₄-carbocyclic-fused and
each R₃₂ is independently
(1) hydrogen is independently
(2) C₁-C₄ alkyl radical or C₁-C₂ alkyl radical substituted by phenyl or heteroaryl radical optionally substituted by 1-3 radicals of amino, dimethylamino, acetamido, hydroxy, methoxy; methyl or trifluoromethyl radicals; or
(3) phenyl or heteroaryl radical optionally substituted by 1-3 radicals of amino, dimethylamino, acetamido, hydroxy, methoxy, methyl or trifluoromethyl radicals; and each R₃₃ is independently hydrogen or C₁-C₄ alkyl radical; provided that when X is C-H, then Q is other than a phenyl radical; and when X is N and J is C-H, A is other than a 4-(methylsulfonyl) phenyl, 4-(aminosulfonyl)- phenyl, 4-(trifluoromethylcarbonylaminosulfonyl) phenyl or 4-(methylaminosulfonyl) phenyl radical with the further proviso that the compound is not selected from the group of compounds of general formula (I) 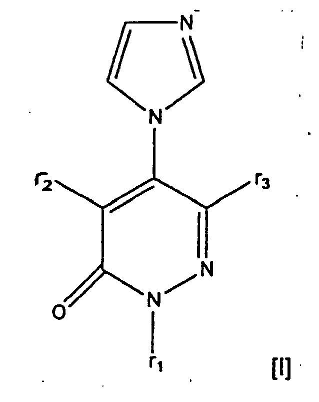
where r₁ is a hydrogen atom, methyl group, ethyl group, proply group or butyl group, r₂ is a hydrogen atom and r₃ is an optionally-halogen-substituted phenyl group selected from a 4-chlorophenyl group and 2, 4-dichlorophenyl group

There is disclosed compounds of formula 1: 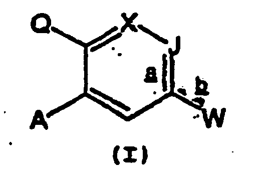 or a pharmaceutically acceptable salt thereof, wherein
W is R₁, R₂, O or N-R3;
A and Q are each independently R₁₁ or R₁₂;
X is N or C-H;
J is N-R₃, N, C-R₁ or C-R₂, provided at least one of X or J is N or N-R₃; and
when W is R₁, then a is a double bond, b is a single bond and J is other than N-R₃ or C-R₁; when W is R₂, then a is a double bond, b is a single bond and J is other than N-R₃ or C-R₂; and when W is 0 or N-R₃, then a is a single bond, b is a double bond and J is N-R₃;

Preferably, W is R₁, R₂, O or N-R₃;
A is R₁₁ and Q is R₁₂, or A is R₁₂ and Q is R₁₁;
X is N or C-H;
J is N-R₃, N, C-R₁ or C-R₂, provided at least one of X or J is N or N-R₃; and
when W is R₁, then a is a double bond, b is a single bond and J is other than N-R₃ or C-R₁; when W is R₂,
then a is a double bond, b is a single bond and J is other than N-R₃ or C-R₂; and when W is 0 or N-R₃, then a is a single bond, b is a double bond and J is N-R₃;

More preferably, W is R₁, R₂ or O;
A is R₁₁ and Q is R₁₂, or A is R₁₂ and Q is R₁₁;
X is N or C-H;
J is N-R₃, N, C-R₁ or C-R₂, provided at least one of X or J is N or N-R₃; and
when W is R₁, then a is a double bond, b is a single bond and J is other than N-R₃ or C-R₁; when W is R₂,
then a is a double bond, b is a single bond and J is other than N-R₃ or C-R₂; and when W is 0 or N-R₃, then a is a single bond, b is a double bond and J is N-R₃;

More preferably, W is R₁ or R₂;
A is R₁₁ and Q is R₁₂, or A is R₁₂ and Q is R₁₁;
X is N or C-H;
J is N, C-R₁ or C-R₂, provided at least one of X or J is N;
a is a double bond and b is a single bond; and
when W is R₁, then J is other than C-R₁; when W is R₂, then C-R₂;

Most preferably, W is R₁;
A is R₁₂ and Q is R₁₁;
X is N and J is C-R₂, or X is C-H and J is N, or X and J are both N; and
a is a double bond and b is a single bond; or
alternatively, W is R₂;
A is R₁₁ and Q is R₁₂;
X is N and J is C-R₁; and
a is a double bond and b is a single bond;

Alternatively more preferably, W is 0;
A is R₁₁ and Q is R₁₂, or A is R₁₂ and Q is R₁₁;
X is N or C-H;
J is N-R₃; and
a is a single bond and b is a double bond;

More preferably, W is O;
A is R₁₁ and Q is R₁₂;
X is N or C-H;
J is N-R₃; and
a is a single bond and b is a double bond;

Most preferably, W is O;
A is R₁₁ and Q is R₁₂;
X is C-H;
J is N-R₃; and
a is a single bond and b is a double bond;
R₁ is -Z-Y or -Y; and each R₃ is independently a hydrogen radical or -Z-Y; provided that the total number of aryl, heteroaryl, cycloalkyl and heterocyclyl radicals in R₁, R₂ and R₃ is 0-3; and preferably, 0-2;
R₂ is (1) a hydrogen, halo, trifluoromethyl, cyano, -C(O)-OR₂₁ or -C(O)-NR₅R₂₁ radical;
(2) alkyl radical optionally substituted by (a) 1-2 radicals of amino, alkylamino, dialkylamino, alkanoylamino, alkoxycarbonylamino, alkylsulfonylamino, hydroxy, alkoxy or alkylthio, and (b) a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, halo, alkyl, carboxy, carboxamide, trifluoromethoxy or trifluoromethyl radicals; or
(3) aryl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl, carboxy, carboxamide, trifluoromethoxy or trifluoromethyl radicals;
preferably, R₂ is (1) a hydrogen, halo, trifluoromethyl, cyano, -C(O)-OR₂₁ or -C(O)-NR₅R₂₁ radical;
(2) C₁-C₈ alkyl radical optionally substituted by (a) 1-2 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy or C₁-C₄ alkylthio, and (b) a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, carboxy, carboxamide, trifluoromethoxy or trifluoromethyl radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, carboxy, carboxamide, trifluoromethoxy or trifluoromethyl radicals;
more preferably, R₂ is (1) a hydrogen, halo, trifluoromethyl, cyano, carboxy or carboxamide radical;
(2) C₁-C₈ alkyl radical optionally substituted by (a) 1-2 radicals of amino, C₁-C₄ alkylamino, di-(C₁-C₄ alkyl)amino, hydroxy, C₁-C₄ alkoxy or C₁-C₄ alkylthio; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, carboxy, carboxamide, trifluoromethoxy or trifluoromethyl radicals;
more preferably, R₂ is (1) a hydrogen, halo, trifluoromethyl or cyano radical; or
(2) C₁-C₄ alkyl radical optionally substituted by (a) 1-2 radicals of amino, C₁-C₄ alkylamino or di-(C₁-C₄ alkyl)amino; or
most preferably, R₂ is a hydrogen, halo, trifluoromethyl, cyano or C₁-C₄ alkyl radical;
Z is independently a
(1) alkyl, alkenyl or alkynyl radical optionally substituted by (a) 1-3 radicals of amino, alkylamino, dialkylamino, alkanoylamino, alkoxycarbonylamino, alkylsulfonylamino, hydroxy, alkoxy, alkylthio or halo, and (b) 1-2 radicals of heterocyclyl, aryl or heteroaryl; or
(2) heterocyclyl, aryl or heteroaryl radical; wherein the heterocyclyl radicals are optionally substituted by 1-3 radicals of amino, alkylamino, dialkylamino, alkanoylamino, alkoxycarbonylamino, alkylsulfonylamino, hydroxy, alkoxy, alkylthio, alkyl, arylalkyl, heteroarylalkyl or haloalkyl; and the aryl and heteroaryl radicals are optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
preferably, each Z is independently a
(1) C₁-C₈ alkyl, C₂-C₈ alkenyl or C₂-C₈ alkynyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl; or
(2) heterocyclyl, aryl or heteroaryl radical;
wherein the heterocyclyl radicals are optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl, aryl-C₁-C₄ alkyl, heteroaryl-C₁-C₄ alkyl or C₁-C₄ haloalkyl of 1-3 halo radicals; and the aryl and heteroaryl radicals are optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
more preferably, each Z is independently a
(1) C₁-C₈ alkyl or C₂-C₈ alkenyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or halo, and (b) 1-2 radicals of heterocyclyl, aryl or heteroaryl; or
(2) heterocyclyl, aryl or heteroaryl radical;
wherein the heterocyclyl radicals are optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, C₁-C₄ alkyl, aryl-C₁-C₄ alkyl, heteroaryl-C₁-C₄ alkyl or C₁-C₂ haloalkyl of 1-3 halo radicals; and the aryl and heteroaryl radicals are optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₂ haloalkyl of 1-3 halo radicals;
more preferably, each Z is independently a
(1) C₁-C₄ alkyl or C₂-C₅ alkenyl radical optionally substituted by (a) 1-3 radicals of amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio or halo, and (b) 1-2 radicals of heterocyclyl, aryl or heteroaryl; or
(2) heterocyclyl, aryl or heteroaryl radical;
wherein the heterocyclyl radicals are optionally substituted by 1-3 radicals of amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, C₁-C₄ alkyl, aryl-C₁-C₄ alkyl, heteroaryl-C₁-C₄ alkyl or trifluoromethyl radicals; and the aryl and heteroaryl radicals are optionally substituted by 1-3 radicals of amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
more preferably, each Z is independently a
(1) C₁-C₄ alkyl or C₂-C₅ alkenyl radical optionally substituted by (a) 1-3 radicals of amino, di-(C₁-C₂ alkyl)amino, (C₁-C₄ alkoxy)carbonylamino, hydroxy, C₁-C₂ alkoxy, C₁-C₂ alkylthio or halo, and (b) 1-2 radicals of aryl or heteroaryl; or
(2) heterocyclyl, aryl or heteroaryl radical;
wherein the heterocyclyl radicals are optionally substituted by 1-2 radicals of C₁-C₄ alkyl or aryl-C₁-C₂ alkyl radicals; and the aryl and heteroaryl radicals are optionally substituted by 1-3 radicals of amino, di-(C₁-C₂ alkyl)amino, acetamido, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
more preferably, each z is independently a
(1) C₁-C₄ alkyl radical optionally substituted by (a) 1-2 radicals of amino, di-(C₁-C₂ alkyl)amino, hydroxy, C₁-C₂ alkoxy or C₁-C₂ alkylthio, and (b) an aryl radical; or
(2) a heterocyclyl radical optionally substituted by 1-2 radicals of C₁-C₂ alkyl or aryl-C₁-C₂ alkyl radicals;
wherein the aryl radicals are optionally substituted by 1-2 radicals of amino, di-(C₁-C₂ alkyl)amino, hydroxy, C₁-C₂ alkoxy, C₁-C₂ alkylthio, cyano, halo, C₁-C₂ alkyl or trifluoromethyl radicals; and
most preferably, each Z is independently a
(1) C₁-C₄ alkyl radical optionally substituted by 1-2 radicals of amino, dimethylamino or phenyl radical; or
(2) a heterocyclyl radical optionally substituted by 1-2 radicals of methyl or phenylmethyl;
wherein the phenyl radicals are optionally substituted by 1-2 radicals of amino, di-(C₁-C₂ alkyl)amino, hydroxy, C₁-C₂ alkoxy, C₁-C₂ alkylthio, cyano, halo, C₁-C2 alkyl or trifluoromethyl radicals;
each Y is independently a
(1) hydrogen radical;
(2) halo or nitro radical;
(3) -C(O)-R₂₀, -C(O)-OR₂₁, -C(O)-NR₅R₂₁ or -C(NR₅)-NR₅R₂₁ radical;
(4) -OR₂₁, -O-C(O)-R₂₁, -O-C(O)-NR₅R₂₁ or -O-C(O)-NR₂₂-S(O)₂-R₂₀ radical;
(5) -SR₂₁, -S(O)-R₂₀, -S(O)₂-R₂₀, -S(O)₂-NR₅R₂₁, -S(O)₂-NR₂₂-C(O)-R₂₁, -S(O)₂-NR₂₂-C(O)-OR₂₀ or -S(O)₂-NR₂₂-C(O)-NR₅R₂₁ radical; or
(6) -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ or -NR₂₂-S(O)₂-NR₅R₂₁ radical;
preferably, each Y is independently a
(1) hydrogen or halo radical;
(2) -C(O)-R₂₀, -C(O)-OR₂₁, -C(O)-NR₅R₂₁ or -C(NR₅)-NR₅R₂₁ radical;
(3) -OR₂₁, -O-C(O)-R₂₁ or -O-C(O)-NR₅R₂₁ radical;
(4) -SR₂₁, -S(O)-R₂₀, -S(O)₂-R₂₀ or -S(O)₂-NR₅R₂₁ radical; or
(5) -NR₅R₂₁, -NR₂₂-C(O)-R₂₁, -NR₂₂-C(O)-OR₂₀ or -NR₂₂-C(O)-NR₅R₂₁ radical;
more preferably, each Y is independently a
(1) hydrogen radical;
(2) -C(O)-R₂₀ or -C(O)-NR₅R₂₁ radical;
(3) -OR₂₁, -SR₂₁, -S(O)-R₂₀, -S(O)₂-R₂₀ or -S(O)₂-NR₅R₂₁ radical; or
(4) -NR₅R₂₁ or -NR₂₂-C(O)-R₂₁ radical;
more preferably, each Y is independently a hydrogen, -OR₂₁, -SR₂₁, -S(O)-R₂₀, -S(O)₂-R₂₀ or -NR₅R₂₁ radical;
most preferably, each Y is independently a -OR₂₁, -SR₂₁ or -NR₅R₂₁ radical;
wherein each R₅ is independently
(1) hydrogen radicals;
(2) alkyl, alkenyl or alkynyl radicals optionally substituted by 1-3 radicals of amino, alkylamino, dialkylamino, hydroxy, alkoxy, alkylthio, -SO₃H or halo; or
(3) aryl, heteroaryl, aralkyl, heteroaralkyl, heterocyclyl, heterocyclylalkyl, cycloalkyl or cycloalkylalkyl radicals optionally substituted by 1-3 radicals of amino, alkylamino, dialkylamino, hydroxy, alkoxy, alkylthio, alkyl or haloalkyl;
preferably, each R₅ is independently
(1) hydrogen radicals;
(2) C₁-C₈ alkyl, C₂-C₈ alkenyl or C₂-C₈ alkynyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, -SO,H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
more preferably, each R₅ is independently
(1) hydrogen radicals;
(2) C₁-C₄ alkyl, C₂-C₅ alkenyl or C₂-C₅ alkynyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, -SO,H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
more preferably, each R₅ is independently
(1) hydrogen radicals;
(2) C₁-C₄ alkyl or C₂-C₅ alkenyl radicals optionally substituted by 1-3 radicals of amino, di-(C₁-C₄-alkyl)amino, hydroxy, C₁-C₄ alkoxy, C₁-C₄ alkylthio, -SO₃H or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₄-alkyl)amino, hydroxy, C₁-C₄ alkoxy, C₁-C₄ alkylthio, C₁-C₄ alkyl or C₁-C₂ haloalkyl of 1-3 halo radicals;
more preferably, each R₅ is independently
(1) hydrogen radical;
(2) C₁-C₄ alkyl radical optionally substituted by 1-3 radicals of amino, di-(C₁-C₂-alkyl)amino, hydroxy, C₁-C₂ alkoxy, C₁-C₂ alkylthio or halo; or
(3) phenyl-C₁-C₂-alkyl, heteroaryl-C₁-C₂-alkyl, heterocyclyl-C₁-C₂-alkyl or C₃-C₅-cycloalkyl-C₁-C₂-alkyl radicals optionally substituted by 1-3 radicals of amino, di-(C₁-C₂-alkyl)amino, hydroxy, C₁-C₂ alkoxy, C₁-C₂ alkylthio, methoxy, methylthio, C₁-C₄ alkyl or trifluoromethyl radicals;
more preferably, each R₅ is independently
(1) hydrogen radical;
(2) C₁-C₄ alkyl radical optionally substituted by 1-3 halo radicals; or
(3) phenyl-C₁-C₂-alkyl or heteroaryl-C₁-C₂-alkyl, radicals optionally substituted by 1-3 radicals of amino, dimethylamino, hydroxy, methoxy, methylthio, methyl or trifluoromethyl radicals;
more preferably, each R₅ is independently hydrogen or C₁-C₄ alkyl radical; and most preferably, each R₅ is a hydrogen or methyl radical;
wherein each R₂₀ is independently
(1) alkyl, alkenyl or alkynyl radicals optionally substituted by 1-3 radicals of amino, alkylamino, dialkylamino, alkanoylamino, alkoxycarbonylamino, N-(alkoxycarbonyl)-N-(alkyl)amino, aminocarbonylamino, alkylsulfonylamino, hydroxy, alkoxy, alkylthio, alkylsulfinyl, alkylsulfonyl, halo or aralkoxy, aralkylthio, aralkylsulfonyl, cycloalkyl, heterocyclyl, aryl or heteroaryl radicals optionally substituted by 1-3 radicals of amino, alkylamino, dialkylamino, alkanoylamino, alkoxycarbonylamino, alkylsulfonylamino, alkanoyl, hydroxy, alkoxy, alkylthio, alkylsulfinyl, alkylsulfonyl, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, alkoxycarbonyl, hydroxy, alkoxy, alkylthio, cyano, halo, azido, alkyl or haloalkyl;
preferably, each R₂₀ is independently
(1) C₁-C₈ alkyl, C₂-C₈ alkenyl or C₂-C₈ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl) amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
more preferably, each R₂₀ is independently
(1) C₁-C₈ alkyl, C₂-C₅ alkenyl or C₂-C₅ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
more preferably, each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy) carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₂ haloalkyl of 1-3 halo radicals;
more preferably, each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, di-(C₁-C₄ alkyl)amino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-2 radicals of amino, C₁-C₄ alkylamino, di-(C₁-C₄ alkyl)amino, acetamido, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or trifluoromethyl radicals;
more preferably, each R₂₀ is independently
(1) C₁-C₈ alkyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl) amino, aminocarbonylamino, hydroxy, C₁-C₄ alkoxy, C₁-C₄ alkylthio, C₁-C₄ alkylsulfinyl, C₁-C₄ alkylsulfonyl, halo or C₃-C₆ cycloalkyl, heterocyclyl, aryl or heteroaryl radicals optionally substituted by 1-2 radicals of amino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of hydroxy, C₁-C₄ alkoxy, C₁-C₄ alkylthio or C₁-C₄ alkyl; or
(3) aryl or heteroaryl radicals optionally substituted by 1-2 radicals of (C₁-C₄ alkoxy)carbonyl, amino, C₁-C₄ alkylamino, di-(C₁-C₄ alkyl)amino, hydroxy, C₁-C₄ alkoxy, C₁-C₄ alkylthio, cyano, halo, azido, C₁-C₄ alkyl or trifluoromethyl radicals;
more preferably, each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical optionally substituted by 1-2 radicals of hydroxy or C₁-C₄ alkyl; or
(3) aryl or heteroaryl radicals optionally substituted by 1-2 radicals of amino, dimethylamino, hydroxy, methoxy, methylthio, halo, methyl or trifluoromethyl radicals;
more preferably, each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl) amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
most preferably, each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
each R₂₂ is independently
(1) hydrogen radical;
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl; or
(3) heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
preferably, each R₂₂ is independently
(1) hydrogen radical;
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals; or
(3) heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
more preferably, each R₂₂ is independently
(1) hydrogen radical; or
(2) C₁-C₄ alkyl radical optionally substituted by a radical of phenyl or heteroaryl optionally substituted by 1-3 radicals of amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₂ haloalkyl of 1-3 halo radicals;
more preferably, each R₂₂ is independently hydrogen or C₁-C₄ alkyl radical; and most preferably, each R₂₂ is independently hydrogen or methyl radical;
R₁₁ is an aryl or heteroaryl radical other than an "N"-heteroaryl radical, and R₁₂ is an "N"-heteroaryl radical,
wherein the aryl, heteroaryl and "N"-heteroaryl radicals are optionally substituted by 1-3 radicals of
(1) R₃₀;
(2) halo or cyano radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁P₃₂, -NP₃₃-S(O)₂-R₃₀ or -NR₃₃-S(O)₂-NR₃₁R₃₂ radicals;
provided that the total number of aryl, heteroaryl, cycloalkyl and heterocyclyl radicals substituted on each of R₁₁ and R₁₂ is 0-1;
preferably, R₁₁ is an aryl or heteroaryl radical other than an "N"-heteroaryl radical, and R₁₂ is a "N"-heteroaryl radical, wherein the aryl, heteroaryl and "N"-heteroaryl radicals are optionally substituted by 1-2 radicals of
(1) R₃₀;
(2) halo or cyano radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ or -NR₃₃-S(O)₂-NR₃₁R₃₂ radicals;
provided that the total number of aryl, heteroaryl, cycloalkyl and heterocyclyl radicals substituted on each of R₁₁ and R₁₂ is 0-1;
more preferably, R₁₁ is an aryl or heteroaryl radical other than an "N"-heteroaryl radical, and R₁₂ is a "N"-heteroaryl radical, wherein the aryl, heteroayyl and "N"-heteroaryl radicals are optionally substituted by 1-2 radicals of
(1) R₃₀;
(2) halo or cyano radicals;
(3) -C(O)-R₃₀, -C(O)-OR₂₉, -C(O) -NR₃₁R₃₂ or -C(NR₃₁)-NR₃₁R₃₂ radicals; or
(4) -OR₂₉, -SR₂₉, -S(O)-R₃₀, -S(O)₂-R₃₀, -S(O)₂-NR₃₁R₃₂, -NR₃₁R₃₂, -NR₃₃-C(O)-R₂₉ or -NR₃₃-C(O)-OR₃₀ radicals;
more preferably, R₁₁ is an aryl or heteroaryl radical other than an "N"-heteroaryl radical, and R₁₂ is a "N"-heteroaryl radical, wherein the aryl, heteroaryl and "N"-heteroaryl radicals are optionally substituted by 1-2 radicals of
(1) R₃₀;
(2) halo or cyano radicals;
(3) -C(O)-R₃₀, -C(O)-OR₂₉, -C(O)-NR₃₁R₃₂ or -C(NR₃₁)-NR₃₁R₃₂ radicals; or
(4) -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals;
more preferably, R₁₁ is an aryl or heteroaryl radical other than an "N"-heteroaryl radical, and R₁₂ is a "N"-heteroaryl radical, wherein the aryl, heteroayyl and "N"-heteroaryl radicals are optionally substituted by 1-2 radicals of
(1) R₃₀;
(2) halo or cyano radicals; or
(3) -C(O)-NR₃₁R₃₂, -OR₂₉, -SR₂₉, -S(O)-R₃₀, -S(O)₂-R₃₀, - S(O)₂-NR₃₁R₃₂, -NR₃₁R₃₂ or -NR₃₃-C(O)-R₂₉ radicals;
more preferably, R₁₁ is an aryl or heteroaryl radical other than an "N"-heteroaryl radical, optionally substituted by 1-2 radicals of (1) R₃₀; (2) halo or cyano radicals; or (3) -C(O)-NR₃₁R₃₂, -OR₂₉, -SR₂₉, - S(O)-R₃₀, -S(O)₂-R₃₀, -S(O)₂-NR₃₁R₃₂, -NR₃₁R₃₂ or -NR₃₃-C(O)-R₂₉ radicals; more preferably, R₁₁ is an aryl radical optionally substituted by 1-2 radicals of methyl, amino, dimethylamino, acetamido, hydroxy, halo, cyano, methoxy, methylthio, methylsulfinyl, methylsulfonyl, aminocarbonyl, methyl or trifluoromethyl radicals; more preferably, R₁₁ is an unsubstituted phenyl or naphthyl radical or a phenyl radical substituted by 1-2 radicals of methyl, amino, dimethylamino, acetamido, hydroxy, halo, cyano, methoxy, methylthio, methylsulfinyl, methylsulfonyl, aminocarbonyl, methyl or trifluoromethyl radicals; and most preferably, R₁₁ is an unsubstituted phenyl radical or a phenyl radical substituted by 1-2 radicals of methyl, amino, dimethylamino, acetamido, hydroxy, halo, cyano, methoxy, methylthio, methylsulfonyl, methyl or trifluoromethyl radicals;
more preferably, R₁₂ is an "N"-heteroaryl radical optionally substituted by 1-2 radicals of (1) R₃₀; (2) halo or cyano radicals; or (3) -C(O)-NR₃₁R₃₂, -OR₂₉, - SR₂₉, -NR₃₁R₃₂ or -NR₃₃-C(O)-R₂₉ radicals; more preferably, R₁₂ is an "N"-heteroaryl radical optionally substituted by 1-2 radicals of amino, dimethylamino, acetamido, hydroxy, halo, cyano, methoxy, methyl or trifluoromethyl radicals; more preferably, R₁₂ is a 4-pyridyl, 4-pyrimidyl, 4-quinolinyl, 7-imidazo[4,5-b]pyridinyl, 8-quinazolinyl, 6-(1*H*)-purinyl, or a 4-imidazolyl radical optionally substituted by a radical of amino, dimethylamino, acetamido, hydroxy, halo, cyano, methoxy, methyl or trifluoromethyl radicals; and most preferably, R₁₂ is a 4-pyridyl or 4-pyrimidyl radical optionally substituted by a radical of amino, dimethylamino, acetamido, hydroxy, halo, cyano, methoxy, methyl or trifluoromethyl radicals;
wherein each R₃₀ is independently
(1) alkyl, alkenyl or alkynyl radicals optionally substituted by 1-3 radicals of -NR₃₁R₃₁, -CO₂R₂₃, hydroxy, alkoxy, alkylthio, alkylsulfinyl, alkylsulfonyl, cyano, halo or aralkoxy, aralkylthio, aralkylsulfonyl, heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
preferably, each R₃₀ is independently
(1) C₁-C₄ alkyl, C₂-C₄ alkenyl or C₂-C₄ alkynyl radicals optionally substituted by 1-3 radicals of -NR₃₁R₃₁, -CO₂R₂₃, hydroxy, C₁-C₄ alkoxy, C₁-C₄ alkylthio, C₁-C₄ alkylsulfinyl, C₁-C₄ alkylsulfonyl, cyano, halo or aryl-C₁-C₄-alkoxy, aryl-C₁-C₄-alkylthio, axyl-C₁-C₄-alkylsulfonyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
more preferably, each R₃₀ is independently
(1) C₁-C₄ alkyl radical optionally substituted by 1-3 radicals of
   (a) -NR₃₁R₃₁;
   (b) C₁-C₄ alkoxy-carbonyl or phenoxycarbonyl or phenylmethoxycarbonyl optionally substituted by 1-3 radicals of amino, alkylamino, di-(C₁-C₄-alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or trifluoromethyl; or
   (c) hydroxy, C₁-C₄ alkoxy, C₁-C₄ alkylthio, or phenyl-C₁-C₄-alkoxy, phenyl-C₁-C₄-alkylthio, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₄ haloalkyl of 1-3 halo radicals;
(2) C₁-C₄ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
more preferably, each R₃₀ is independently
(1) C₁-C₄ alkyl radical optionally substituted by
   (a) amino, C₁-C₄ alkylamino or di-(C₁-C₄-alkyl)amino radicals; or
   (b) hydroxy, C₁-C₄ alkoxy, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
(2) C₁-C₂ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy) carbonylamino, hydroxy, C₁-C₄ alkoxy, C₁-C₄ alkylthio, cyano, halo, C₁-C₄ alkyl or trifluoromethyl radicals;
more preferably, each R₃₀ is independently
(1) C₁-C₄ alkyl radical optionally substituted by a phenyl or heteroaryl radical optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
(2) trifluoromethyl radical; or
(3) aryl or heteroaryl radicals optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
more preferably, each R₃₀ is independently
(1) C₁-C₄ alkyl radical optionally substituted by a phenyl or heteroaryl radical optionally substituted by 1-3 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
most preferably, R₃₀ is independently
(1) C₁-C₄ alkyl radical optionally substituted by a phenyl or heteroaryl radical optionally substituted by 1-2 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀; and
each R₃₁ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₁ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy) carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₁ is independently
(1) hydrogen radicals; or
(2) C₁-C₄ alkyl radical optionally substituted by an phenyl or heteroaryl radical optionally substituted by 1-3 radicals of amino, C₁-C₄ alkylamino, di-(C₁-C₄ alkyl) amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyl or trifluoromethyl radicals;
more preferably, each R₃₁ is independently hydrogen or C₁-C₄ alkyl radicals; and most preferably, each R₃₁ is independently hydrogen, methyl or ethyl radicals;
each R₃₂ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₂ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₂ is independently
(1) hydrogen radicals;
(2) C₁-C₄ alkyl radical optionally substituted by an C₃-C₆ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₆ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₂ is independently
(1) hydrogen radicals;
(2) C₁-C₄ alkyl radical optionally substituted by phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals; or
(3) phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals;
more preferably, each R₃₂ is independently
(1) hydrogen radicals;
(2) C₁-C₄ alkyl radical or C₁-C₂ alkyl radical substituted by phenyl or heteroaryl radical optionally substituted by 1-3 radicals of amino, dimethylamino, acetamido, hydroxy, methoxy, methyl or trifluoromethyl radicals; or
(3) phenyl or heteroaryl radical optionally substituted by 1-3 radicals of amino, dimethylamino, acetamido, hydroxy, methoxy, methyl or trifluoromethyl radicals;
most preferably, R₃₂ is independently
(1) hydrogen or C₁-C₄ alkyl radical; or
(2) phenyl or heteroaryl radical optionally substituted by 1-2 radicals of amino, dimethylamino, acetamido, hydroxy, methoxy, methyl or trifluoromethyl radicals; and
wherein each R₃₃ is independently
(1) hydrogen radical; or
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₃ is independently
(1) hydrogen radical; or
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₃ is independently hydrogen or C₁-C₄ alkyl radical; and most preferably, each R₃₃ is independently hydrogen or methyl radical; and
provided that when X is C-H, then Q is other than a phenyl radical; and when X is N and J is C-H, A is other than a 4-(methylsulfonyl)phenyl, 4-(aminosulfonyl)-phenyl, 4-(trifluoromethylcarbonylaminosulfonyl)phenyl or 4-(methylaminosulfonyl)phenyl radical.

The compounds of this invention may have in general several asymmetric centers and are typically depicted in the form of racemic mixtures. This invention is intended to encompass racemic mixtures, partially racemic mixtures and separate enantiomers and diasteromers.

Compounds of interest include the following: 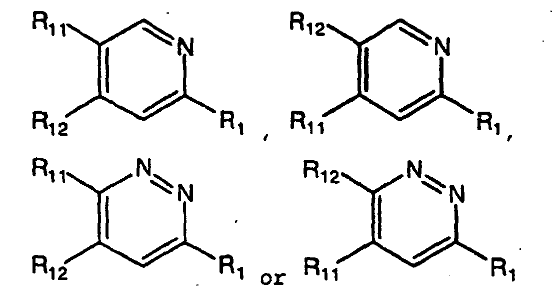 and preferably, 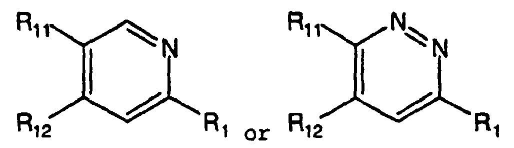 wherein R₁, R₁₁ and R₁₂ are one of the combinations given in the following table:

| R¹¹ | R¹² | R¹ |
|---|---|---|
| Phenyl | 4-pyridyl | 3-phenylpropylamino |
| 3-fluorophenyl | 4-pyridyl | 3-phenylpropylamino |
| 4-fluorophenyl | 4-pyridyl | 3-phenylpropylamino |
| 4-fluorophenyl | 4-pyrimidyl | 3-phenylpropylamino. |
| 3-tolyl | 4-pyrimidyl | 3-phenylpropylamino |
| 3-tolyl | 4-pyridyl | 3-phenylpropylamino |
| 3-CF₃-phenyl | 4-pyridyl | 3-phenylpropylamino |
| 3,4-dichlorophenyl | 4-pyridyl | 3-phenylpropylamino |
| 3,4-dimethyl phenyl | 4-pyridyl | 3-phenylpropylamino |
| 2-thienyl | 4-pyrimidyl | 3-phenylpropylamino |
| 2-furyl | 4-pyridyl | 3-phenylpropylamino |
| 2-benzothienyl | 4-pyridyl | 3-phenylpropylamino |
| 2-benzofuryl | 4-pyridyl | 3-phenylpropylamino |
| Phenyl | 4-pyridyl | 3-benzyl-1-piperidinyl |
| 3-fluorophenyl | 4-pyridyl | 3-benzyl-l-piperidinyl |
| 4-fluorophenyl | 4-pyridyl | 3-benzyl-1-pyrrolidinyl |
| 3-CF₃-phenyl | 4-pyrimidyl | 3-benzyl-1-piperidinyl |
| 3,4-dimethyl phenyl | 4-pyrimidyl | 3-benzyl-1-piperidinyl |
| 3-tolyl | 4-pyridyl | 3-benzyl-1-piperidinyl |
| 3-CF₃-phenyl | 4-pyridyl | 3-benzyl-1-piperidinyl |
| 3,4-dichlorophenyl | 4-pyridyl | 3-benzyl-1-piperidinyl |
| 3,4-dimethyl phenyl | 4-pyridyl | 2-benzyl-4-morpholino |
| 2-thienyl | 4-pyridyl | 3-benzyl-1-piperidinyl |
| 2-furyl | 4-pyridyl | 3-benzyl-1-piperidinyl |
| 2-benzothienyl | 4-pyridyl | 3-benzyl-l-piperidinyl |
| 2-benzofuryl | 4-pyridyl | 3-benzyl-l-piperidinyl |
| Phenyl | 4-pyridyl | 3-benzyl-1-piperazinyl |
| 3-fluorophenyl | 4-pyridyl | 3-benzyl-l-piperazinyl |
| 4-fluorophenyl | 4-pyridyl | 3-benzyl-1-piperazinyl |
| 3-tolyl | 4-pyridyl | 3-benzyl-1-piperazinyl |
| 3-CF₃-phenyl | 4-pyridyl | 3-benzyl-1-piperazinyl |
| 3-fluorophenyl | 4-pyrimidyl | 3-benzyl-1-piperazinyl |
| Phenyl | 4-pyrimidyl | 3-benzyl-1-piperazinyl |
| 3,4-dichlorophenyl | 4-pyridyl | 3-benzyl-1-piperazinyl |
| 3,4-dimethyl phenyl | 4-pyridyl | 3-benzyl-1-piperazinyl |
| 2-thienyl | 4-pyridyl | 3-benzyl-1-piperazinyl |
| 2-furyl | 4-pyridyl | 3-benzyl-1-piperazinyl |
| 2-benzothienyl | 4-pyrimidyl | 3-benzyl-1-piperazinyl |
| 2-benzofuryl | 4-pyridyl | 3-benzyl-1-piperazinyl |
| Phenyl | 4-pyridyl | 2-amino-3-phenylpropylamino |
| 3-fluorophenyl | 4-pyridyl | 2-amino-3-phenylpropylamino |
| 4-fluorophenyl | 4-pyridyl | 2-amino-3-phenylpropylamino |
| 3-tolyl | 4-pyridyl | 2-amino-3-phenylpropylamino |
| 3-CF₃-phenyl | 4-pyridyl | 2-amino-3-phenylpropylamino |
| 3,4-dichlorophenyl | 4-pyridyl | 2-amino-3-phenylpropylamino |
| 3,4-dimethyl phenyl | 4-pyridyl | 2-amino-3-phenylpropylamino |
| 3-fluorophenyl | 4-pyrimidyl | 2-amino-3-phenylpropylamino |
| 3-tolyl | 4-pyrimidyl | 2-amino-3-phenylpropylamino |
| 2-thienyl | 4-pyridyl | 2-amino-3-phenylpropylamino |
| 2-furyl | 4-pyrimidyl | 2-amino-3-phenylpropylamino |
| 2-benzothienyl | 4-pyridyl | 2-amino-3-phenylpropylamino |
| 2-benzofuryl | 4-pyridyl | 2-amino-3-phenylpropylamino |
| Phenyl | 4-pyridyl | 3-amino-3-phenylpropylamino |
| 4-fluorophenyl | 4-pyridyl | 3-amino-3-phenylpropylamino |
| 3,4-dimethyl phenyl | 4-pyrimidyl | 3-amino-3-phenylpropylamino |
| 3-fluorophenyl | 4-pyrimidyl | 3-amino-3-phenylpropylamino |
| 3-tolyl | 4-pyridyl | 3-amino-3-phenylpropylamino |
| 3-CF₃-phenyl | 4-pyridyl | 3-amino-3-phenylpropylamino |
| 2-thienyl | 4-pyridyl | 3-amino-3-phenylpropylamino |
| 2-furyl | 4-pyridyl | 3-amino-3-phenylpropylamino |
| 2-benzothienyl | 4-pyridyl | 3-amino-3-phenylpropylamino |
| 2-benzofuryl | 4-pyrimidyl | 3-amino-3-phenylpropylamino |
| Phenyl | 4-pyridyl | 3-amino-3-phenyl-2,2-dimethylpropylamino |
| 3-fluorophenyl | 4-pyridyl | 3-amino-3-phenyl-2,2-dimethylpropylamino |
| 4-fluorophenyl | 4-pyridyl | 3-amino-3-phenyl-2,2-dimethylpropylamino |
| 3-tolyl | 4-pyridyl | 3-amino-3-phenyl-2,2-dimethylpropylamino |
| 3-CF₃-phenyl | 4-pyridyl | 3-amino-3-phenyl-2,2-dimethylpropylamino |
| 3,4-dichlorophenyl | 4-pyridyl | 3-amino-3-phenyl-2,2-dimethylpropylamino |
| 3,4-dimethyl phenyl | 4-pyridyl | 3-amino-3-phenyl-2,2-dimethylpropylamino |
| 3-fluorophenyl | 4-pyrimidyl | 3-amino-3-phenyl-2,2-dimethylpropylamino |
| 3-tolyl | 4-pyrimidyl | 3-amino-3-phenyl-2,2-dimethylpropylamino |
| 2-thienyl | 4-pyridyl | 3-amino-3-phenyl-2,2-dimethylpropylamino |
| 2-furyl | 4-pyrimidyl | 3-amino-3-phenyl-2,2-dimethylpropylamino |
| 2-benzothienyl | 4-pyridyl | 3-amino-3-phenyl-2,2-dimethylpropylamino |
| 2-benzofuryl | 4-pyridyl | 3-amino-3-phenyl-2,2-dimethylpropylamino |

Further compounds of interest include the following: 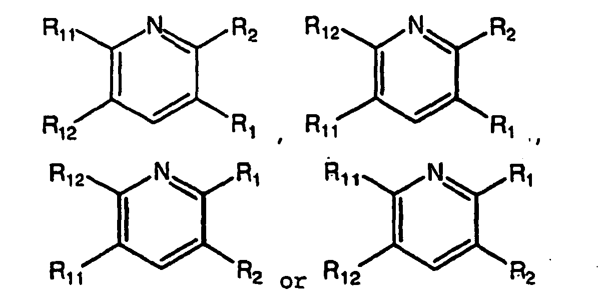 and preferably, 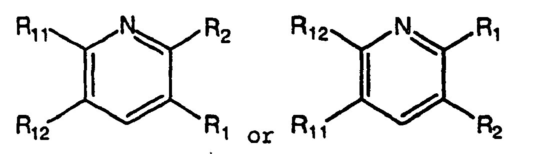 wherein R₂ is a hydrogen, methyl, trifluoromethyl, cyano, phenyl or 4-pyridyl radical, preferably, R₂ is a hydrogen, methyl or trifluoromethyl radical, and R₁, R₁₁ and R₁₂ are one of the combinations given in the following table:

| R¹¹ | R¹² | R¹ |
|---|---|---|
| Phenyl | 4-pyridyl | 3-phenylpropylamino |
| 3-fluorophenyl | 4-pyridyl | 3-phenylpropylamino |
| 4-fluorophenyl | 4-pyridyl | 3-phenylpropylamino |
| 4-fluorophenyl | 4-pyrimidyl | 3-phenylpropylamino |
| 3-tolyl | 4-pyrimidyl | 3-phenylpropylamino |
| 3-tolyl | 4-pyridyl | 3-phenylpropylamino |
| 3-CF₃-phenyl | 4-pyridyl | 3-phenylpropylamino |
| 3,4-dichlorophenyl | 4-pyridyl | 3-phenylpropylamino |
| 3,4-dimethyl phenyl | 4-pyridyl | 3-phenylpropylamino |
| 2-thienyl | 4-pyrimidyl | 3-phenylpropylamino |
| 2-furyl | 4-pyridyl | 3-phenylpropylamino |
| 2-benzothienyl | 4-pyridyl | 3-phenylpropylamino |
| 2-benzofuryl | 4-pyridyl | 3-phenylpropylamino |
| Phenyl | 4-pyridyl | 3-benzyl-1-piperidinyl |
| 3-fluorophenyl | 4-pyridyl | 3-benzyl-1-piperidinyl |
| 4-fluorophenyl | 4-pyridyl | 3-benzyl-1-pyrrolidinyl |
| 3-CF₃-phenyl | 4-pyrimidyl | 3-benzyl-1-piperidinyl |
| 3,4-dimethyl phenyl | 4-pyrimidyl | 3-benzyl-1-piperidinyl |
| 3-tolyl | 4-pyridyl | 3-benzyl-1-piperidinyl |
| 3-CF₃-phenyl | 4-pyridyl | 3-benzyl-1-piperidinyl |
| 3,4-dichlorophenyl | 4-pyridyl | 3-benzyl-1-piperidinyl |
| 3,4-dimethyl phenyl | 4-pyridyl | 2-benzyl-4-morpholino |
| 2-thienyl | 4-pyridyl | 3-benzyl-1-piperidinyl |
| 2-furyl | 4-pyridyl | 3-benzyl-1-piperidinyl |
| 2-benzothienyl | 4-pyridyl | 3-benzyl-1-piperidinyl |
| 2-benzofuryl | 4-pyridyl | 3-benzyl-1-piperidinyl |
| Phenyl | 4-pyridyl | 3-benzyl-1-piperazinyl |
| 3-fluorophenyl | 4-pyridyl | 3-benzyl-1-piperazinyl |
| 4-fluorophenyl | 4-pyridyl | 3-benzyl-1-piperazinyl |
| 3-tolyl | 4-pyridyl | 3-benzyl-1-piperazinyl |
| 3-CF₃-phenyl | 4-pyridyl | 3-benzyl-1-piperazinyl |
| 3-fluorophenyl | 4-pyrimidyl | 3-benzyl-1-piperazinyl |
| Phenyl | 4-pyrimidyl | 3-benzyl-1-piperazinyl |
| 3,4-dichlorophenyl | 4-pyridyl | 3-benzyl-1-piperazinyl |
| 3,4-dimethyl phenyl | 4-pyridyl | 3-benzyl-1-piperazinyl |
| 2-thienyl | 4-pyridyl | 3-benzyl-1-piperazinyl |
| 2-furyl | 4-pyridyl | 3-benzyl-1-piperazinyl |
| 2-benzothienyl | 4-pyrimidyl | 3-benzyl-1-piperazinyl |
| 2-benzofuryl | 4-pyridyl | 3-benzyl-1-piperazinyl |
| Phenyl | 4-pyridyl | 2-amino-3-phenylpropylamino |
| 3-fluorophenyl | 4-pyridyl | 2-amino-3-phenylpropylamino |
| 4-fluorophenyl | 4-pyridyl | 2-amino-3-phenylpropylamino |
| 3-tolyl | 4-pyridyl | 2-amino-3-phenylpropylamino |
| 3-CF₃-phenyl, | 4-pyridyl | 2-amino-3-phenylpropylamino |
| 3,4-dichlorophenyl | 4-pyridyl | 2-amino-3-phenylpropylamino |
| 3,4-dimethyl phenyl | 4-pyridyl | 2-amino-3-phenylpropylamino |
| 3-fluorophenyl | 4-pyrimidyl | 2-amino-3-phenylpropylamino |
| 3-tolyl | 4-pyrimidyl | 2-amino-3-phenylpropylamino |
| 2-thienyl | 4-pyridyl | 2-amino-3-phenylpropylamino |
| 2-furyl | 4-pyrimidyl | 2-amino-3-phenylpropylamino |
| 2-benzothienyl | 4-pyridyl | 2-amino-3-phenylpropylamino |
| 2-benzofuryl | 4-pyridyl | 2-amino-3-phenylpropylamino |
| Phenyl | 4-pyridyl | 3-amino-3-phenylpropylamino |
| 4-fluorophenyl | 4-pyridyl | 3-amino-3-phenylpropylamino |
| 3,4-dimethyl phenyl | 4-pyrimidyl | 3-amino-3-phenylpropylamino |
| 3-fluorophenyl | 4-pyrimidyl | 3-amino-3-phenylpropylamino |
| 3-tolyl | 4-pyridyl | 3-amino-3-phenylpropylamino |
| 3-CF₃-phenyl | 4-pyridyl | 3-amino-3-phenylpropylamino |
| 2-thienyl | 4-pyridyl | 3-amino-3-phenylpropylamino |
| 2-furyl | 4-pyridyl | 3-amino-3-phenylpropylamino |
| 2-benzothienyl | 4-pyridyl | 3-amino-3-phenylpropylamino |
| 2-benzofuryl | 4-pyrimidyl | 3-amino-3-phenylpropylamino |
| Phenyl | 4-pyridyl | 3-amino-3-phenyl-2,2-dimethylpropylamino |
| 3-fluorophenyl | 4-pyridyl | 3-amino-3-phenyl-2,2-dimethylpropylamino |
| 4-fluorophenyl | 4-pyridyl | 3-amino-3-phenyl-2,2-dimethylpropylamino |
| 3-tolyl | 4-pyridyl | 3-amino-3-phenyl-2,2-dimethylpropylamino |
| 3-CF₃-phenyl | 4-pyridyl | 3-amino-3-phenyl-2,2-dimethylpropylamino |
| 3,4-dichlorophenyl | 4-pyridyl | 3-amino-3-phenyl-2,2-dimethylpropylamino |
| 3,4-dimethyl phenyl | 4-pyridyl | 3-amino-3-phenyl-2,2-dimethylpropylamino |
| 3-fluorophenyl | 4-pyrimidyl | 3-amino-3-phenyl-2,2-dimethylpropylamino |
| 3-tolyl | 4-pyrimidyl | 3-amino-3-phenyl-2,2-dimethylpropylamino |
| 2-thienyl | 4-pyridyl | 3-amino-3-phenyl-2,2-dimethylpropylamino |
| 2-furyl | 4-pyrimidyl | 3-amino-3-phenyl-2,2-dimethylpropylamino |
| 2-benzothienyl | 4-pyridyl | 3-amino-3-phenyl-2,2-dimethylpropylamino |
| 2-benzofuryl | 4-pyridyl | 3-amino-3-phenyl-2,2-dimethylpropylamino |

Still further compounds of interest include the following: 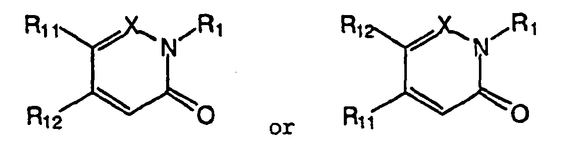 wherein X is N or C-H, and R₁, R₁₁ and R₁₂ are one of the combinations given in the following table:

| R¹¹ | R¹² | R¹ |
|---|---|---|
| Phenyl | 4-pyridyl | 3-phenylpropyl |
| 3-fluorophenyl | 4-pyridyl | 3-phenylpropyl |
| 4-fluorophenyl | 4-pyridyl | 3-phenylpropyl |
| 3-tolyl | 4-pyridyl | 3-phenylpropyl |
| 3-trifluoromethylphenyl | 4-pyridyl | 3-phenylpropyl |
| 3,4-dichlorophenyl | 4-pyridyl | 3-phenylpropyl |
| 3,4-dimethyl phenyl | 4-pyridyl | 3-phenylpropyl |
| Phenyl | 4-pyridyl | 2-amino-3-phenylpropyl |
| 3-fluorohenyl | 4-pyridyl | 2-amino-3-phenylpropyl |
| 4-fluorophenyl | 4-pyridyl | 2-amino-3-phenylpropyl |
| 3-tolyl | 4-pyridyl | 2-amino-3-phenylpropyl |
| 3-trifluoromethylphenyl | 4-pyridyl | 2-amino-3-phenylpropyl |
| 3,4-dichlorophenyl | 4-pyridyl | 2-amino-3-phenylpropyl |
| 3,4-dimethyl phenyl | 4-pyrimidiny | 2-amino-3-phenylpropyl |
| Phenyl | 4-pyridyl | 3-amino-3-phenylpropyl |
| 4-fluorophenyl | 4-pyridyl | 3-amino-3-phenylpropyl |
| 3-tolyl | 4-pyridyl | 3-amino-3-phenylpropyl |
| 3-trifluoromethylphenyl | 4-pyridyl | 3-amino-3-phenylpropyl |
| 2-thienyl | 4-pyridyl | 2-amino-3-phenylpropyl |
| 3-benzofuryl | 4-pyridyl | 2-amino-3-phenylpropyl |
| Phenyl | 4-pyrimidyl | 3-amino-3-phenylpropyl |
| 4-fluorophenyl | 4-pyrimidyl | 3-amino-3-phenylpropyl |
| 3-tolyl | 4-pyrimidyl | 3-amino-3-phenylpropyl |
| 3-trifluoromethylphenyl | 4-pyrimidyl | 2-amino-3-phenylpropyl |
| 2-thienyl | 4-pyrimidyl | 2-amino-3-phenylpropyl |
| 3-benzofuryl | 4-pyrimidyl | 2-amino-3-phenylpropyl |
| Phenyl | 4-(2-aminopyrimidyl | 3-amino-3-phenylpropyl |
| 4-fluorophenyl | 4-(2-aminopyrimidyl | 3-amino-3-phenylpropyl |
| 3-tolyl | 4-(2-aminopyrimidyl | 3-amino-3-phenylpropyl |
| 3-trifluoromethylphenyl | 4-(2-aminopyrimidyl | 2-amino-3-phenylpropyl |
| 2-thienyl | 4-(2-aminopyrimidyl | 2-amino-3-phenylpropyl |
| 3-benzofuryl | 4-(2-aminopyrimidyl | 2-amino-3-phenylpropyl |
| Phenyl | 4-quinolyl | 3-amino-3-phenylpropyl |
| 4-fluorophenyl | 4-quinolyl | 3-amino-3-phenylpropyl |
| 3-tolyl | 4-quinolyl | 3-amino-3-phenylpropyl |
| 3-trifluoromethylphenyl | 4-quinolyl | 2-amino-3-phenylpropyl |
| 2-thienyl | 4-quinolyl | 2-amino-3-phenylpropyl |
| 3-benzofuryl | 4-quinolyl | 2-amino-3-phenylpropyl |
| Phenyl | 4-pyridyl | 3-amino-3-phenyl-2,2-dimethylpropyl |
| 3-fluorophenyl | 4-pyridyl | 3-amino-3-phenyl-2,2-dimethylpropyl |
| 4-fluorophenyl | 4-pyridyl | 3-amino-3-phenyl-2,2-dimethylpropyl |
| 3-tolyl | 4-pyridyl | 3-amino-3-phenyl-2,2-dimethylpropyl |
| 3-CF₃-phenyl | 4-pyridyl | 3-amino-3-phenyl-2,2-dimethylpropyl |
| 3,4-dichlorophenyl | 4-pyridyl | 3-amino-3-phenyl-2,2-dimethylpropyl |
| 3,4-dimethyl phenyl | 4-pyridyl | 3-amino-3-phenyl-2,2-dimethylpropyl |
| 3-fluorophenyl | 4-pyrimidyl | 3-amino-3-phenyl-2,2-dimethylpropyl |
| 3-tolyl | 4-pyrimidyl | 3-amino-3-phenyl-2,2-dimethylpropyl |
| 2-thienyl | 4-pyridyl | 3-amino-3-phenyl-2,2-dimethylpropyl |
| 2-furyl | 4-pyrimidyl | 3-amino-3-phenyl-2,2-dimethylpropyl |
| 2-benzothienyl | 4-pyridyl | 3-amino-3-phenyl-2,2-dimethylpropyl |
| 2-benzofuryl | 4-pyridyl | 3-amino-3-phenyl-2,2-dimethylpropyl |
| Phenyl | 4-pyridyl | 2-benzyl-4-piperidinyl |
| 3-fluorophenyl | 4-pyridyl | 2-benzyl-4-piperidinyl |
| 4-fluorophenyl | 4-pyridyl | 2-benzyl-4-piperidinyl |
| 3-CF₃-phenyl | 4-pyrimidyl | 2-benzyl-4-piperidinyl |
| 3,4-dimethyl phenyl | 4-pyrimidyl | 2-benzyl-4-piperidinyl |
| 3-tolyl | 4-pyridyl | 2-benzyl-4-piperidinyl |
| 3-CF₃-phenyl | 4-pyridyl | 2-benzyl-4-piperidinyl |
| 3,4-dichlorophenyl | 4-pyridyl | 2-benzyl-4-piperidinyl |
| 3,4-dimethyl phenyl | 4-pyridyl | 2-benzyl-4-piperidinyl |
| 2-thienyl | 4-pyridyl | 2-benzyl-4-piperidinyl |
| 2-furyl | 4-pyridyl | 2-benzyl-4-piperidinyl |
| 2-benzothienyl | 4-pyrimidyl | 2-benzyl-4-piperidinyl |
| 2-benzofuryl | 4-pyridyl | 2-benzyl-4-piperidinyl |
| Phenyl | 4-pyridyl | phenylethyl |
| 3-fluorophenyl | 4-pyridyl | phenylethyl |
| 4-fluorophenyl | 4-pyridyl | phenylethyl |
| 3-tolyl | 4-pyridyl | phenylethyl |
| 3-trifluoromethylphenyl | 4-pyridyl | phenylethyl |
| 3,4-dichlorophenyl | 4-pyridyl | phenylethyl |
| 3,4-dimethyl phenyl | 4-pyridyl | phenylethyl |
| Phenyl | 4-pyridyl | benzyl |
| 3-fluorohenyl | 4-pyridyl | benzyl |
| 4-fluorophenyl | 4-pyridyl | benzyl |
| 3-tolyl | 4-pyridyl | benzyl |
| 3-trifluoromethylphenyl | 4-pyridyl | benzyl |
| 3,4-dichlorophenyl | 4-pyridyl | benzyl |
| 3,4-dimethyl phenyl | 4-pyrimidiny | benzyl |
| Phenyl | 4-pyridyl | 2-chlorophenylmethyl |
| 4-fluorophenyl | 4-pyridyl | 2-chlorophenylmethyl |
| 3-tolyl | 4-pyridyl | 2-chlorophenylmethyl |
| 3-trifluoromethylphenyl | 4-pyridyl | 2-chlorophenylmethyl |
| 2-thienyl | 4-pyridyl | 2-chlorophenylmethyl |
| 3-benzofuryl | 4-pyridyl | 2-chlorophenylmethyl |
| Phenyl | 4-pyrimidyl | 4-pyridylmethyl |
| 4-fluorophenyl | 4-pyrimidyl | 4-pyridylmethyl |
| 3-tolyl | 4-pyrimidyl | 4-pyridylmethyl |
| 3-trifluoromethylphenyl | 4-pyrimidyl | 4-pyridylmethyl |
| 2-thienyl | 4-pyrimidyl | 4-pyridylmethyl |
| 3-benzofuryl | 4-pyrimidyl | 4-pyridylmethyl |
| Phenyl | 4-(2-aminopyrimidyl | 4-pyrolidinylmethyl |
| 4-fluorophenyl | 4-(2-aminopyrimidyl | 4-pyrolidinylmethyl |
| 3-tolyl | 4-(2-aminopyrimidyl | 4-pyrolidinylmethyl |
| 3-trifluoromethylphenyl | 4-(2-aminopyrimidyl | 4-pyrolidinylmethyl |
| 2-thienyl | 4-(2-aminopyrimidyl | 4-pyrolidinylmethyl |
| 2-benzothiophenyl | 4-pyridyl | 4-pyrolidinylmethyl |
| 2-quinolyl | 4-pyridyl | 4-pyrolidinylmethyl |
| 3-isopropylphenyl | 4-pyridyl | 4-pyrolidinylmethyl |

Additional preferred compounds are included in the Examples, *infra*.

As utilized herein, the following terms shall either have the meanings defined by the claims, or shall have the following meanings:
"a" means the bond order of the bond between J and the adjacent ring carbon atom to which W is attached. "a" may be either a single or double bond. "b" means the bond order of the bond between W and the adjacent ring carbon atom to which W is attached. "b" may be either a single or double bond.
"Alkyl", alone or in combination, means a straight-chain or branched-chain alkyl radical containing preferably 1-15 carbon atoms (C₁-C₁₅), more preferably 1-8 carbon atoms (C₁-C₈), even more preferably 1-6 carbon atoms (C₁-C₆), yet more preferably 1-4 carbon atoms (C₁-C₄), still more preferably 1-3 carbon atoms (C₁-C₃), and most preferably 1-2 carbon atoms (C₁-C₂)- Examples of such radicals include methyl; ethyl, n-propyl, isopropyl, n-butyl, isobutyl, sec-butyl, tert-butyl, pentyl, isoamyl, hexyl, octyl and the like.
"Hydroxyalkyl", alone or in combination, means an alkyl radical as defined above wherein at least one hydrogen radical is replaced with a hydroxyl radical, preferably 1-3 hydrogen radicals are replaced by hydroxyl radicals, more preferably 1-2 hydrogen radicals are replaced by hydroxyl radicals, and most preferably one hydrogen radical is replaced by a hydroxyl radical. Examples of such radicals include hydroxymethyl, 1-, 2-hydroxyethyl, 1-, 2-, 3-hydroxypropyl, 1,3-dihydroxy-2-propyl, 1,3-dihydroxybutyl, 1,2,3,4,5,6-hexahydroxy-2-hexyl and the like.
"Alkenyl", alone or in combination, means a straight-chain or branched-chain hydrocarbon radical having one or more double bonds, preferably 1-2 double bonds and more preferably one double bond, and containing preferably 2-15 carbon atoms (C₂-C₁₅), more preferably 2-8 carbon atoms (C₂-C₈), even more preferably 2-6 carbon atoms (C₂-C₆), yet more preferably 2-4 carbon atoms (C₂-C₄), and still more preferably 2-3 carbon atoms (C₂-C₃). Examples of such alkenyl radicals include ethenyl, propenyl, 2-methylpropenyl, 1,4-butadienyl and the like.
"Alkoxy", alone or in combination, means a radical of the type "R-O*-"* wherein "R*"* is an alkyl- radical as defined above and "O" is an oxygen atom. Examples of such alkoxy radicals include methoxy, ethoxy, n-propoxy, isopropoxy, n-butoxy, iso-butoxy, sec-butoxy, tert-butoxy and the like.
"Alkoxycarbonyl", alone or in combination, means a radical of the type "R-O-C(O)-" wherein "R-O-" is an alkoxy radical as defined above and "C(O)" is a carbonyl radical.
"Alkoxycarbonylamino", alone or in combination, means a radical of the type "R-O-C(O)-NH-" wherein "R-O-C(O)" is an alkoxycarbonyl radical as defined above, wherein the amino radical may optionally be substituted, such as with alkyl, aryl, aralkyl, cycloalkyl, cycloalkylalkyl and the like.
"Alkylthio", alone or in combination, means a radical of the type "R-S-" wherein "R" is an alkyl radical as defined above and "S" is a sulfur atom. Examples of such alkylthio radicals include methylthio, ethylthio, n-propylthio, isopropylthio, n-butylthio, iso-butylthio, sec-butylthio, tert-butylthio and the like.
"Alkylsulfinyl", alone or in combination, means a radical of the type "R-S(O)-" wherein "R" is an alkyl radical as defined above and "S(O)" is a mono-oxygenated sulfur atom. Examples of such alkylsulfinyl radicals include methylsulfinyl, ethylsulfinyl, n-propylsulfinyl, isopropylsulfinyl, n-butylsulfinyl, iso-butylsulfinyl, sec-butylsulfinyl, tert-butylsulfinyl and the like.
"Alkylsulfonyl", alone or in combination, means a radical of the type "R-S(O)₂-" wherein "R" is an alkyl radical as defined above and "S(O)₂" is a di-oxygenated sulfur atom. Examples of such alkylsulfonyl radicals include methylsulfonyl, ethylsulfonyl, n-propylsulfonyl, isopropylsulfonyl, n-butylsulfonyl, iso-butylsulfonyl, sec-butylsulfonyl, tert-butylsulfonyl and the like.
"Aryl", alone or in combination, means a phenyl or biphenyl radical, which is optionally benzo fused or heterocyclo fused and which is optionally substituted with one or more substituents selected from alkyl, alkoxy, halogen, hydroxy, amino, azido, nitro, cyano, haloalkyl, carboxy, alkoxycarbonyl, cycloalkyl, alkanoylamino, amido, amidino, alkoxycarbonylamino, N-alkylamidino, alkylamino, dialkylamino, aminoalkyl, alkylaminoalkyl, dialkylaminoalkyl, N-alkylamido, N,N-dialkylamido, aralkoxycarbonylamino, alkylthio, alkylsulfinyl, alkylsulfonyl, oxo and the like. Examples of aryl radicals are phenyl, o-tolyl, 4-methoxyphenyl, 2-(tert-butoxy)phenyl, 3-methyl-4-methoxyphenyl, 2-CF₃-phenyl, 2-fluorophenyl, 2-chlorophenyl, 3-nitrophenyl, 3-aminophenyl, 3-acetamidophenyl, 2-amino-3-(aminomethyl)phenyl, 6-methyl-3-acetamidophenyl, 6-methyl-2-aminophenyl, 6-methyl-2,3-diaminophenyl, 2-amino-3-methylphenyl, 4,6-dimethyl-2-aminophenyl, 4-hydroxyphenyl, 3-methyl-4-hydroxyphenyl, 4-(2-methoxyphenyl)phenyl, 2-amino-1-naphthyl, 2-naphthyl, 3-amino-2-naphthyl, 1-methyl-3-amino-2-naphthyl, 2,3-diamino-1-naphthyl, 4,8-dimethoxy-2-naphthyl and the like.
"Aralkyl" and "arylalkyl", alone or in combination, means an alkyl radical as defined above in which at least one hydrogen atom, preferably 1-2, is replaced by an aryl radical as defined above, such as benzyl, 1-, 2-phenylethyl, dibenzylmethyl, hydroxyphenylmethyl, methylphenylmethyl, diphenylmethyl, dichlorophenylmethyl, 4-methoxyphenylmethyl and the like. For example, phenylmethyl means a methylene diradical substituted with a phenyl radical, i.e., Ph-CH₂-, whereas a methylphenyl means a phenylene diradical substituted with a methyl radical, i.e., CH₃-Ph-.
"Aralkoxy", alone or in combination, means an alkoxy radical as defined above in which at least one hydrogen atom, preferably 1-2, is replaced by an aryl radical as defined above, such as benzyloxy, 1-, 2-phenylethoxy, dibenzylmethoxy, hydroxyphenylmethoxy, methylphenylmethoxy, dichlorophenylmethoxy, 4-methoxyphenylmethoxy and the like.
"Aralkoxycarbonyl", alone or in combination, means a radical of the type "R-O-C(O)-" wherein "R-O-" is an aralkoxy radical as defined above and "-C(O)-" is a carbonyl radical.
"Alkanoyl", alone or in combination, means a radical of the type "R-C(O)-" wherein "R" is an alkyl radical as defined above and "-C(O)-" is a carbonyl radical. Examples of such alkanoyl radicals include acetyl, trifluoroacetyl, hydroxyacetyl, propionyl, butyryl, valeryl, 4-methylvaleryl, and the like.
"Alkanoylamino", alone or in combination, means a radical of the type "R-C(O)-NH-'' wherein "R-C(O)-" is an alkanoyl radical as defined above, wherein the amino radical may optionally be substituted, such as with alkyl, aryl, aralkyl, cycloalkyl, cycloalkylalkyl and the like.
"Aminocarbonyl", alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like.
"Aminosulfonyl", alone or in combination, means an amino substituted sulfonyl radical.
"Benzo", alone or in combination, means the divalent radical C₆H₄= derived from benzene. "Benzo fused" forms a ring system in which benzene and a cycloalkyl or aryl group have two carbons in common, for example tetrahydronaphthylene and the like.
"Bicyclic" as used herein is intended to include both fused ring systems, such as naphthyl and β-carbolinyl, and substituted ring systems, such as biphenyl, phenylpyridyl and diphenylpiperazinyl.
"Cycloalkyl", alone or in combination, means a saturated or partially saturated, preferably one double bond, monocyclic, bicyclic or tricyclic carbocyclic alkyl radical, preferably monocyclic, containing preferably 5-12 carbon atoms (C₅-C₁₂), more preferably 5-10 carbon atoms (C₅-C₁₀), even more preferably 5-7 carbon atoms (C₅-C₇), which is optionally benzo fused or heterocyclo fused and which is optionally substituted as defined herein with respect to the definition of aryl. Examples of such cycloalkyl radicals include cyclopentyl, cyclohexyl, dihydroxycyclohexyl, ethylenedioxycyclohexyl, cycloheptyl, octahydronaphthyl, tetrahydronaphthyl, octahydroquinolinyl, dimethoxytetrahydronaphthyl, 2,3-dihydro-1H-indenyl, azabicyclo[3.2.1]octyl and the like.
"Heteroatoms" means nitrogen, oxygen and sulfur heteroatoms.
"Heterocyclo fused" forms a ring system in which a heterocyclyl or heteroaryl group of 5-6 ring members and a cycloalkyl or aryl group have two carbons in common, for example indole, isoquinoline, tetrahydroquinoline, methylenedioxybenzene and the like.
"Heterocyclyl" means a saturated or partially unsaturated, preferably one double bond, monocyclic or bicyclic, preferably monocyclic, heterocycle radical containing at least one, preferably 1 to 4, more preferably 1 to 3, even more preferably 1-2, nitrogen, oxygen or sulfur atom ring member and having preferably 3-8 ring members in each ring, more preferably 5-8 ring members in each ring and even more preferably 5-6 ring members in each ring. "Heterocyclyl" is intended to include sulfone and sulfoxide derivatives of sulfur ring members and N-oxides of tertiary nitrogen ring members, and carbocyclic fused, preferably 3-6 ring carbon atoms and more preferably 5-6 ring carbon atoms, and benzo fused ring systems. "Heterocyclyl" radicals may optionally be substituted on at least one, preferably 1-4, more preferably 1-3, even more preferably 1-2, carbon atoms by halogen, alkyl, alkoxy, hydroxy, oxo, thioxo, aryl, aralkyl, heteroaryl, heteroaralkyl, amidino, N-alkylamidino, alkoxycarbonylamino, alkylsulfonylamino and the like, and/or on a secondary nitrogen atom by hydroxy, alkyl, aralkoxycarbonyl, alkanoyl, alkoxycarbonyl, heteroaralkyl, aryl or aralkyl radicals. More preferably, "heterocyclyl", alone or in combination, is a radical of a monocyclic or bicyclic saturated heterocyclic ring system having 5-8 ring members per ring, wherein 1-3 ring members are oxygen, sulfur or nitrogen heteroatoms, which is optionally partially unsaturated or benzo-fused and optionally substituted by 1-2 oxo or thioxo radicals. Examples of such heterocyclyl radicals include pyrrolidinyl, piperidinyl, piperazinyl, morpholinyl, thiamorpholinyl, 4-benzyl-piperazin-1-yl, pyrimidyl, tetrahydrofuryl, pyrazolidonyl, pyrazolinyl, pyridazinonyl, pyrrolidonyl, tetrahydrothienyl and its sulfoxide and sulfone derivatives, 2,3-dihydroindolyl, tetrahydroquinolinyl, 1,2,3,4-tetrahydroisoquinolinyl, 1,2,3,4-tetrahydro-1-oxo-isoquinolinyl, 2,3-dihydrobenzofuryl, benzopyranyl, methylenedioxyphenyl, ethylenedioxyphenyl and the like.
"Heteroaryl" means a monocyclic or bicyclic, preferably monocyclic, aromatic heterocycle radical, having at least one, preferably 1 to 4, more preferably 1 to 3, even more preferably 1-2, nitrogen, oxygen or sulfur atom ring members and having preferably 5-6 ring members in each ring, which is optionally saturated carbocyclic fused, preferably 3-4 carbon atoms (C₃-C₄) to form 5-6 ring membered rings and which is optionally substituted as defined above with respect to the definitions of aryl. Examples of such heteroaryl groups include thienyl, furyl oxazolyl, thiazolyl, benzothiazolyl, benzofuryl, benzothienyl, imidazolyl, pyrrolyl, pyrazolyl, pyridyl, 3-(2-methyl)pyridyl, 3-(4-trifluoromethyl)pyridyl, pyrimidyl, 5-(4-trifluoromethyl)pyrimidyl, pyrazinyl, triazolyl, indolyl, quinolinyl, 5,6,7,8-tetrahydroquinolyl, 5,6,7,8-tetrahydroisoquinolinyl, quinoxalinyl, benzimidazolyl, benzoxazolyl and the like.
"N"-heteroaryl" means an aromatic 5-10 membered monocyclic or bicyclic, preferably a monocyclic, aromatic heterocycle radical containing at least one, preferably 1 to 3, more preferably 1 to 2, even more preferably 1 nitrogen atoms with the remaining atoms being carbon, and having preferably 5-6 ring members in each ring, which is optionally saturated carbocyclic fused, preferably 3-4 carbon atoms (C₃-C₄) to form 5-6 ring membered rings and which is optionally substituted as defined above with respect to the definitions of aryl. Examples of such "N"-heteroaryl groups include imidazolyl, pyrrolyl,' pyrazolyl, pyridyl, 4-(2-amino)pyridyl, 3-(4-trifluoromethyl)pyridyl, pyrimidyl, 5-(4-trifluoromethyl)pyrimidyl, pyrazinyl, triazolyl, indolyl, quinolinyl, imidazopyridine, 5,6,7,8-tetrahydroquinolyl, 5,6,7,8-tetrahydroisoquinolinyl, benzimidazolyl, and the like.
"Heteroaralkyl" and "heteroarylalkyl," alone or in combination, means an alkyl radical as defined above in which at least one hydrogen atom, preferably 1-2, is replaced by a heteroaryl radical as defined above, such as 3-furylpropyl, 2-pyrrolyl propyl, chloroquinolinylmethyl, 2-thienylethyl, pyridylmethyl, 1-imidazolylethyl and the like.
"Halogen" and "halo", alone or in combination, means fluoro, chloro, bromo or iodo radicals.
"Haloalkyl", alone or in combination, means an alkyl radical as defined above in which at least one hydrogen atom, preferably 1-3, is replaced by a halogen radical, more preferably fluoro or chloro radicals. Examples of such haloalkyl radicals include 1,1,1-trifluoroethyl, chloromethyl, 1-bromoethyl, fluoromethyl, difluoromethyl, trifluoromethyl, bis(trifluoromethyl)methyl and the like.
"Pharmacologically acceptable salt" means a salt prepared by conventional means, and are well known by those skilled in the art. The "pharmacologically acceptable salts" include basic salts of inorganic and organic acids, including but not limited to hydrochloric acid, hydrobromic acid, sulphuric acid, phosphoric acid, methanesulphonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. When compounds of the invention include an acidic function such as a carboxy group, then suitable pharmaceutically acceptable cation pairs for the carboxy group are well known to those skilled in the art and include alkaline, alkaline earth, ammonium, quaternary ammonium cations and the like. For additional examples of "pharmacologically acceptable salts," *see infra* and Berge et al, J. Pharm. Sci. 66, 1 (1977).
"Leaving group" (refered to as "L" in the Schemes) generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.
"Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, orthomethylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, tri-fluoroacetyl, tri-chloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluenesulfonic acid, trifluoroacetic acid and the like. Many of the amino protecting groups are also suitable for protecting carboxy, hydroxy and mercapto groups. For example, aralkyl groups. Alkyl groups are also sutiable groups for protecting hydroxy and mercapto groups, such as tert-butyl.

Silyl protecting groups are silicon atoms optionally substituted by one or more alkyl, aryl and aralkyl groups. Suitable silyl protecting groups include, but are not limited to, trimethylsilyl, triethylsilyl, tri-isopropylsilyl, tertbutyldimethylsilyl, dimethylphenylsilyl, 1,2-bis(dimethylsilyl)benzene, 1,2-bis(dimethylsilyl)ethane and diphenylmethylsilyl. Silylation of an amino groups provide mono- or di-silylamino groups. Silylation of aminoalcohol compounds can lead to a N,N,O-tri-silyl derivative. Removal of the silyl function from a silyl ether function is readily accomplished by treatment with, for example, a metal hydroxide or ammonium flouride reagent, either as a discrete reaction step or in situ during a reaction with the alcohol group. Suitable silylating agents are, for example, trimethylsilyl chloride, tert-buty-dimethylsilyl chloride, phenyldimethylsilyl chloride, diphenylmethyl silyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A t-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, tert-butyl, 4-methoxyphenylmethyl and the like, can be removed under hydroylsis and hydrogenolysis conditions well known to those skilled in the art.

The symbols used above have the following meanings:

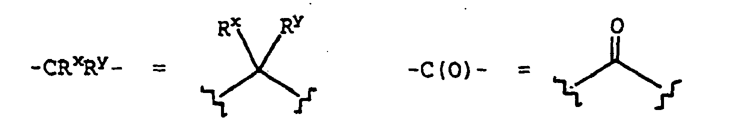

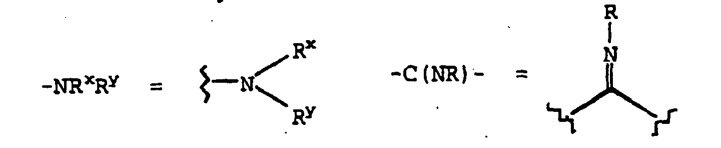

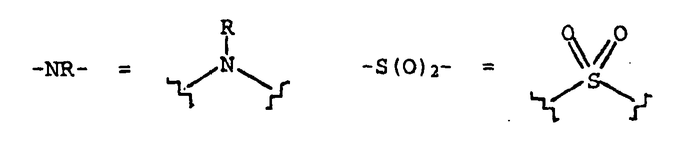

Prodrugs of the compounds of this invention are also contemplated by this invention. A prodrug is an active or inactive compound that is modified chemically through in vivo physicological action, such as hydrolysis, metabolism and the like, into a compound of this invention following adminstration of the prodrug to a patient. The suitability and techniques involved in making and using prodrugs are well known by those skilled in the art. For a general discussion of prodrugs involving esters see Svensson and Tunek Drug Metabolism Reviews 165 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bungaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxy groups have been masked as esters and ethers. EP 039,051 (Sloan and Little, 4/11/81) discloses Mannich-base hydroxamic acid prodrugs, their preparation and use.

Compounds according to the invention can be synthesized according to one or more of the following methods. It should be noted that the general procedures are shown as it relates to preparation of compounds having unspecified stereochemistry. However, such procedures are generally applicable to those compounds of a specific stereochemistry, e.g., where the stereochemistry about a group is (S) or (R). In addition, the compounds having one stereochemistry (e.g., (R)) can often be utilized to produce those having opposite stereochemistry (i.e., (S)) using well-known methods, for example, by inversion.

The invention relates to substituted pyridines or pyridazines which are useful for the treatment of inflammtory disease and diseases in which IL-1 and TNF play a role. Substituted pyridines and pyridazines embodied in the current invention may be prepared as described in the following schemes and synthetic examples.

Pyridines of Formula I wherein X = C-H and J = N may be prepared utilizing the chemistry outlined in Schemes 1 through 3. As shown in Scheme 1, The R₁₂ and R₁₁ substituents are conveniently introduced from the alcohol and aldehyde precursors to provide dione III. 3,4-substituted pyridones VIIIa and VIIIb may be prepared from cyclopentenones IV and V, respectively, via Beckmann rearrangement and acetate elimination on the intermediate oximes (one isomer represented by VII). 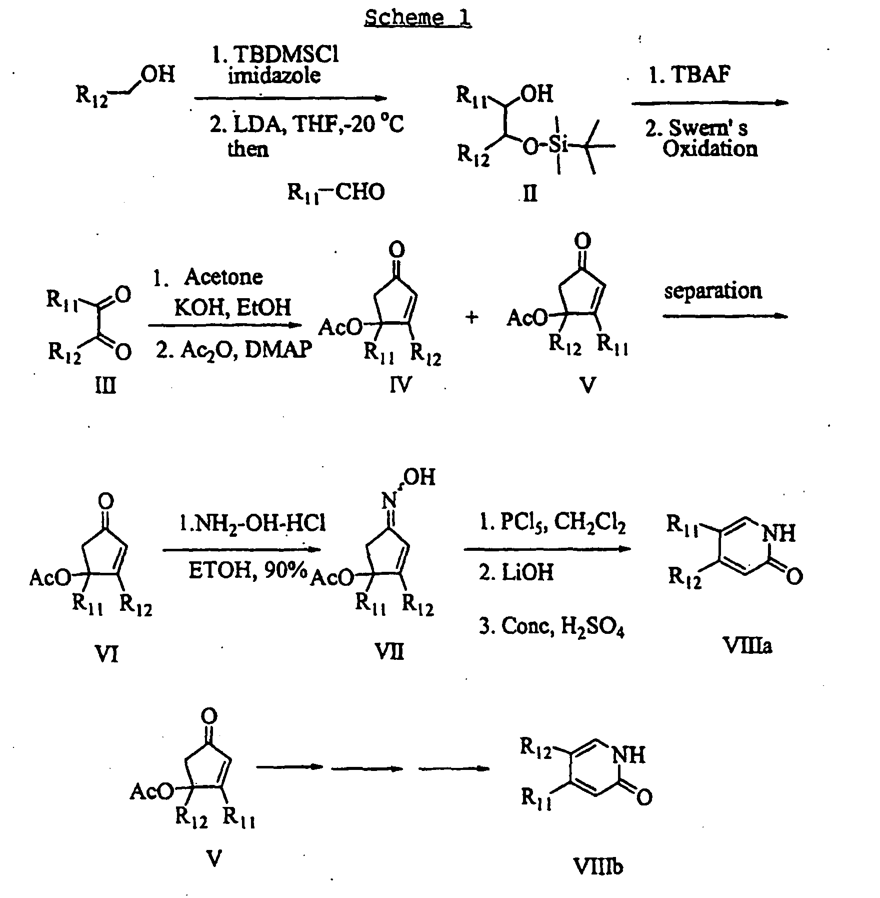 Pyridones VIIIa and VIIIb may be further modified by reaction with POCl₃ or SO₂Cl₂, as shown in Scheme 2, to form the intermediate 2-chloropyridine which can be used in a variety of displacement reactions with HNR₅R₂₁, or HOR₂₁, or HSR₂₁ in the presence or absence of base at temperatures from 25°C to 250°C, or carbon bound substituents may be introduced using palladium or nickel catalyzed cross coupling reactions with aryl or alkyl boronic acids, aryl or alkyl stannanes, or aryl or alkyl zinc halides to form compounds of Formula I.
Intermediate 2-chloropyridines may be converted to 2-bromopyridines, which are more preferable as partners in palladium or nickel catalyzed cross coupling reactions, by reaction with HBr in HOAc. Furthermore, pyridones VIIIa and VIIIb may be alkylated with an alkyl halide, mesylate, tosylate or the like, in the presence or absence of base, or may be alkylated with an alcohol under Mitsunobu conditions (Ph,P, dialkylazo-dicarboxylate) to provide compounds of Formula I wherein X = C-H, J = N, and W = -OR₂₁.

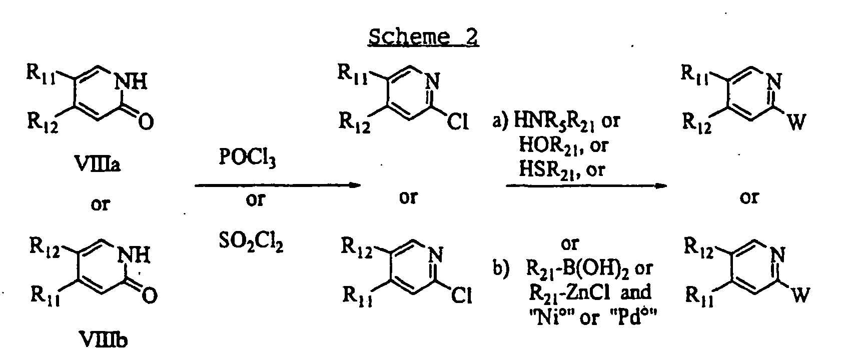

An alternative general route to compounds of formula I wherein X = C-H and J = N is shown in Scheme 3. 4-substituted pyridine IX can be converted to the N oxide X by reaction with an oxidizing agent such as peroxides, peracids, or oxone, followed by treatment with POCl₃ to afford XI. Treatment of XI. with an amine, alcohol, or sulfide in the presence or absence of a base at a temperature from 25°C to 250°C affords XII which is subsequently halogenated by treatment with an appropriate halogenating reagent such as Br₂ to afford XIII. Introduction of an R₁₁ or R₁₂ substituent to XIII may be performed as shown, utilizing an aryl or heteroaryl or "N"-heteroaryl boronic acid, or alternatively, utilizing a corresponding stannane or corresponding zinc halide in the presence of an appropriate palladium or nickel catalyst in an aprotic solvent to provide XIV.

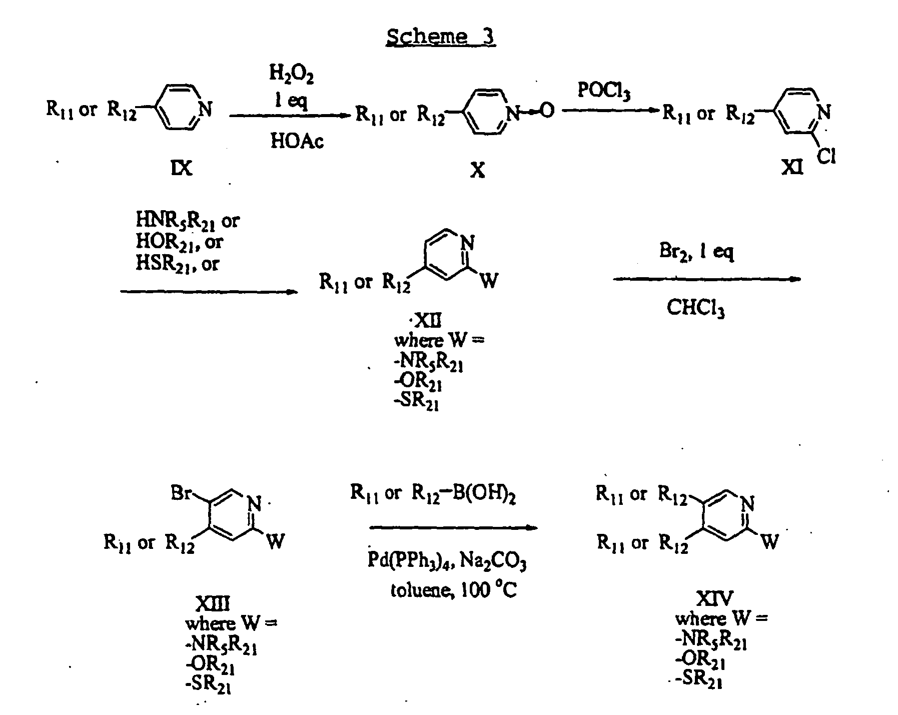

Pyridines of Formula I, wherein X = N and J = C-R₁ may be prepared as described in shown in Schemes 4 - 6. As shown in Scheme 4, 2,6-disubstituted pyridines XVII may be prepared from 2,6-dibromopyridine XV via a metal catalyzed cross coupling reaction with an appropriate coupling partner and displacement with an appropriate nucleophile.

Another method of preparing intermediate XVII is shown in Scheme 5. The cuprate derived from bromide, IXX, is reacted with N-ethoxycarbonylpyridinium chloride to provide an intermediate dihydropyridine which is oxidized in the presence of O₂ affording XX. Debenzylation, and reaction of the intermediate pyridone with POCl₃ provides 2-chloropyridine XXI, which may may be converted to XVII as described above and shown in the Scheme. 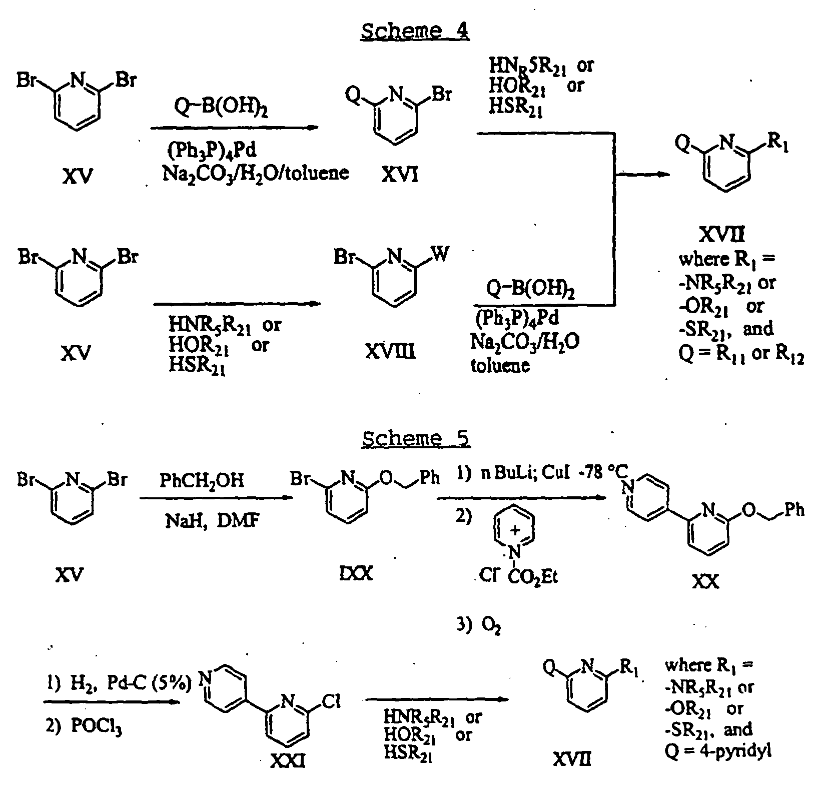 Elaboration of 2,6-disubstitutedpyridines XVII to provide compounds of Formula I wherein X = N, and J = C-R₁ is shown in Scheme 6. Bromination of XVII provides an intermediate bromopyridine (not shown) which upon reaction with an aryl or heteroaryl or "N"-heteroaryl boronic acid, or a corresponding organostannane or organozinc halide in the presence of an appropriate palladium or nickel catalyst in an aprotic solvent affords XXII. Introduction of R₂ substituents (W = C-R₁) may be accomplished by bromination of XXII providing a versatile intermediate, XXIII for the preparation of XXIV. For example, a) aryl or alkyl groups may be introduced by Pd or Ni catalyzed cross coupling reactions with appropriate boronic acids or organozinc reagents; b) acyl groups are readily introduced by reaction with acid chlorides in the presence of Pd catalysts, and; c) cyano groups may be introduced by the action of CuCN in pyridine. 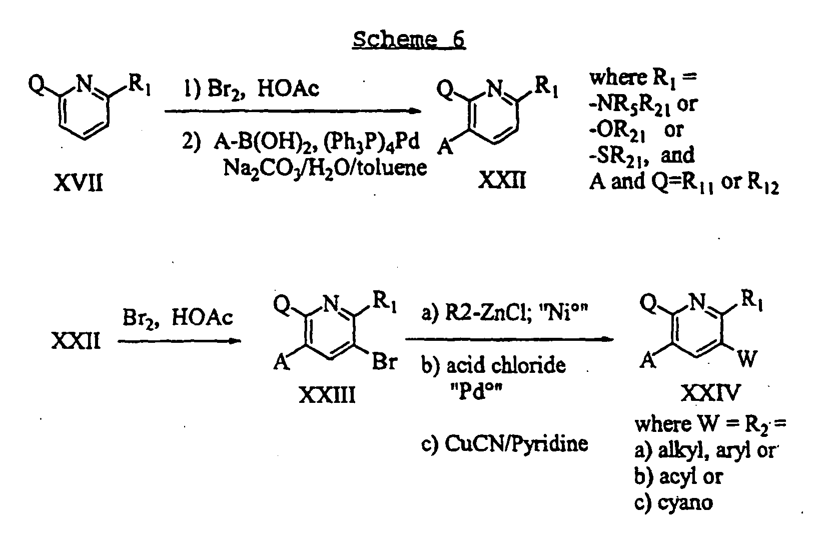 Pyridines of Formula I, wherein X = N, J = C-R₂ and W = R₁ may be prepared as described in shown in Schemes 7 and 8. 2-Chloro-3-bromo-5-carbomethoxypyridine XXIX may be prepared as described in J. Org. Chem., (1984), 49(26), pp. 5237-5247. Hydrolysis of XXIX followed by coupling of the intermediate pyridone with an appropriate boronic acid and subsequent esterification provides XXX (Scheme 7). Conversion of the pyridone to the intermediate 2-chloropyridine may be performed by treatment with POCl₃ or SO₂Cl₂. Treatment with an appropriate boronic acid, organostannane or organozinc reagent in the presence of Pd or Ni catalysis provides XXXI.

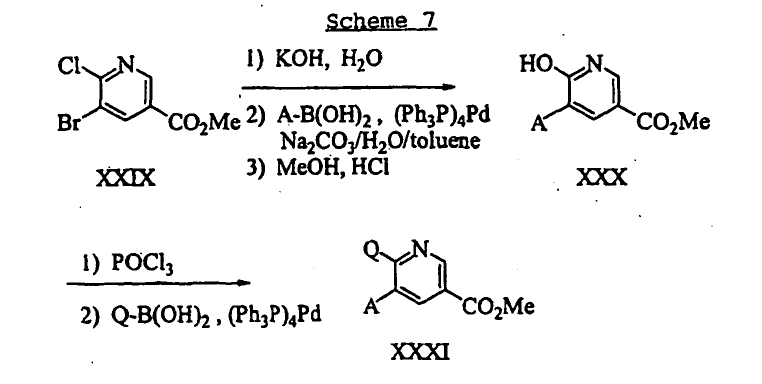

Scheme 8 illustrates the conversion of XXXI to the amine XXXII via a modified curtius reaction (Ninomiya, K, et. al., Tetrahedron (1974) 30(14):2151-2157). Compounds of formula I wherein W = R₁ = NH-R₂₁ are prepared by reductive alkylation to provide XXXIII.

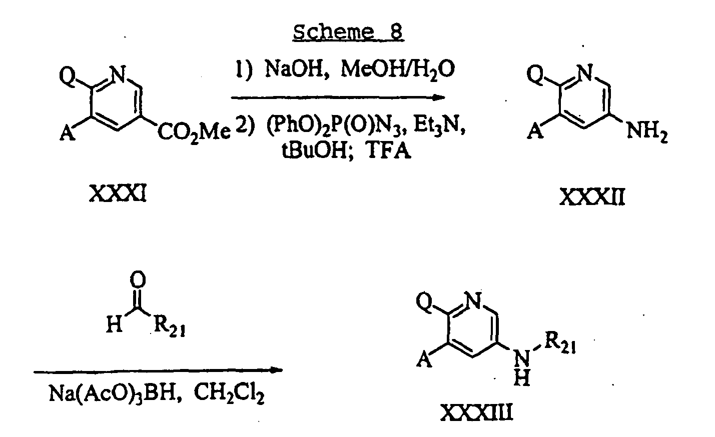

A widely applicable method for the preparation of pyridazines involves the condensation of a 1,4-dicarbonyl compound with hydrazine (Scheme 9). An oxidative step is required to give the aromatic pyridazine unless the carbonyl component is unsaturated.

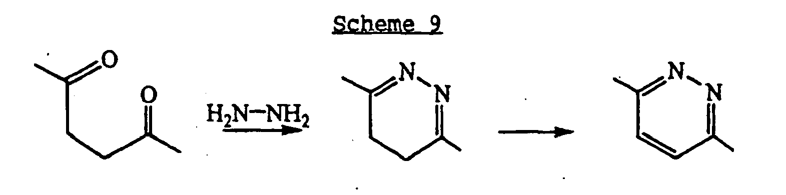

Thus, a 4-keto carbonic acid or - ester may be reacted with hydrazine to give a dihydropyridazinone which may be dehydrogenated by a bromination-dehydrobromination step or by using sodium 3-nitrobenzenesulfonate as an oxidant (Scheme 10) (e.g. Th. Curtius, J. Prakt. Chem. 50, 509, 1894; Gabriel and Colman, Chem. Ber. 32,395, 1899; D. Libermann and A. Rouaix, Bull. Soc. Chim. Fr. 117, 1959; E. Ravina et al., Arch. Pharm. (Weinheim) 324, 455, 1991).

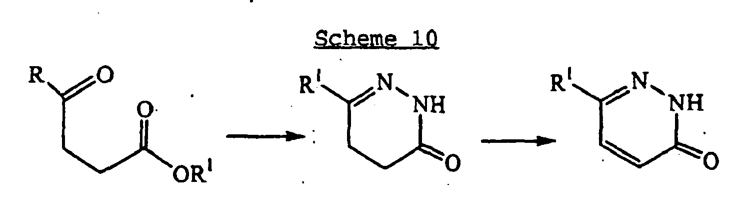

This approach allows the preparation of 5,6-disubstituted 2H-pyridazin-3-ones by using the corresponding 3,4-disubstituted 4-keto butyric acid or - ester as demonstrated in Scheme 11 (Almstroem, Just. Lieb. Ann. Chem. 400, 137, 1913; E. Ravina et al., Eur. J. Med. Chem.-Chim. Ther.20, 475, 1985; E. Ravina et al., Arch. Pharm. (weinheim), 324, 455, 1991):

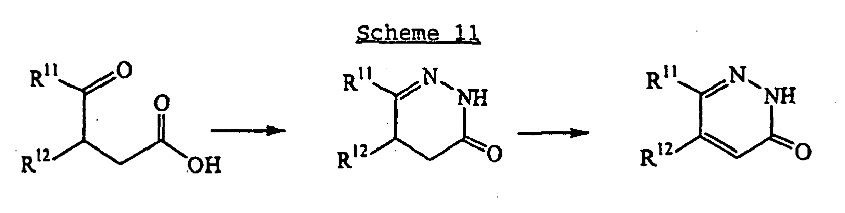

In a related approach (Scheme 12) that does not require an oxidation step, glyoxylic acid may be reacted with a methylen ketone in a thermic condensation reaction to give a disubstituted 5-hydroxy-2(5H)-furanone. Reaction of this intermediate with hydrazine then may lead directly to the disubstituted pyridazinone (C.-G. Wermuth et al., J.Med.Chem. 30, 239, 1987):

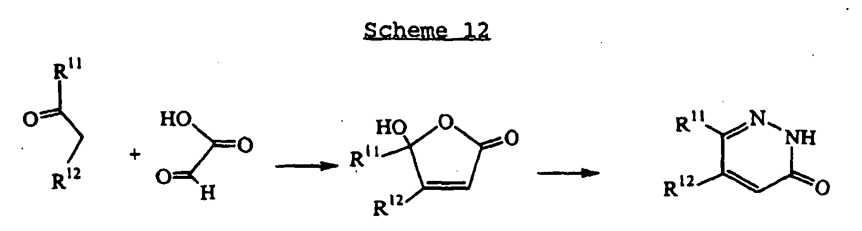

2H-Pyridazin-3-ones can easily be converted into 3-chloropyridazines (Scheme 13) by treatment with e.g. phosphorus oxychloride at elevated temperature (e.g. Gabriel and Colman, Chem. Ber. 32,395, 1899; D. Libermann and A. Rouaix, Bull. Soc. Chim. Fr. 117, 1959; E. Ravina et al. Arch. Pharm. (Weinheim), 324, 455, 1991; F. Khalifa, Arch. Pharm. (Weinheim) 323, 883, 1990)). The 3-chloropyridazine represents a versatile intermediate for nucleophilic substitution reactions with e.g. primary or secondary amines (e.g. E. Ravina, Arch. Pharm.(weinheim) 324, 455 (1991)).

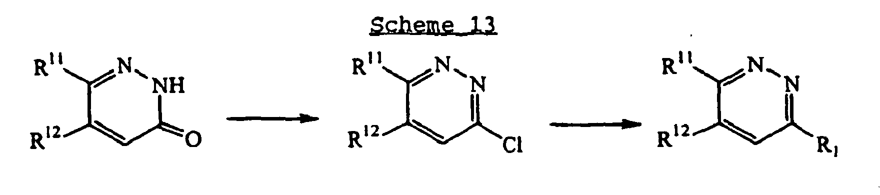

Furthermore, the 3-chloropyridazine may also be subjected to palladium or nickel catalyzed cross coupling reactions with aryl boronic acids or arylzinc halides to provide compounds wherein the 3-substituent is an aryl or heteroaryl (e.g. A. Turck et al. Bull. Soc. Chim. Fr. 130, 488, 1993).

A synthesis leading to 6-substituted-3-(4-fluorophenyl)-4-(4-pyridyl)-pyridazines XL is displayed in Scheme 14. Ketone XXXIV (P. J. Gilligan et al., J. Med. chem. 35, 4344, 1992) may be alkylated with ethyl bromoacetate in the presence of sodium ethoxide (E. Knoevenagel, Chem. Ber. 21, 1344, 1888) to give the ketoester XXXV. Cyclization with hydrazine monohydrate to give the dihydropyridazinone XXXVI is followed by a bromination-dehydrobromination step using bromine in acetic acid and leading to (2H)-pyridazin-3-one XXXVII.

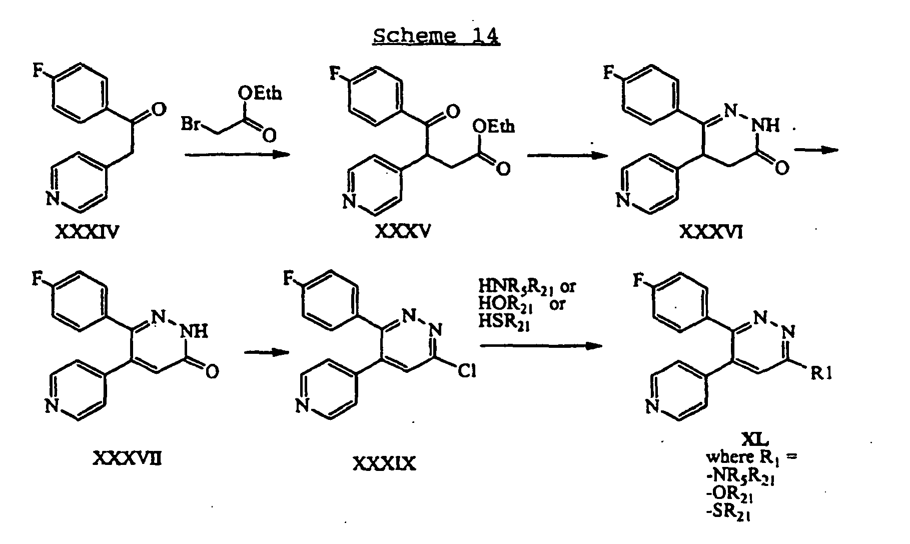

XXXVII may be converted into the chloro derivative XXXIX by treatment with a chlorinating agent such as phosphorus oxychloride at elevated temperature. Treatment of XXXIX with an amine, alcohol, or sulfide in the presence or absence of a base at a temperature from 25°C to 250°C yields XL.

Substituted halopyridines may be readily prepared from the corresponding pyridones using phosphorus oxychloride or pentachloride.

Amines of formula NHR₅R₂₁ and NHR₃₁R₃₂ are commercially available or can be readily prepared by those skilled in the art from commercially available starting materials. For example, an amide, nitro or cyano group can be reduced under reducing conditions, such as in the prescence of a reducing agent like lithium aluminum hydride and the like, to form the corresponding amine. Alkylation and acylation of amino groups are well known in the art. Chiral and achiral substituted amines can be prepared from chiral amino acids and amino acid amides (for example, alkyl, aryl, heteroaryl, cycloalkyl, arylalkyl, heteroarylalkyl, cycloalkylalkyl and the like) using methods well known in the art, such as H. Brunner, P. Hankofer, U. Holzinger, B. Treittinger and H. Schoenenberger, Eur. J. Med. Chem. 25, 35-44, 1990; M. Freiberger and R. B. Hasbrouck, J. Am. Chem. Soc. 82, 696-698, 1960; Dornow and Fust, Chem. Ber. 87, 984, 1954; M. Kojima and J. Fujita, Bull. Chem. Soc. Jpn. 55, 1454-1459, 1982; W. Wheeler and D. O'Bannon, Journal of Labelled Compounds and Radiopharmaceuticals XXXI, 306, 1992; and S. Davies, N. Garrido, O. Ichihara and I. Walters, J. Chem. Soc., Chem. Commun. 1153, 1993.

Alkyl sulfonic acids, aryl sulfonic acids, heterocyclyl sulfonic acids, heteroaryl sulfonic acids, alkylmercaptans, arylmercaptans, heterocyclylmercaptans, heteroarylmercaptans, alkylhalides, arylhalides, heterocyclylhalides, heteroarylhalides, and the like are commercially available or can be readily prepared from starting materials commercially available using standard methods well known in the art.

Thioether derivatives can be converted into the corresponding sulfone or sulfoxide by oxidizing the thioether derivative with a suitable oxidation agent in a suitable solvent. Suitable oxidation agents include, for example, hydrogen peroxide, sodium meta-perborate, oxone (potassium peroxy monosulfate), meta-chloroperoxybenzoic acid, periodic acid and the like, including mixtures thereof. Suitable solvents include acetic acid (for sodium meta-perborate) and, for other peracids, ethers such as THF and dioxane, and acetonitrile, DMF and the like, including mixtures thereof.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily prepared from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. The following Examples are presented for illustrative purposes only and are not intended, nor should they be construed, as limiting the invention in any manner. Those skilled in the art will appreciate that modifications and variations of the compounds disclosed herein can be made without violating the spirit or scope of the present invention.

### Example 1

The following amines were prepared as intermediates and used to obtain compounds claimed within the scope of this invention.

### Example 1A: Procedure for the preparation of 3-phenylbutylamine

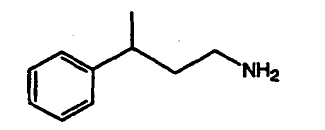

A mixture of 3-phenylbutyraldehyde (3 ml, 20.18 mmol), ammonium acetate (15 g, 195 mmol) and sodium cyanoborohydride (900 mg, 14.32 mmol) in methanol (50 ml) was stirred overnight under an argon atmosphere. The reaction was acidified to pH 2 by the addition of conc HCl. The solvent was evaporated, dichloromethane and water were added, and the aqueous layer was made basic (pH 12) by the addition of solid potassium hydroxide. Extraction (dichloromethane) and concentration gave the title compound as an oil. ES-MS (m/z) : 150.2 (M+H)⁺; ¹H NMR (CDCl₃) : d 7.40-7.17 (m, 5H, Ph), 2.81 (q, 1H, CH), 2.62 (m, 2H, CH₂), 1.76 (dq, 2H, CH₂), 1.29 (d, 3H, CH₃).

### Example 1B: Procedure for the preparation of 3-(2-methylphenyl)propylamine

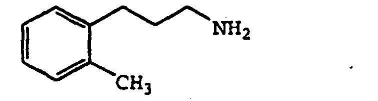

Diethyl cyanomethylphosphonate (5.0 ml, 30.9 mmol) was added to a stirring suspension of sodium hydride (60% oily suspension, 1.24 g, 31 mmol) in tetrahydrofuran (50 ml) under argon. After 30 min, 2-methylbenzaldehyde (3.6 ml, 31.1 mmol) was added and stirring continued for 1 h. The reaction was quenched by the addition of water and extracted with dichloromethane followed by drying and evaporation of the organic solution. Column chromatography (hexane; hexane : ethylacetate = 3 : 1) provided 2-(2-methylphenyl)acrylonitrile as an oil. This material (3.8 g), 10% palladium on carbon (3.8 g) and 12 N hydrochloric acid (11.8 ml, 142 mmol) in methanol (125 ml) were hydrogenated with hydrogen at atmospheric pressure for 2 d. The catalyst was removed by filtration and the solvent was evaporated. The resultant material was partitioned between dichloromethane and water. The aqueous layer was made basic with 10 N sodium hydroxide and extracted with dichloromethane, followed by drying and evaporation. The resultant material was purified on a silica gel column (chloroform : methanol : triethylamine = 85 : 10 : 5) to provide the title compound as an oil.

### Example 1C: Procedure for the preparation of 2-Methyl-3-phenylpropylamine

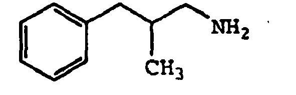

A mixture of commercially available 2-methyl-3-phenylpropylamide (4.32 g, 26.5 mmol) and lithium aluminium hydride (1.3 g, 34.3 mmol) in tetrahydrofuran (184 ml) was stirred at room temperature for 5 h. The reaction mixture was poured into saturated aqueous sodium sulfate and extracted with dichloromethane followed. The combined organic extracts were dried (sodium sulfate) and evaporated to provide the amine as an oil. For alternative preparations see: Dornow and Fust, Chem. Ber. 87, 984 (1954).

### Example 1D: Procedure for the preparation of 3-Fluoro-3-phenylpropylamine

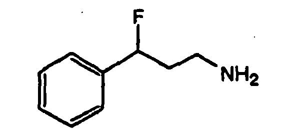

Step A. 3-Hydroxy-3-phenylpropionitrile: Sodium borohydride (1.4 g, 37.00 mmol) was added in portions to a stirring solution of benzoylacetonitrile (10 g, 68.90 mmol) in methanol (200 ml) at ice-bath temperature. After 30 min, the reaction was quenched by the addition of a few drops of acetic acid followed by evaporation. The mixture was partitioned between dichloromethane and water and the combined organic extracts were dried (magnesium, sulfate) and evaporated to provide the Step A compound as a syrup. (cf. Florin, C.; Chantegrel, J.; Charlon, C.; Marsura, A.; Luu-Duc, C. Nouvelle voie de synthese des a-fluorophenylacetonitriles. Ann. pharmaceuttiques fr. 1985, 43, 595-599.)
Step B. 3-Fluoro-3-phenylpropionitrile: A solution of 3-hydroxy-3-phenylpropionitrile (3.5 g, 23.8 mmol) in dichloromethane (20 ml) was added at -78 °C to a stirred solution of diethylaminosulfur trifluoride (5g, 31 mmol) in dichloromethane (23 ml). After 1.5 h, the mixture was allowed to reach room temperature. The reaction was quenched by the addition of water, followed by extraction with dichloromethane, drying of the organic phase and evaporation. Flash chromatography on a column of silica gel (hexane-ethyl acetate = 5:1) provided 3-fluoro-3-phenylpropionitrile. ¹H NMR (CDCl₃) : d 7.50-7.29 (m, 5H, Ph), 5.73 (dt, 1H, *J_{H.}* 46.2 Hz, CHF), 3.00 and 2.96 (dd, t, each 1H, CH₂).
Step C. 3-Fluoro-3-phenylpropylamine: A 2N borane-dimethyl sulfide complex solution in tetrahydrofuran (8.8 ml, 17.6 mmol) was added dropwise at room temperature to a stirred solution of 3-fluoro-3-phenylpropionitrile (2 g, 13.41 mmol) in tetrahydrofuran (12 ml). The mixture was warmed to 50°C, the dimethylsulfide was distilled off, and the mixture was then refluxed for 2.5 h. After cooling to 0 °C, 1N methanolic hydrogen chloride (20 ml) was added, and the mixture was concentrated. To the resulting concentrate was added dichloromethane and water, and solid potassium hydroxide was added to acheive a pH of aproximately 12. Extraction (dichloromethane) and concentration gave the crude product as a mixture of phenylpropylamine and 3-fluoro-3-phenylpropylamine. Column chromatography on a column of Iatrobeads^{R} (chloroform-methanol-triethylamine = 90:7:3) provided the title compound 3-fluoro-3-phenylpropylamine in the first fraction. ES-MS *(m*/*z):* 154.0 (M+H)⁺; ¹H NMR (CDCl₃) : d 7.45-7.28 (m, 5H, Ph), 5.60 (ddd, 1H, *J_{H, F}* 48.2 Hz, CHF), 2.91 (t, 2H, CH₂N), 2.15 and 1.96 (2m, each 1H, CH₂).

### Example 1E: Procedure for the preparation of 2-Fluoro-3-phenylpropylamine

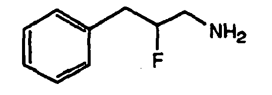

Step A. 1-Azido-2-hydroxy-3-phenylpropane: A mixture of (2,3-epoxypropyl)benzene (9.69 g, 72.22 mmol), sodium azide (16.5 g, 253.8 mmol) and ammonium chloride (6.3 g, 109.5 mmol) in methanol (190 ml) and water (32 ml) was heated at reflux for 1.5 h. The solvent was evaporated, the remainder was partitioned between dichloromethane and water. The organic solution was dried and evaporated to give the Step A compound as an MS *(m*/*z):* 178.1 (M+H)⁺; ¹H NMR (CDCl₃): d 7.43-7.15 (m, 5H, Ph), 4.08 (m, 1H, CH), 3.41 and 3.32 (2dd, each 1H, CH₂), 2.85 and 2.83 (2d, each 1H, CH₂), 1.98 (bs, OH).
Step B. 1-Azido-2-fluoro-3-phenylpropane: A solution of 1-azido-2-hydroxy-3- phenylpropane (3.5 g, 19.75 mmol) in dichloromethane (23 ml) was added at -78 °C to a stirred solution of diethylaminosulfur trifluoride (3.4 ml, 25.74 mmol) in dichloromethane (23 ml). The mixture was slowly warmed to room temperature over 2.5 h. The reaction was quenched by the addition of water, and extracted with dichloromethane. Concentration and prification by flash chromatography on a column of silica gel (hexane-ethyl acetate= 8:1 to 6:1:1) provided 1-Azido-2-fluoro-3-phenylpropane as an oil. ¹H NMR (CDCl₃): d 7.46-7.20 (m, 5H, Ph), 4.86 (m, 1H, *J_{H,F}* 48.2 Hz, CHF), 3.41 (m, 2H, CH₂), 3.04 (m, 2H, CH₂).
Step C. 2-Fluoro-3-phenylpropylamine: A mixture of 1-azido-2-fluoro-3-phenylpropane (900 mg, 5.0 mmol) and 20% palladium-on-carbon (wet, 50%, 500 mg) in methanol (40 ml) was hydrogenated under a balloon of hydrogen for 2 h. The catalyst was removed by filtration and the solvent was evaporated. The resultant product was purified on a short column of Iatrobeads^{R} (chloroform-methanol-triethylamine = 90:7:1) to provide the title compound as an oil. ES-MS (m/z): 153.9 (M+H)⁺; ¹H NMR (CDCl₃) : d 7.40-7.22 (m, 5H, Ph), 4.68 (m, 1H, *J*_{*H*,F} 48.7 Hz, CHF), 3.11-2.83 (m, 4H, 2CH₂).

### Example 1F: Procedure for the preparation of 2-amino-3-(2-fluorophenyl)-propylamine

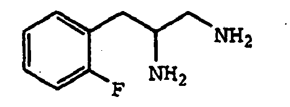

Step A. Methyl 2-amino-3-(2-fluorophenyl)propionate: 5g (27.3 mmol) of (D,L)-(2-fluoro-phenyl)alanine was suspended in 50 ml methanolic HCl and stirred at room temperature for 3 days. The reaction mixture was concentrated in vacuo and dried to give a yellow oil. MS (m/z): 198 (M+H)⁺; C₁₀H₁₂FNO₂ requir. 197.2.
Step B. 2-Amino-3-(2-fluorophenyl)propionamide: Methyl 2-amino-3-(2-fluorophenyl) propionate was suspended in 50 ml 30% ammonium hydroxide and stirred at room temperature for 18 hrs. The mixture was filtered, washed with cold water and 2-amino-3-(2-fluorophenyl) propionamide was collected as a white solid. MS (m/z): 183.1 (M+H)⁺ ; C₉H₁₁FN₂O requir. 182.2.
Step C. 2-Amino-3-(2-fluorophenyl)-propylamine: 2-Amino-3-(2-fluorophenyl)propionamide was added carefully to a chilled (5°) mixture of LAH (1.0g, 26.3 mmol) and 20 ml THF under argon. The reaction was then heated at reflux for 10 hrs. The reaction was cooled to 5°C and carefully treated with Na₂SO₄·10 H₂O. The resulting mixture was stirred for 18 hrs, then filtered to remove the solids. The filtrate was concentrated in *vacuo* to give an amber oil. MS (m/z): 169 (M+H)⁺ ; C₉H₁₃FN₂ requir. 168.19

### Example 1G: Procedure for the preparation of 2-Amino-2-methyl-3-phenylpropylamine

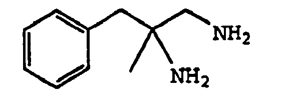

Step A: D.L-α-methyl phenylalanine amide: A solution of commercially available D,L-α-methyl phenylalanine methyl ester (5.0 g, 25.7 mmol) in aqu. 28% ammonium hydroxide (50 ml) was kept at room temperature for 3 d. The resulting white precipitate of D,L-α-methyl phenylalanine amide was filtered and dried.
Step B: 2-Amino-2-methyl-3-phenylpropylamine: D,L-α-methyl phenylalanine amide (2.0 g, 11.22 mmol) was reduced with lithium aluminium hydride (1.3 g, 34.26 mmol) in boiling tetrahydrofuran for 24 h. The reaction was quenched by the addition of sodium sulfate decahydrate at ice-bath temperature. The salts were filtered off, followed by evaporation to leave the title compound as an oil. MS (m/z): 165.1 (M+H)⁺; C₁₀H₁₆N₂ requir. 164.2. An alternative preparation was reported by M. Freiberger and R. B. Hasbrouck, J. Am. Chem. Soc. 82, 696-698 (1960).

### Example 1H: Procedure for the preparation of (S)-1,2-benzylethylenediamine

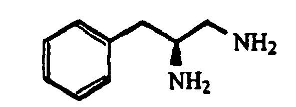

(S)-1,2-Benzylethylendiamine was prepared according to the literature (H. Brunner, P. Hankofer, U. Holzinger, B. Treittinger and H. Schoenenberger, Eur. J. Med. Chem. 25, 35-44, (1990)) by reduction of L-phenylalanine amide with lithium aluminium hydride. The (R)-enantiomer was prepared in the same manner from D-phenylalanine amide.

### Example 1I: Procedure for the preparation of (S)-2-N,N-Dimethylamino-3-phenylpropylamine

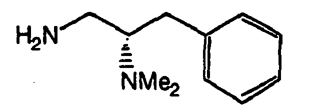

Sodium triacetoxyhydride (13.0 g, 61.3 mmol) was added to a stirring mixture of phenylalanine amide (3.6 g, 21.9 mmol) and 37% formaldehyde solution (4.4 ml, 58.7 mmol) in 1,2-dichloroethane (77 ml). After stirring for 2 h, the reaction was quenched by the addition of sat. aqu. sodium hydrogencarbonate. Then potassium hydroxide pellets were added followed by extraction with dichloromethane, drying of the organic solution and evaporation. The resulting (*S*)-2-*N,N*-dimethylamino-3-phenylpropylamide was reduced with lithium aluminium hydride according to the literature (H. Brunner, P. Hankofer, U. Holzinger, B. Treittinger and H. Schoenenberger, Eur. J. Med. Chem. 25, 35-44, (1990)) to provide the title compound.

### Example 1J: Procedure for the preparation of (S)-2-N-Ethylamino-3-phenylpropylamine

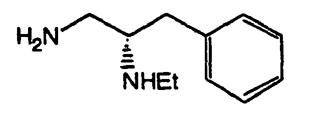

(S)-2-N-Ethylamino-3-phenylpropylamine: Acetic anhydride (1.2 ml, 12.7 mmol) was added to a stirring solution of L-phenylalanine amide (1.0 g, 6.10 mmol) in methanol (25 ml). After 1.5 h at room temperature, it was evaporated followed by drying in an oil pump vacuum. The resultant L-N-ethylphenylalanine amide (6.1 mmol) was reduced with lithium aluminium hydride (570 mg, 15.0 mmol) in tetrahydrofuran (65 mml) at 55°C for 4 h. The reaction mixture was poured into sat. aqu. sodium hydrogencarbonate followed by extraction with dichloromethane, drying and evaporation. Column chromatography on silica gel (chloroform : methanol : triethylamine = 90:7:3) provided the amine as a yellowish oil. MS (m/z): 179.1 (M+H)⁺; C₁₁H₁₈N₂ requir. 178.3.

### Example 1K: Procedure for the preparation of (S)-2-Benzylpiperazine

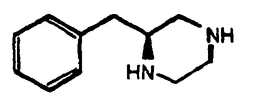

Lithium aluminium hydride (1.6 g, 42.16 mmol) was added in portions to a stirred mixture of (S)-2-benzyl piperazine-3,6-dione (3.0 g, 14.70 mmol) and tetrahydrofuran (80 ml) at 0 °C. After 30 min at ice-bath temperature, the mixture was refluxed for 4 h with stirring. The reaction was quenched by the portionwise addition of sodium sulfate decahydrate and some methanol until hydrogen evolution ceased. It was filtered and the solids were washed several times with dichloromethane. The combined filtrates were evaporated to leave a white solid. MS *(m*/*z):* 177.1 (M+H)⁺; C₁₁H₁₆N₂ requir. 176.3.

### Example 1L: Procedure for the preparation of ((S) - 1,2,3,4-tetrahydroisoquinolin-3-ylmethyl)amine

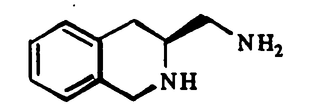

The title compound was obtained from the reduction of (S)-decahydroquinoline-3-carboxamides according to the procedure set forth in Example 1c. Alternatively the title compound may be prepared from (S)-decahydroquinoline-3-carboxylic acid using the procedures described in Example 1f.

### Example 1M: Procedure for the preparation of 1-Phenyl-1,3-propanediamine

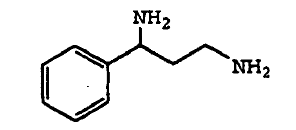

3-Phenyl-3-aminopropionic acid (S. G. Cohen and S. Y. Weinstein, J. Am. Chem. Soc. 86, 725-728, 1964) was converted into 1-phenyl-1,3-propanediamine as reported in the literature (M. Kojima and J. Fujita, Bull. Chem. Soc. Jpn. 55, 1454-1459 (1982)). 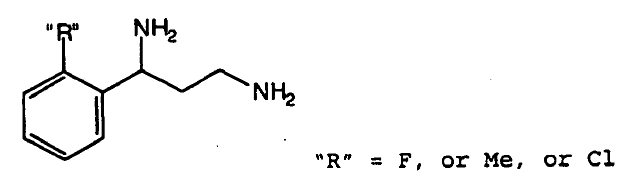 Analogously, 1-(2-fluorophenyl)-1,3-propanediamine. 1-(2-methylphenyl)-1,3-propanediamine and 1-(2-chlorophenyl)-1,3-propanediamine were prepared by using the above procedure and the appropriately substituted 3-phenyl-3-aminopropionic acid.

### Example 1N: Procedure for the preparation of (S)-1-Phenyl-1,3-propanediamine

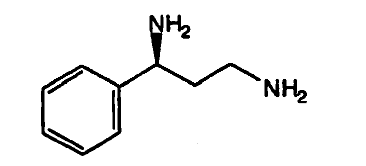

S-3-N-*tert*.--Butoxycarbonylamino-3-phenylpropionitrile was prepared according to the literature (W.J. Wheeler and D.D. O'Bannon, J. Label.Compds. Radiopharm. XXXI (4), 305-315, 1992) from D-(-)-α-phenylglycinol. For reduction (D. Mitchell and T.M. Koenig, Synth. Comm. 25 (8), 1231-1238, 1995), borane-methyl sulfide complex (2N, 3 ml, 6 mmol) was added dropwise to a solution of the nitrile (1 g, 4.06 mmol) in tetrahydrofuran (6 ml). Methyl sulfide was distilled off and the resulting solution refluxed for 2.5 h. With ice-cooling, methanolic hydrogen chloride (1N, 3 ml) was added followed by evaporation. The remainder was taken up in methanol (10 ml) and 4N hydrogen chloride/dioxane (10 ml) was added. After 1 h at room temperature, it was evaporated and the aqueous solution of the resultant product was washed with dichloromethane. The aqueous solution was made basic by the addition of solid potassium hydroxide followed by repeated dichloromethane extractions. Drying and evaporation of the dichloromethane solution left the crude diamine as an oil. MS (m/z): 150.8 (M+H)⁻: C₉H₁₄N₂ requir. 150.2. The enantiomer, (R)-1-phenyl-1,3-propanediamine, was prepared analogously from L-(+)-α-phenylglycinol. MS (*m*/*z*)*:* 150.9 (M+H)⁺; C₉H₁₄N₂ requir. 150.2.

### Example 10: Procedure for the preparation of (1R,2R)-2-methyl-1-phenyl-1,3-propanediamine

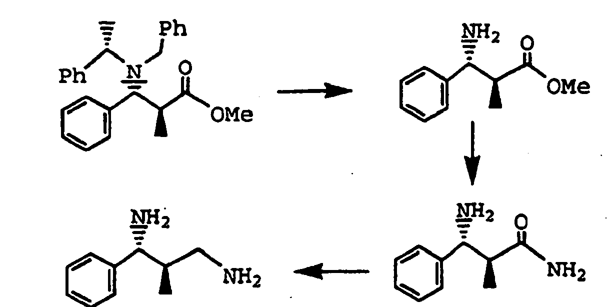

Step A: Methyl (2S,3R,αS)-3-(*N*-benzyl-*N*-α-methylbenzylamino)-2-methyl-3-phenylpropionate was prepared as reported for the 2R,3S,αR-enantiomer (S).G. Davies and I.A.S. Walters, J. Chem. Soc. Perkin Trans.I, 1129-1139 (1994).
Step B: Methyl (2S,3R)-3-amino-2-methyl-3-phenylpropionate: A mixture of methyl (2S,3R,αS)-3-(*N-*benzyl-*N*-α-methylbenzylamino)-2-methyl-3-phenylpropionate (13.0 g, 33.55 mmol) and 10% palladium-on-carbon (13.0 g) in glacial acetic acid (260 ml) was hydrogenated under a balloon of hydrogen for 24 h. The catalyst was removed by filtration followed by evaporation and co-distillation with toluene to provide the title compound as a white solid. MS (m/z): 194.2 (M+H)⁺; C₁₁H₁₅NO₂ requir. 193.3.
Step C: (2S,3R)-3-Amino-2-methyl-3-phenylpropionamide: A solution of methyl (2S,3R)-3-amino-2-methyl-3-phenylpropionate (6.3 g, 33 mmol) in 2N methanolic ammonia (20 ml) and ammonium hydroxide (28-30%, 40 ml) was stirred at room temperature. After 4d, concentration followed by chromatography on a short column of silica gel (dichloromethane - methanol - conc. ammonium hydroxide = 93 : 7 : 0.7; 90 : 10 : 0.8) provided the amide as a white solid. MS (m/z): 179.2 (M+H)⁺; C₁₀H₁₄N₂O requir. 178. 2.
Step D: (1R, 2R)-2-methyl-1-phenyl-1,3-propanediamine : Lithium aluminium hydride (2.3 g, 60.60 mmol) was added in portions to a stirring solution of (2S,3R)-3-amino-2-methyl-3-phenylpropionamide (2.6 g, 14.59 mmol) in tetrahydrofuran (54 ml) at ice-bath temperature. After 45 min, the mixture was heated at reflux for 16 h. With ice-bath cooling, the reaction was quenched by the portionwise addition of sodium sulfate decahydrate and some methanol until hydrogen evolution ceased. The solids were removed by filtration and washed with dichloromethane. The combined filtrates were evaporated to provide the title compound. MS (*m*/*z*): 165.2 (M+H)⁺; C₁₀H₁₆N₂ requir. 164.3.

### Example 1P: Procedure for the preparation of (1S,2S)-2-methyl-1-phenyl-1,3-propanediamine

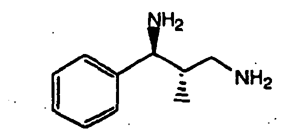

The title compound was prepared as described in the example for the synthesis of the enantiomer, (1R,2R)-2-methyl-1-phenyl-1,3-propanediamine, from methyl (2R,3S,αR)-3-(N-benzyl-*N*-α-methylbenzylamino)-2-methyl-3-phenylpropionate (Davies et al., J. Chem. Soc. Chem. Commun. 1153-1155, 1993). The title compound was obtained as a crystallizing oil, MS *(m*/*z):* 165.3 (M+H)⁺: C₁₀H₁₆N₂ requir. 164.3.

### Example 1Q: Procedure for the preparation of 3-phenyl-2,2-dimethyl-1,3-propanediamine

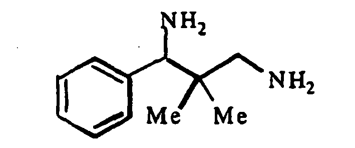

The title compound was prepared according to the procedure described in: W. Ten Hoeve and H. Wynberg, Synth. Commun. 24 (15), 2215-2221, 1994, MS (m/z): 179.1 (M+H)⁺; C₁₁H₁₈N₂ requir. 168.1

### Example 1R: Procedure for the preparation of 3-phenyl-2,2-dimethyl-1-aminopropane

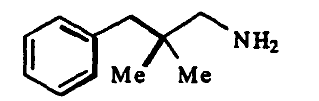

Step A: of 2,2-dimethyl-3-phenyl-1-azidopropane: Diisopropyl azodicarboxylate (19.7 mL, 100 mmol) was added dropwise to a stirred mixture of 2,2-dimethyl-3-phenyl-1-propanol (8.2 gm, 50 mmol), triphenylphosphine (26.2 gm, 100 mmol), and Zn(N₃)₂·2 pyridine (11.5 gm, 37.5 mmol) in toluene (250 mL). [reference: Synthesis, (1990) page 131] After 2.5h, celite (25 gm) was added, and the mixture was filtered and concentrated to an oil. Purification (SiO₂, 40% EtOAc/hexanes) gave the step A product as an oil.
Step B: of 2,2-dimethyl-3-phenyl-1-aminopropagne: A mixture of 2,2-dimethyl-3-phenyl-1-azidopropane (3 gm), 10% Pd-C, methanol (60 mL) and tetrahydrofuran (15 mL) was stirred under 1 atmosphere of hydrogen at rt for 18h. The mixture was filtered and concentrated to give the title compound as an oil, MS (m/z): 164.1 (M+H)⁺; C₁₁H₁₇N requir. 163 .1.

### Example 1S: Procedure for the preparation of 1-(aminomethyl)-2-benzylcyclopentane

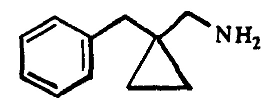

Step A: 1-benzyl-1-cyclopropanecarbonitrile: A solution of cyclopropyl cyanide (3.0 mL, 40 mmol) in 20 mL THF was dropwise added to a stirred, freshly prepared, mixture of lithium diisopropylamide (40 mmol) in THF (100 mL) at -78 °C. After 30 min, a solution of benzyl bromide 7.8 mL, 60 mmol) in THF (20 mL) was dropwise added. The resulting mixture was warmed slowly over several hrs and stirred at rt 48 hr. The reaction was quenched (250 mL saturated NH₄Cl), extracted with ether (3 X 100 mL) and the combined organic extracts were dried (MgSO₄), filtered and concentrated to afford a yellow oil.
Step **B** ; 1-(aminomethyl)-2-banzylcyclopentane: A solution of 1-benzyl-1-cyclopropanecarbonitrile (9.16 gm, 58 mmol), 10% Pd-C (1.5 gm), in MeOH (200 mL), THF (50 mL), and conc. HCl (6 mL) was shaken under a hydrogen atmosphere (50 psi) for 15 hr. The mixture was concentrated, water (300 mL0 was added and made basic (pH 10 -11) with 2N NaOH. The mixture was extracted with EtOAc (2 x 100 mL), the combined organic layers were dried (MgSO₄), filtered and concentrated to provide the title compound.

### Example 2;

### Procedure for the preparation of 6-bromo-[2,4']bipyridine

Step A: pyridine-4-boronic acid: 4-bromopyridine hydrochloride (19.46 gm, 0.1 mole) was neutralized with 60 mL of 2 M aqueous Na₂CO₃ and extracted with ether (200 mL). The dried (MgSO₄) organic layer was concentrated to obtain 4-bromopyridine which was dropwise added to a cooled (-78 °C) stirred solution of *t*-butyllithium (88 mL, 1.7 M in hexanes) in ether (150 mL). 30 min after complete addition, triisopropyl borate (22 mL, 0.2 mole) was dropwise added. The reaction mixture was warmed to rt and quenched with 50% aqueous methanol (40 mL), followed by water (100 mL). Acidification of the mixture with conc HCl (to pH 5.5 - 6.0) provided a white precipitate which was collected by filtration and rinsed (H₂O) and dried to give pyridine-4-boronic acid.
Step B: 6-bromo-[2,4']bipyridine: Dry N₂ was bubbled through a stirred solution of 2,6-dibromopyridine (1.6 gm, 6.7 mmole), pyridine-4-boronic acid (317 mg, 2.6 mmol), and Pd(PPh₃)₄ (160 mg) in aqueous 2M Na₂CO₃ (8 mL) and toluene (8 mL) at rt for 20 min. The reaction mixture was then heated to reflux for 10 hr. After cooling to rt CH₂Cl₂ (100 mL) was added and the mixture was washed with brine and dried (Na₂SO₄). Purification (SiO₂, CH₂Cl₃/MeOH/NH₄OH: 100/11/8) gave 6-bromo-[2,4']bipyridine. MS (m/z): Calcd. C₁₀H₇N₂Br (M⁺): 235, found: 234.9.

### Example 3

### General procedure for the preparation of 6-alkylamino-3-bromo-2-(4-pyridyl)pyridines

### Example 3A: Preparation of 6-((S)-2-Amino-3-phenylpropylamino)-3-bromo-2-(4-pyridyl)pyridine

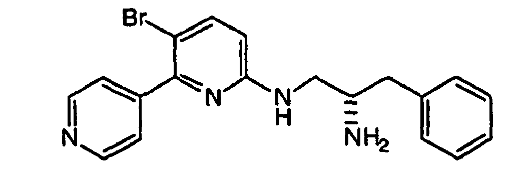

Sten A: Preparation of 6-((S)-2-Amino-3-phenylpropylamino)-2-(4(pyridyl) pyridine : A neat mixture of 6-bromo-[2,4']bipyridine (2.35 gm, 10 mmole) and (S)-2-amino-3-phenylpropylamine (3 gm, 20 mmole) was heated to 190 °C for 4 hr. The reaction was cooled to rt and purified (SiO₂, CH₂Cl₂/MeOH/NH₄OH: 100/11/8) to give the step 1 compound. (This reaction provides major products wherein the less hindered amine functionality displaces the bromide,when the nucleophile is an alkyldiamine) MS (*m*/*z*): Calcd. C₁₉H₂₀N₄ (M⁺) : 304, found (M+H)⁺: 305.2.
Step B: Preparation of 6-((S)-2-Amino-3-phenylpropylamino)-3-bromo-2-(4-pyridyl)pyridine : A mixture of bromine (1.6 gm, 10 mmole) and HOAc (10 mL)was added in three portions to a stirred solution of 6-((S)-2-Amino-3-phenylpropylamino)-2-(4-pyridyl)pyridine (3.04 gm, 10 mmole) in HOAc (20 mL) at rt. After 1 hr, the mixture was concentrated and purified (SiO₂, CH₂Cl₂/MeOH/NH₄OH: 100/11/8) to give 6-((S)-2-Amino-3-phenylpropylamino)-3-bromo-2-(4-pyridyl)pyridine. MS (*m*/*z*): Calcd. C₁₉H₁₉N₄Br (M⁺): 383, found : 383.1 and 385.1.
The following compounds (derivatives of 3-bromopyridine) may be prepared according to the procedure set forth in Example 3A, using the appropriate amine in Step A, followed by bromination as in Step B.

### Example 3B: 6-(3-phenylpropylamino)-3-bromo-2-(4-pyridyl)pyridine

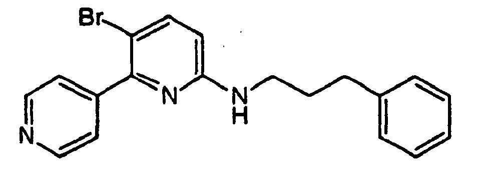

### Example 3C: 6-((R,S)-2-methyl-3-phenylpropylamino)-3-bromo-2-(4-pyridyl)pyridine

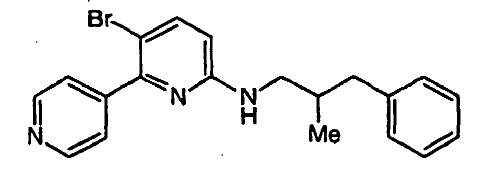

### Example 3D: 6-(2,2-dimethyl-3-phenylpropylamino)-3-bromo-2-(4-pyridyl)pyridine

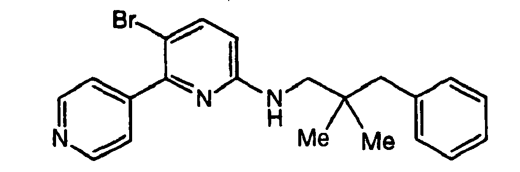

### Example 3E: 6-((R,S)-3-amino-2,2-dimethyl-3-phenylpropylamino)-3-bromo-2-(4-pyridyl)pyridine

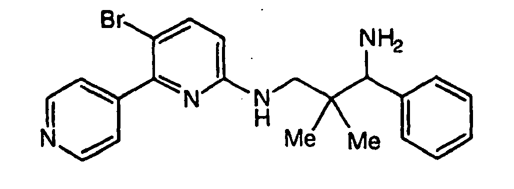

### Example 3F: 6-((R, S)-3-amino-3-phenylpropylamino)-3-bromo-2-(4-pyridyl)pyridine

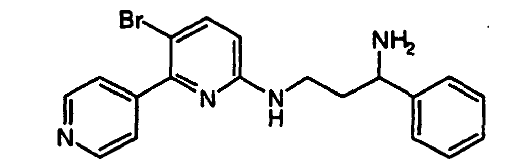

### Example 3G: 6-(R,S)-amino-3-(2-chlorophenyl)propylamino)-3-bromo-2-(4-pyridyl)pyridine

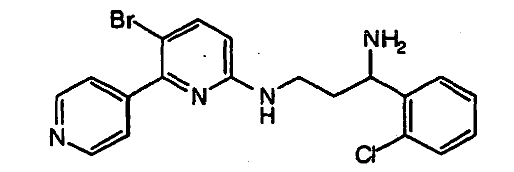

### Example 3H: 6-((R,S)-3-amino-3-(2-fluorophenyl)propylamino)-3-bromo-2-(4-pyridyl)pyridine

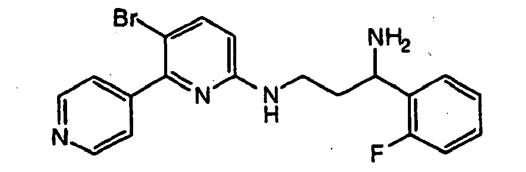

### Example 3I : 6-((R,S)-3-amino-3-(2-methylphenyl)propylamino)-3-bromo-2-(4-pyridyl)pyridine

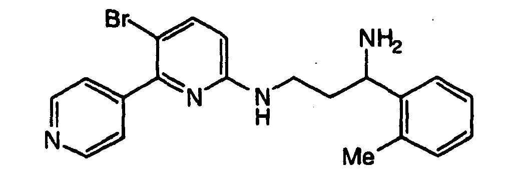

### Example 3J: 6-((S)-2-methyl-(R)-3-amino-3-phenylpropylamino)-3-bromo-2-(4-pyridyl)pyridine

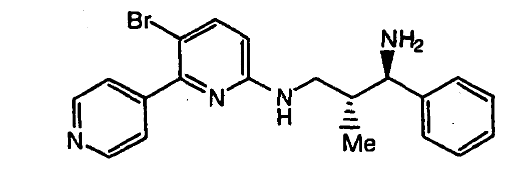

### Example 3K: 6-(1,2,3,4-tetrahydroisoguinolinyl-3-methylamino)-3-bromo-2-(4-pyridyl)pyridine

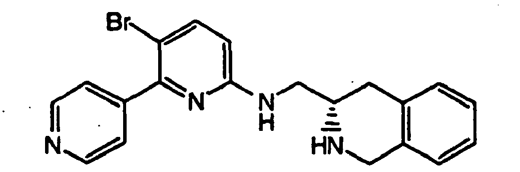

### Example 3L: 6-(N-(3-benzylpiperazin-1-yl)-3-bromo-2-(4-pyridyl)pyridine

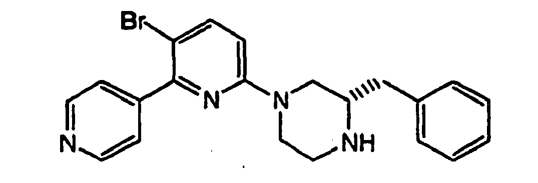

### Example 4

### General procedure for the preparation of 6-alkylamino-3-aryl-2-(4-pyridyl)pyridines

### Example 4A: Preparation 6-((S)-2-Amino-3-phenylpropylamino) -3-(3-methylphenyl)-2- (4-pyridyl)pyridine

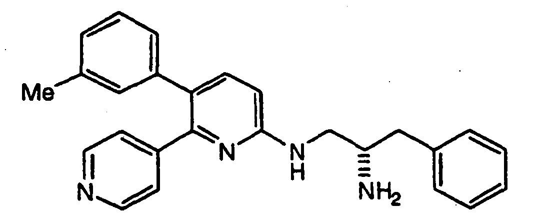

To a stirred, degassed mixture of 6-((S)-2-Amino-3-phenylpropylamino)-3-bromo-2-(4-pyridyl)pyridine (4.2 gm, 10.9 mmole), 3-methylbenzene boronic acid (1.8 gm, 13 mmole), in aqueous 2 M Na₂CO₃ (50 mL) and toluene (50 mL) at rt was added Pd(PPh₃)₄ (400 mg, 0.35 mmole). The mixture was heated to reflux for 12 hrs, cooled to rt, and extracted with toluene. The combined organic layers were washed with brine, concentrated and purified (SiO₂, CH₂Cl₂/MeOH/NH₄OH: 100/11/8) to give the title compound. MS (*m*/*z*): Calcd. C₂₆H₂₆N₄ (M⁺) : 394, found (M+H)⁺: 395.1. The following compounds were prepared according to the procedure set forth in Example 4A, using the appropriate boronic acid and using the 3-bromopyridine derivative (whose preparation is described in Example 3).

### Example 4B: 6-((S)-2-Amino-3-phenylpropylamino)-3-(3-trifluoromethylphenyl)-2-(4-pyridyl)pyridine

MS (m/z): Calcd. C₂₆H₂₃N₄F₃ (M⁺) : 448, found (M+H) 449.3.

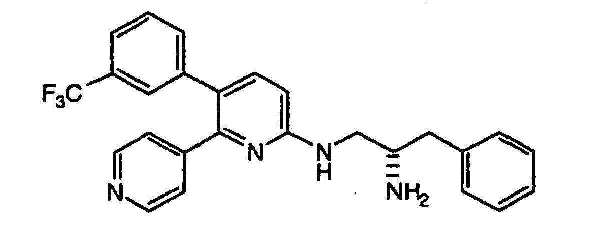

### Example 4C: 6-((S)-2-Amino-3-phenylpropylamino)-3-(2-napthyl)-2-(4-pyridyl)pyridine

MS (*m*/*z*): Calcd. C₂₉H₂₆N₄ (M'): 431, found (M+H) ⁺: 431.5.

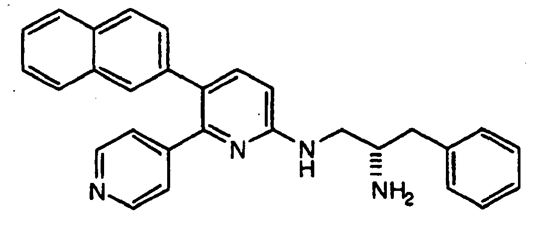

### Example 4D: 6-((S)-2-Amino-3-phenylpropylamino)-3-(4-chlorophenyl)-2-(4-pyridyl)pyridine

MS (m/z): Calcd. C₂₅H₂₃N₄Cl (M⁺) : 414, found (M+H) : 415.4.

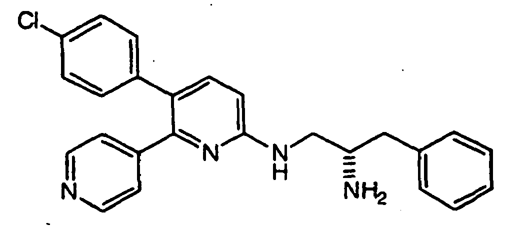

### Example 4E: 6-((S)-2-Amino-3-phenylpropylamino)-3-(3-isopropylphenyl)-2-(4-pyridyl)pyridine

MS *(m*/*z) :* Calcd. C₂₈H₃₀N₄ (M⁺): 422, found (M+H)⁺: 423.2.

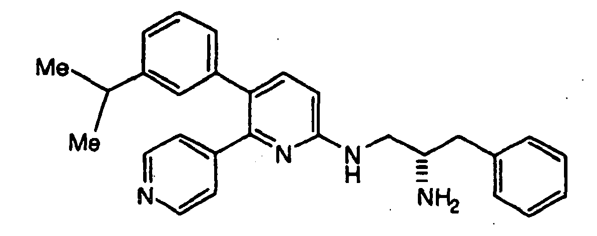

### Example 4F: 6-((S)-2-Amino-3-phenylpropylamino)-3-(4-methoxyphenyl) -2 - (4-pyridyl)pyridine

MS (m/z): Calcd. C₂₆H₂₆ON₄ (M⁺): 410, found (M+H) ⁺: 411.3

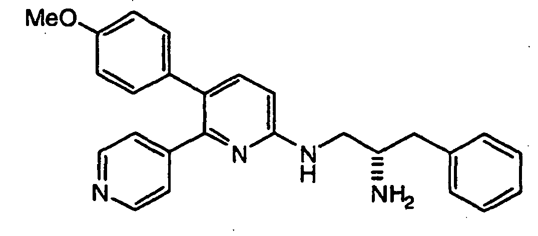

### Example 4G: 6-((S)-2-Amino-3-phenylpropylamino)-3-(4-chloro-3-fluorophenyl)-2-(4-pyridyl)pyridine

MS (m/z): Calcd. C₂₅H₂₂N₄FCl (M'): 432, found (M+H)⁺ : 433.3

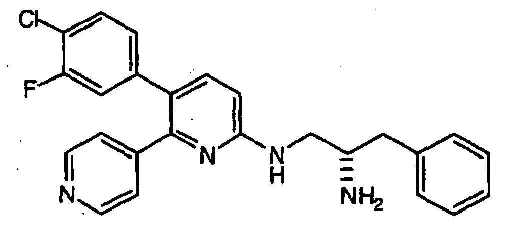

### Example 4H: 6-((S)-2-Amino-3-phenylpropylamino)-3-(2-benzothiophenyl)-2-(4-pyridyl)pyridine

MS (*m*/*z*): Calcd. C₁₇H₂₄N₄S (M⁻): 436, found (M+H) : 437.5 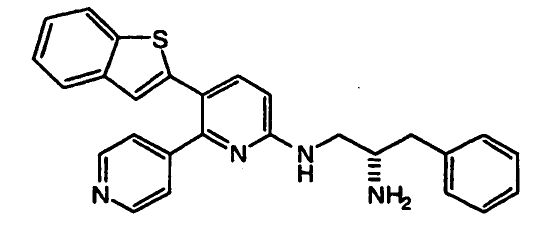 The following compounds can be prepared according to the procedure set forth in Example 4A, using the appropriate boronic acid and using the 3-bromopyridine derivative (whose preparation is described in Example 3).

### Example 4I: 6-((S)-2-Amino-3-phenylpropylamino)-3-(3-fluorophenyl)-2-(4-pyridyl)pyridine

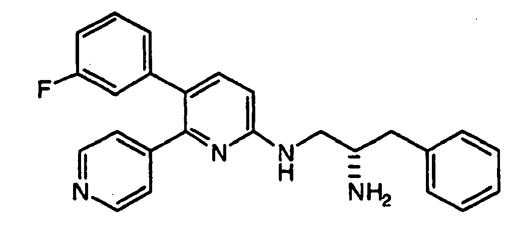

### Example 4j: 6-((S)-2-Amino-3-phenylpropylamino)-3-(4-fluorophenyl)-2-(4-pyridyl)pyridine

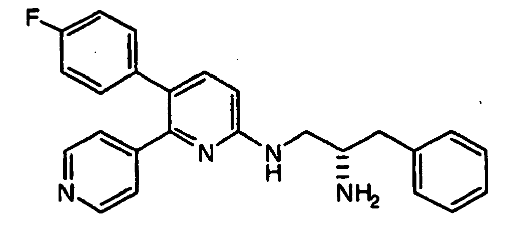

### Example 4k: 6-(3-Amino-3-phenylpropylamino)-3-(3-methylphenyl)-2-(4-pyridyl)pyridine

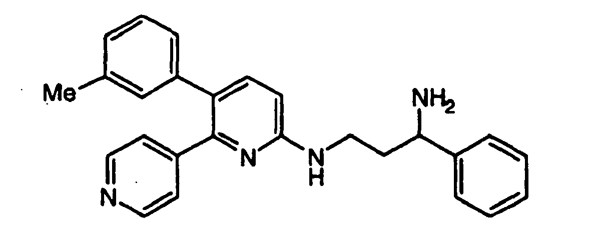

### Example 4l: 6-(3-Amino-3-phenylpropylamino)-3-(4-fluorophenyl)-2-(4-pyridyl)pyridine

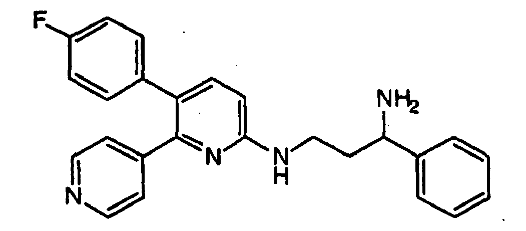

### Example 4m: 6-(3-Amino-3-phenylpropylamino)-3-(3-trifluoromethylphenyl)-2-(4-pyridyl)pyridine

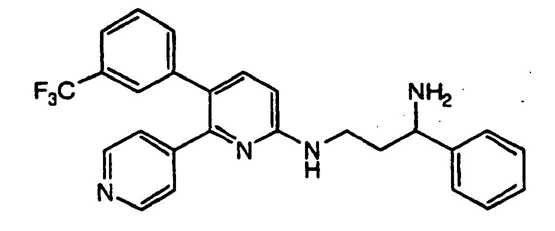

### Example 4n: 6-(3-Amino-3-phenylpropylamino)-3-(2-benzothiophenyl)-2-(4 pyridyl)pyridine

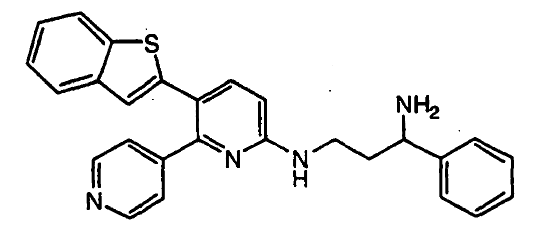

### Example 4o: 6-(3-amino-2,2-dimethyl-3-phenylpropylamino)-(3-methylphenyl)-2-(4-pyridyl)pyridine

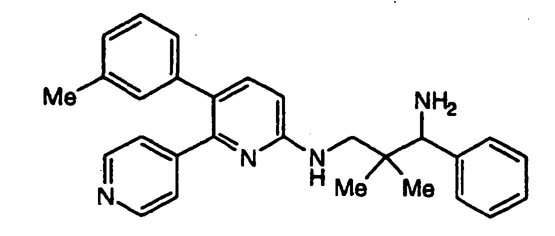

### Example 4p: 6-(3-amino-2,2-dimethyl-3-phenylpropylamino)- (4-fluorophenyl) -2-(4-pyridyl)pyridine

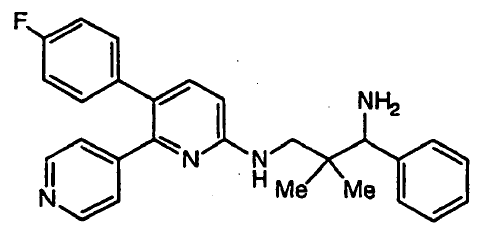

### Example 4q: 6-(3-amino-2,2-dimethyl-3-Phenylpropylamino)-(4-chloro-3-fluorophenyl)-2-(4-pyridyl)pyridine

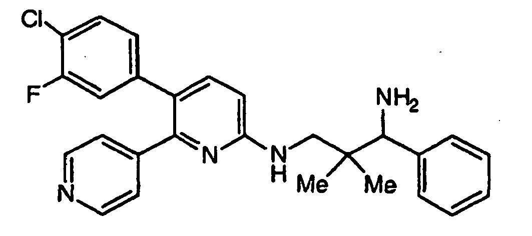

### Example 4r: 6-(3-amino-2,2-dimethyl-3-phenylpropylamino)-(2-benzothiophenyl)-2-(4-pyridyl)pyridine

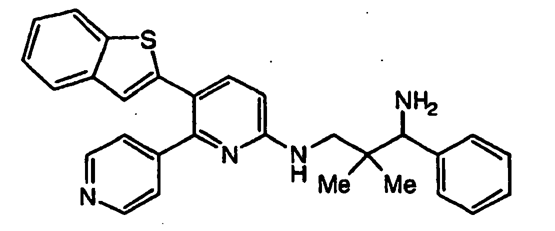

### Example 4s: 6-(3-amino-3-(2-chlorophenyl)propylamino)-(3-methylphenyl)-2-(4-pyridyl)pyridine

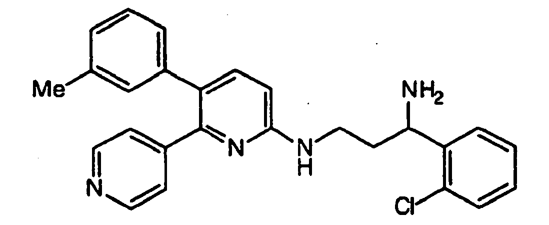

### Example 4t: 6-(3-amino-3-(2-chlorophenyl)propylamino)-(4-fluorophenyl)-2-(4-pyridyl)pyridine

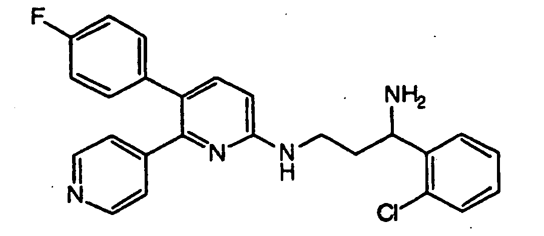

### Example 4u: 6-(3-amino-3-(2-fluorophenyl)propylamino)-3-(3-methylphenyl) -2- (4-pyridyl)pyridine

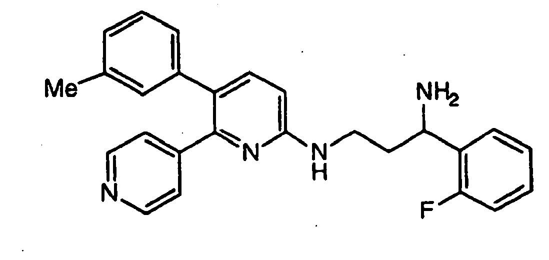

### Example 4v: 6-(3-amino-3-(2-methylphenyl)propylamino)-3-(3-trifluoromethylphenyl)-2-(4-pyridyl)pyridine

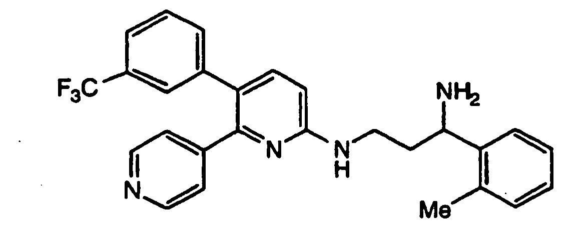

### Example 4w: 6-((S)-2-methyl-(R)-3-amino-3-phenylpropylamino) -3- (4-fluorophenyl) -2- (4-pyridyl) pyridine

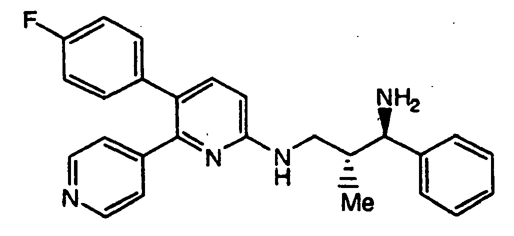

### Example 4x: 6-((S)-2-methyl-(R)-3-amino-3-phenylpropylamino)-3-(3-methylphenyl)-2-(4-pyridyl)pyridine

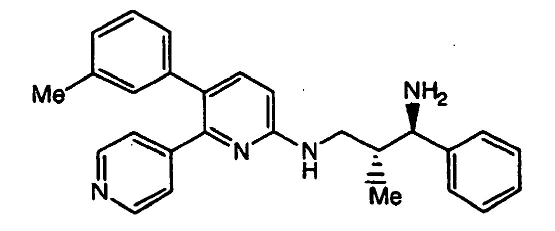

### Example 4y: 6-(1,2,3,4-tetrahydroisoquinolinyl-3-methylamino)-3-(3-chloro-4-fluorophenyl)-2-(4-pyridyl)pyridine

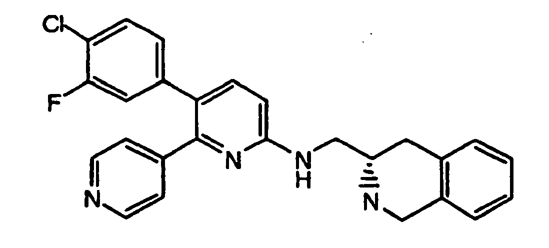

### Example 4z: 6-(N-(3-benzylpiperazin-1-yl)-3-(4-fluorophenyl)-2-(4-pyridyl)pyridine

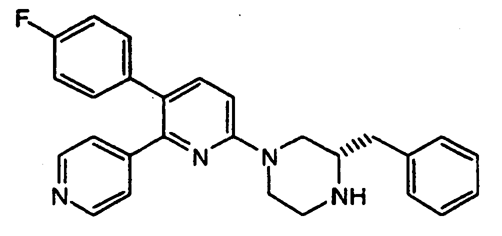

### Example 5

### Procedure for the preparation of 6-(4-Fluorophenyl)-5-(4-pyridyl)-2H-pyridazin-3-one

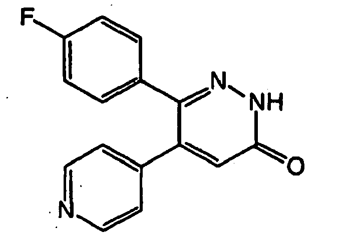

Step A: Ethyl 3-(4-fluorobenzoyl)-3-(4-pyridyl)-propionate: Sodium (400 mg, 17.40 mmol) was added to a stirred solution of 1-(4-fluorophenyl)-2-(4-pyridyl)ethanone (3.35 g, 15.58 mmol) (P. J. Gilligan et al., J. Med. Chem. 35, 4344, 1992) in ethanol (50 ml) at room temperature. After dissolution of the sodium, ethyl bromoacetate (1.93 ml, 17.40 mmol) was added dropwise at ice-bath temperature. After stirring for 4 h at room temperature, the reaction mixture was concentrated by evaporation. It was diluted with dichloromethane and made neutral by washing with diluted acetic acid followed by drying of the organic solution and evaporation. Flash chromatography (hexane - acetone = 3 : 1, 2 : 1) provided the title compound as a syrup. MS (m/z) : 302.2 (M+H)⁺; C₁₇H₁₆FNO, requir. 301.3.
Step B: 6-(4-Fluorophenyl)-4,5-dihydro-5-(4-pyridyl)-2H-pyridazin-3-one: A solution of ethyl 3-(4-fluorobenzoyl)-3-(4-pyridyl)- propionate (1.0 g, 3.32 mmol) and hydrazine monohydrate (1 ml, 20.6 mmol) in ethanol (1 ml) was refluxed for 2.5 h. The solvent and hydrazine monohydrate were evaporated. The remainder was taken up in n-butanol and the mixture was heated at reflux for 45 min. Evaporation was followed by column chromatography on silica gel (3-7.5% methanol/dichloromethane) to provide the title compound. MS (m/z): 270.2 (M+H)⁺; C₁₅H₁₂FN₃O requir. 269.3.
Step C: 6-(4-Fluorophenyl)-5-(4-pyridyl)-2H-pyridazin-3-one: A solution of bromine (78.3 µl, 1.48 mmol) in acetic acid (6 ml) was added dropwise to a stirred solution of 6-(4-fluorophenyl)-4,5-dihydro-5-(4-pyridyl)-2H-pyridazin-3-one (314 mg, 1.17 mmol) in acetic acid (4.6 ml) at room temperature. After 2 h at room temperature, more bromine (41.7 µl, 0.78 mmol) in acetic acid (3.2 ml) was added to the turbid mixture. A gum precipitated. After 30 minutes, it was evaporated and co-evaporated with toluene. Residual acid was neutralized with methanolic 2N ammonia followed by evaporation. The resulting product was purified on a column of silica gel (3-5% methanol/dichloromethane) to provide the title compound as a solid. MS (m/z): 268.1 (M+H)⁺; C₁₅H₁₀FN₃O requir. 267.3.

### Example 6

### Procedure for the preparation of 6-[((S)-2-amino-3-phenylpropyl)-amino]-3-(4-fluorophenyl)-4-(4-pyridyl)-pyridazine

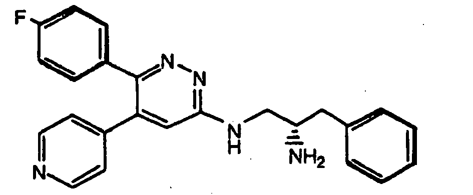

Step A: 6-Chloro-3-(4-fluorophenyl)-4-(4-pyridyl)-pyridazine: A stirred mixture of 6-(4-fluorophenyl)-5-(4-pyridyl)-2H-pyridazin-3-one (105 mg, 0.40 mmol) and phosphorus oxychloride (2 ml) was heated at reflux for 2 h. It was evaporated, followed by co-evaporation with toluene and drying of the resultant product in an oil pump vacuum for several hours. Then dichloromethane was added and dil. ammonium hydroxide to neutrality with cooling. The organic solution was washed with water, dried and evaporated to leave the title compound. MS (m/z): 286.0 (M)⁺; C₁₅H₉ClFN₃ requir. 285.7.
Step B: 6-[((S)-2-amino-3-phenylpropyl)-amino]-3-(4-fluorophenyl)-4-(4-pyridyl)-opyridazine: A stirred mixture of 6-chloro-3-(4-fluorophenyl)-4-(4-pyridyl)-pyridazine (102 mg, 0.36 mmol) and (S)-1,2-benzylethylendiamine (200 µl, -1.3 mmol) was heated at 160° C for 2 h. The resultant product was applied to a column of silica gel (dichloromethane - methanol = 93:7; dichloromethane - methanol - conc. ammonium hydroxide = 93:7:0.7) to provide the title compound. MS (m/z): 400.1 (M+H)⁺; C₂₄H₂₂FN₅ requir. 399.5.

### Example 7

### Procedure for preparation of 2-((S)-2-amino-3-phenylpropylamino)-5-(3-methylphenyl)-4-(4-pyridyl)pyridine

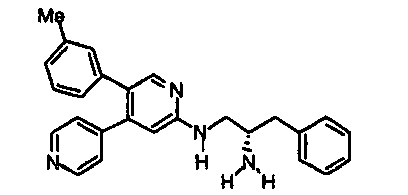

Step A: Preparation of 4-(*tert*-butyl-dimethyl-silanyloxymethyl)-pyridine: To a stirred solution of 4-pyridylcarbinol ( 21.8 g, 0.20 mole) in DMF (200 mL) at 25 °C was added imidazole (15.64 g, 0.23 mole) and t-butyldimethylsilyl chloride (31.65 g, 0.21 mole). The reaction mixture was allowed to stirred at that temperature for 3 hr. Standard aqueous work up (ethyl acetate extraction, washed with water and brine, dried with MgSO₄, evaporation), followed by chromatographic purification (silica gel, hexane/ethyl acetate) gave the title compound. ¹H-NMR (CDCl₃, 400 MHz) δ: 8.50 (d, 2H), 7.25(d, 2H), 4.86(s, 2H), 0.90(s, 9H), 0.05(s, 6H).
Step B: Preparation of 2-(tert-butyl-dimethyl-silanyloxy)-2-pyridine-4-yl-1-*m*-tolyl-ethanole: To a solution of 4-(*tert*-butyl-dimethyl-silanyloxymethyl)-pyridine (5 g, 22 mmole) in THF (100 mL) at -20 °C was added LDA (2M, 13.2 mL, 26.4 mmole) dropwise. The mixture was strired at that temperature for 1 hr before addition of 3-methylbenalhehyde (2.9 g, 24 mmole) in THF (20 mL). The reaction was then warmed up to r. t. for additional 1 hr. The mixture was diluted with EtOAc, washed with NH₄Cl and brine, dried with MgSO₄, evaporarted and, finally, purified on column (silica gel, hexane/ethyl acetate) to give the title compound.
Step C: Preparation of 1-pyridine-4-yl-2-*m*-tolyl-ethane-1,2-diol : To a solution of 2-(tert-butyl-dimethyl-silanyloxy)-2-pyridine-4-yl-1-*m*-tolyl-ethanol (5 g, 14.5 mmole) in THF (50 mL) was added t-butylamonium fluoride (1M, 16 mL, 16 mmole) at 25 °C. The solution was stirred at that temperature for 1 hr before evaporation of solvent and purification (silica gel, hexane/ethyl acetate) to give the title compound. MS (m/z): Calcd. C₁₄H₁₅NO₂(M⁺): 229, found (M+H)⁺ : 230.1, (M-H)⁻ : 228.1
Step D: Preparation of 1-pyridine-4 -yl-2-m-tolyl-ethane-1.2-dione: Dimethylsufoxide (2.85 mL, 40 mmole) was dropwise added into a solution of trifluoroacetic anhydride (4.24 mL, 30 mmole) in methylene chloride (100 mL) at 78 °C. The reaction mixture was stirred at that temperature for 10 min before the addition of 1-pyridine-4-yl-2-*m*-tolyl-ethane-1,2-diol (2.29 g, 10 mmole) in methylene chloride (50 mL). The mixture was stirred additional 1 hr at that temperature. Finally, the mixture was quenched with triethylamine (8.5 mL, 60 mmole) and the resulting mixture was allowed to warmed to r.t.. The reaction was diluted with methylene chloride, washed with NH₄Cl and brine, dried with MgSO₄, evaporated, and finally, purified through a silica column (ethyl acetate/hexane) to give the title compound. MS *(m*/*z):* Calcd. C₁₄H₁₁NO₂(M⁺) : 225, found (M+H) ⁺ : 226.1.
Step E: Preparation of 4-hydroxy-3-pyridine-4-yl-4-*m-*tolyl-cyclopent-2-en-1-one: To a solution of 1-pyridine-4-yl-2-*m*-tolyl-ethane-1,2-dione (1.8 g, 8.0 mmole) in acetone (20 mL) was added crushed KOH (448 mg, 8.0 mmole) in one portion at r.t. The reaction mixture was stirred at that temperature for 1 hr before quenching the reaaction with aqueous NH₄Cl. Standard aqueous work up, followed by chromatographic purificaion (silica gel, hexane/ethyl acetate) gave the a mixture of the title compound and the regiosiomer, 4-hydroxy-4-pyridine-4-yl-3-*m*-tolyl-cyclopent-2-en-1-one. MS (*m*/*z*): Calcd. C₁₇H₁₅NO₂(M⁺) : 265, found (M+H)⁺ : 265.9.
Step F: Preparation of 4-acetoxy-3-pyridine-4-yl-4-*m-*tolyl-cyclopent-2-en-1-one: To a solution of 4-hydroxy-3-pyridine-4-yl-4-*m*-tolyl-cyclopent-2-en-1-one and it's regioisomer (265 mg, 1.0 mmole) in methylene chloride (5 mL) was added dimethylamino pyridine (183 mg, 1.5 mmole) and acetic anhydride (0.12 mL, 1.2 mmole) at r.t.. The reaction mixture was stirred at that temperature for 1 hr before quenching the reaction with 1 mL of methanol. Concentration and purification (silica, hexane/ethyl acetate) gave the title compound as the faster eluting isomer. MS (m/z): Calcd. C₁₉H₁₇NO₃(M⁺): 307, found (M+H)⁺ : 308.1.
Step G: Preparation of 1-acetoxy-4-hydroxyimino-2-pyridine-4-yl-1-*m*-tolyl-cyclopent-2-ene: To a solution 4-acetoxy-3-pyridine-4-yl-4-*m*-tolyl-cyclopent-2-en-1-one (307 mg, 1.0 mmole) in etanol (10 mL) was added hydroxylamine hydrochloride (105 mg, 1.5 mmole) and pyridine (5 drops) at r. t. The reaction mixture was heated to 70 °C for 3 hr before cooling down to r.t.. Concentration and purification (silica gel, hexane/ethyl acetate) gave the title compound. MS (m/z): Calcd. C₁₉H₁₈N₂O) (M⁺): 322, found (M+H)⁺ : 323.2.
Step H: Preparation of 5-acetoxy-5-m-tolyl-5,6-dihydro-1H-[4,4']bipyridinyl-2-one: To a solution of 1-acetoxy-4-hydroxyimino-2-pyridine-4-yl-1-*m*- tolyl-cyclopent-2-ene (322 mg, 1.0 mmole) in methylene chloride (10 mL) at r.t. was added PCl₅ (417 mg, 2.0 mmole) in one portion. The reaction mixture was stirred at that temperature for 1 hour before quenching the reaction with sodium bicarbonate solution. Stanard basic work up, followed by purification gave the title compound. MS (m/z): Calcd. C₁₉H₁₈N₂O₃(M⁺) : 322, found (M+H)⁺: 322.9.
Step I: Preparation of 5-hydroxy-5-*m*-tolyl-5, 6-dihydro-1H-[4,4']bipyridinyl-2-one: To a solution of 5-acetoxy-5-*m*-tolyl-5,6-dihydro-1H-[4,4']bipyridinyl-2-one (322 mg, 1.0 mmole) in THF (5 mL) and water (5 mL) at r.t. was added LiOH (126 mg, 3.0 mmole) in one portion. The reaction mixture was stirred at that temperature for 1 hr before quenching the mixture with aqueous NH₄Cl. Standard work up (extraction of compound with methylene chloride), followed by purification (methano/methylene chloride) gave the title compound. MS (*m*/*z*) : calcd. C₁₇H₁₆N₂O₂(M⁺) : 280, found (M+H)⁺ : 281.0.
Step J: Preparation of 5-*m*-tolyl-1H-[4,4']bipyridinyl-2-one: To a solution of 5-hydroxy-5-*m*-tolyl-5,6-dihydro-1H-[4,4']bipyridinyl-2-one (280 mg, 1.0 mmole) in CHCl₃ (5 mL) at r.t.was added 1 ml of conc. H₂SO₄. The resulting mixture was heated to 55 °C for 1 hr. The mixture was cooled down to r.t. and was carefully quenched with aqueous sodium carbonate. Standard work up (extraction of compound with methylene chloride), followed by purification (silica gel, methanol /methylene chloride) gave the title compound. MS (m/z): Calcd. C₁₇H₁₄N₂O (M⁺) : 262, found (M+H)⁺: 263.3.
Step K: Preparation of 2-chloro-5-(3-methylphenyl)-4-(4-pyridyl) pyridine; 5-*m*-tolyl-1H-[4,4']bipyridinyl-2-one (262 mg, 1.0 mmole) in POCl, (5 mL) was heated to 105 °C for 12 hr. POCl, was removed under reduced pressure. The residue was diluted with methylene chloride and was carefully quenched with aqueous sodium carbonate. Standard work up, followed by purification (silica gel, hexane/ethyl acetate) gave the title compound. MS (m/z): Calcd. C₁₇H₁₃N₂Cl(M⁺): 280.5, found (M+H)⁺: 281.0 and 283.1.
Step L: Preparation of 2-((S)-2-amino-3-phenylpropylamino)-5- (3-methylphenyl)-4- (4-pyridyl)pyridine: A mixture of 2-chloro-5-(3-methylphenyl)-4-(4-pyridyl)pyridine (281 mg, 1.0 mmole) and (S)-1,2-benzylethylenediamine (375 mg, 2.5 mmole) was heated to 160 °C for 5 hr. The mixture was cooled down and was added 2 mL of methylene chloride. The resulting mixture was subjected to chromatographic purification (silica gel, methanol/methylene chloride) to give the title compound. MS (m/z): Calcd. C₂₆H₂₆N₄ (M⁺): 394, found (M+H)⁺: 395.1

### Example 8

### An alternative procedure for the preparation 2-((S)-2-amino-3-phenylpropylamino)-5-(3-methylphenyl)-4-(4-pyridyl)pyridine

Step A Preparation of 2-((S)-2-amino-3-phenylpropylamino)-4-(4-pyridyl)pyridine: A mixture of 2-chloro-[4,4']-bipyridine (Moran, D.B. et al, J. Heterocyclic Chem. 1986, 23, 1071) (1 g, 5.26 mmole) and (S)-1,2-benzylethylenediamine (1.8 g, 12 mmole) was heated at 190 °C for 3 hr. The mixture was cooled down to room temperature and was subjected to chromatographic purification (20% MeOH in CH₂Cl₂) to give the title compound. MS *(m*/*z)*: Calcd. C₁₉H₂₀N₄ (M⁺): 304, found (M+H)⁺ : 305.4. ¹H-NMR (CDCl₃, 400 MHz) δ: 8.60(d, 2H), 8.0(d, 1H), 7.38-7.10(m, 5H), 7.26(d, 2H), 6.62(d, 1H), 6.45(s, 1H), 5.82(bs, 1H), 3.70-3.40(m, 3H), 2.95(m, 2H).
Step B: Preparation of 2-((S)-2-amino-3-phenylpropylamino)-5-bromo-4-(4-pyridyl) pyridine: Bromine (757 mg, 4.7 mmole) in CHCl₃ (10 mL) was added in one portion to a stirring solution of 2-((S)-2-amino-3-phenylpropylamino)-4-(4-pyridyl)pyridine (1.44 g, 4.7 mmole) in CHCl₃ (30 mL) at room temperature. After 1 hr, the mixture was partitioned between dichloromethane and aqueous sodium bicarbonate. The organic solvent was washed with brine, dried and evaporated. The residue was purified on a column of silica gel (CH₂Cl₂-MeOH-Conc. NH₄OH = 1000 : 110 : 8). MS (m/z): Calcd. C₁₉H₁₉N₄Br (M'): 383, found : 383, 385.1. ¹H-NMR (CDCl₃, 400 MHz) δ: 8.62(d, 2H), 8.20(s, 1H), 7.30-7.10(m, 7H), 6.32(s, 1H), 5.78(bs, 1H), 3.70-3.30(m, 3H), 2.97(dd, 1H), 2.92(dd, 1H).
Step C: Preparation of 2-((S)-2-amino-3-phenylpropylamino)-5-(3-methylphenyl)-4-(4-pyridyl)pyridine: A mixture of 2-((S)-2-amino-3-phenylpropylamino)-5-bromo-4-(4-pyridyl)pyridine (4.2 g, 10.9 mmole), aqueous N₂CO₃, (2M, 50 mL) and 3-methylbenzene boronic acid (1.8 g, 13 mmole) in toluene (50 mL) was stirred for 10 min. The mixture was thoroughly degassed (10 min) with nitrogen, before the addition of tetrakis(triphenyl phosphine)palladium (400 mg, 0.35 mmole). After heating at reflux for 12 hr, the reaction mixture was diluted with toluene and washed with brine. The organic solvent was dried and evaporated and the residue wasubjected to chrmatographic purification (CH₃Cl₂-MeOH-Conc. NH₄OH = 1000 : 110 : 8). MS (m/z): Calcd. C₂₆H₂₆N₄ (M') : 394, found (M+H)⁺: 395.1. ¹H-NMR (CDCl₃, 400 MHz) δ: 8.50(d, 2H), 8.15(s, 1H), 7.38-7.00(m, 9H), 6.90(, 1H), 6.80(d, 1H), 6.40(s, 1H), 5.38(bs, 1H), 3.62-3.20(m, 3H), 2.92(dd, 1H), 2.62(dd, 1H).

### Example 9

The following compounds were prepared according to the procedure outlined in Example 8, step C, using 2-((S)-2-amino-3-phenylpropylamino)-5-bromo-4-(4-pyridyl)pyridine and the appropriate boronic acid.

### Example 9a: 2-((S)-2-amino-3-phenylpropylamino)-5-(3-isopropylphenyl) -4-(4-pyridinyl)pyridine

MS *(m*/*z) :* Calcd. C₂₈H₃₀N₄ (M⁺) : 422, found (M+H)⁺ : 423.2

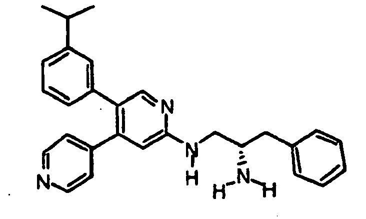

### Example 9b: 2-((S)-2-amino-3-phenylpropylamino)-5-(3-trifluoromethylphenyl-4-(4-pyridinyl)pyridine

MS *(m*/*z):* Calcd. C₂₆H₂₃N₄F₃ (M⁺): 448, found (M+H)⁺ : 449.2

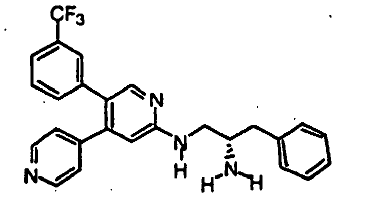

### Example 9c: 2-((S)-2-amino-3-phenylpropylamino)-5-(3-fluorophenyl-4-(4-pyridinyl)pyridine

MS (m/z): Calcd. C₂₅H₂₃N₄F(M⁺) : 398, found (M+H)⁺ : 399.1

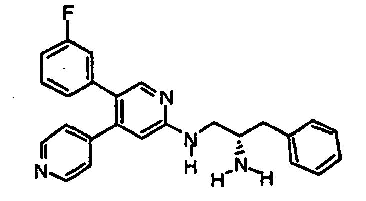

### Example 9d: 2-((S)-2-amino-3-phenylpropylamino)-5-(4-chlorophenyl)-4-(4-pyridinyl)pyridine

MS (m/z) : Calcd. C₂₆H₂₃N₄Cl (M⁺): 414, found (M+H)⁺ : 415.0.

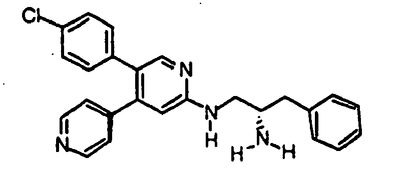

### Example 9e: 2-((S)-2-amino-3-phenylpropylamino)-5-(4-fluorophenyl)-4-(4-pyridinyl)pyridine

MS (*m*/*z*): Calcd. C₂₅H₂₃N₄F(M⁺): 398, found (M+H)⁺: 399.1

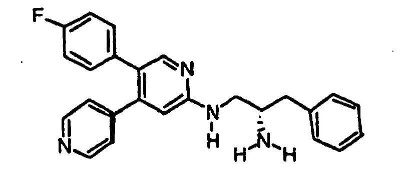

### Example 10

The following compounds were prepared according to Example 8 Step A (using 2-chloro-[4,4']-bipyridine and the corresponding amine described in Example 1), followed by Step B (bromination), and Step C (Suzuki coupling using the appropriate boronic acid):

### Example 10a: Preparation of 2-(3-amino-3-phenylpropylamino)-5-(3-methylphenyl)-4-(4-pyridinyl)pyridine

MS (m/z): Calcd. C₂₆H₂₆N₄ (M⁺) : 394, found (M+H)⁺ : 395.1

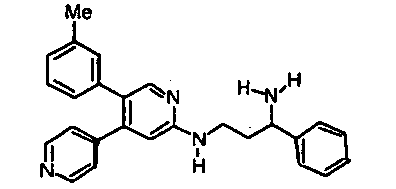

### Example 10b: Preparation of 2-(3-amino-3-phenylpropylamino)-5-(3-isopropylphenyl)-4-(4-pyridinyl)pyridine

MS (m/z): Calcd. C₂₈H₃₀N₄ (M⁺) : 422, found (M+H)⁺ : 422.9

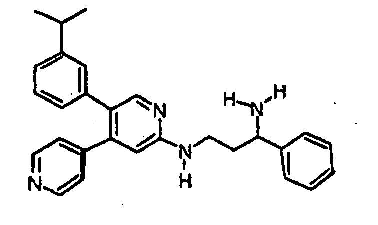

### Example 10c: Preparation of 2-(3-amino-3-phenylpropylamino) -5- (3-trifluoromethylphenyl) -4- (4-pyridinyl)pyridine

MS <*m*/*z*>: Calcd. C₂₆H₂₃N₄F₃ (M⁺): 448, found (M+H)⁺ : 449.4

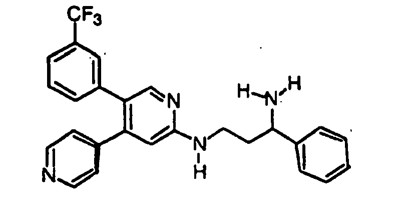

### Example 10d: Preparation of 2-(3-amino-3-phenylpropylamino)-5-(3-fluorophenyl)-4-(4-pyridinyl)pyridine

MS (m/z) : Calcd. C₂₅H₂₃N₄F (M⁺) : 398, found (M+H)⁺: 399.2

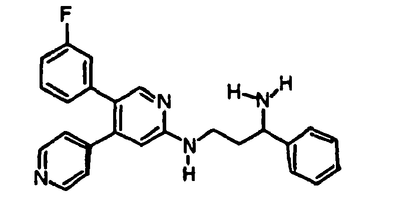

### Example 10e: Preparation of 2-(3-amino-3-phenylpropylamino)-5-(4-chlorolphenyl)-4-(4-pyridinyl)pyridine

MS (m/z): Calcd. C₂₆H₂₃N₄Cl (M⁺): 414, found (M+H)⁺ : 415.5.

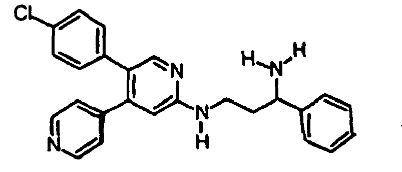

### Example 10f: Preparation of 2-(3-amino-3-phenylpropylamino)-5-(4-fluorophenyl)-4-(4-pyridinyl)pyridine

MS (m/z): Calcd. C₂₅H₂₃N₄F (M⁺): 398, found (M+H)⁺ : 399.1

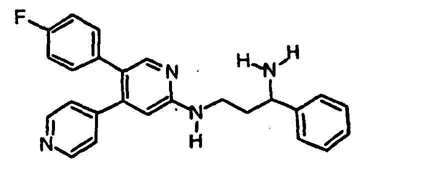

### Example 11

### Procedure for preparation of Preparation of 2-(3-phenylpropylamino)-5-(3-methylphenyl)-4-(4-pyridinyl)pyridine

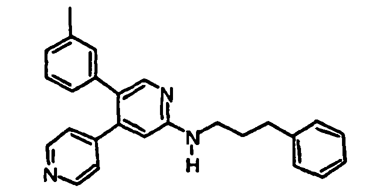

The title compound was prepared according to the procedure in Step L of Example 7 using 3-phenyl-propyl amine: MS (m/z): Calcd. C₂₆H₂₅N₃ (M⁺) : 379, found (M+H)⁺ : 380.3

### Example 12

### Procedure for preparation of 2-amino-[4,4']-bipyridine

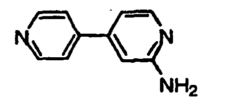

The title compound was prepared by heating 2-chloro-[4,4']bipyridinyl and NH₄OH (30% in H₂O) in a bomb at 210 °C for 48 hours:MS (*m*/*z*)*:* Calcd. C₁₀H₉N₃ (M⁺) : 171, found (M+H)⁺: 172.1

### Example 13

### Procedure for preparation of 2-(3-phenylpropylamino)-4-(3-methylphenyl)-5-(4-pyridyl)pyridine

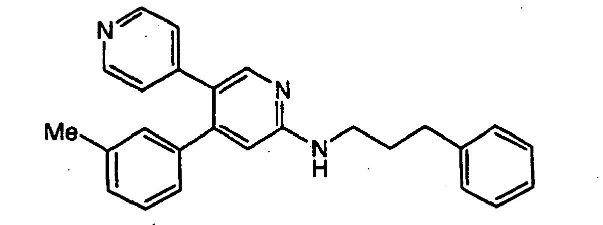

Step A: Preparation of 4-acetoxy-3-pyridine-4-yl-4-*m-*tolyl-cyclopent-2-en-1-one: To a solution of 4-hydroxy-4-pyridine-4-yl-3-*m*-tolyl-cyclopent-2-en-1-one, and it's regioisomer 4-hydroxy-3-pyridine-4-yl-4-m-tolyl-cyclopent-2-en-1-one prepared as described in Example 8, Step E (265 mg, 1.0 mmole) in methylene chloride (5 mL) was added dimethylamino pyridine (183 mg, 1.5 mmole) and acetic anhydride (0.12 mL, 1.2 mmole) at r.t.. The reaction mixture was stirred at that temperature for 1 hr before quenching the reaction with 1 mL of methanol. Concentration and purification (silica, hexane/ethyl acetate) gave the title compound as the slower eluting isomer. MS (m/z): Calcd. C₁₉H₁₇NO₃(M⁺) : 307, found (M+H)⁺ : 308.1.
Step B: Preparation of 6-acetoxy-4-m-tolyl-5,6-dihydro-1H-[5,4']bipyridinyl-2-one: To a solution of 4-acetoxy-3-pyridine-4-yl-4-*m*-tolyl-cyclopent-2-en-1-one (160 mg, 0.52 mmole) in chloroform (3 mL) at r.t. was added NaN, (85 mg, 1.3 mmole), and MsOH (0.3 mL). The reaction mixture was stirred at that reflux for 1.5 hour before quenching the reaction with sodium bicarbonate solution. Stanard basic work up, followed by purification gave the title compound. MS (m/z): Calcd. C₁₉H₁₈N₂O₃(M⁺): 322, found (M+H)⁺: 323.
Step C: Preparation of 6-hydroxy-4-*m*-tolyl-5,6-dihydro-1H-[5,4']bipyridinyl-2-one: To a solution of 6-acetoxy-4-m-tolyl-5,6-dihydro-1H-[5,4']bipyridinyl-2-one (200 mg, 0.6 mmole) in THF (2 mL) and water (2 mL) at r.t. was added LiOH (51 mg, 1.2 mmole) in one portion. The reaction mixture was stirred at that temperature for 10 min before quenching the mixture with aqueous NH₄Cl. The reaction was quenched with 1.45 mL of 1N HCl, the resulting white precipitate was filtered, rinsed with water and dried to give the title compound as a white solid.
Step D : preparation of 4-(3-methylphenyl)-5-(4-pyridyl)-1H-pyrid-2-one To a solution of 6-hydroxy-4-*m*-tolyl-5,6-dihydro-1H-[5,4']bipyridinyl-2-one (83 mg, 0.29 mmole) in CHCl, (3 mL) at r.t.was added 2 ml of conc. H₂SO₄. The resulting mixture was heated to 55 °C for 2 hr. The mixture was cooled down to r.t. and was carefully quenched with aqueous sodium carbonate. Standard work up (extraction of compound with methylene chloride), followed by purification (silica gel, methanol /methylene chloride) gave the title compound.
Step E: Preparation of 2-chloro-4-(3-methylphenyl)-5-(4-pyridyl)pyridine: 4-(3-methylphenyl)-5-(4-pyridyl)-1H-pyrid-2-one (33 mg, 0.13 mmole) in POC1, (2 mL) was heated to 105 °C for 12 hr. POCl₃ was removed under reduced pressure. The residue was diluted with methylene chloride and was carefully quenched with aqueous sodium carbonate. Standard work up, followed by purification (silica gel, hexane/ethyl acetate) gave the title compound. MS *(m*/*z):* Calcd. C₁₇H₁₃N₂Cl(M⁺): 280.5, found (M+H)⁺: 281 and 283.
Step F: Preparation of 2-(3-phenylpronylamino)-4-(3-methylphenyl)-5-(4-pyridyl)pyridine: A mixture of 2-chloro-4-(3-methylphenyl)-5-(4-pyridyl)pyridine (13 mg) and 3-phenylpropylamine (5 drops) was heated to 160 °C for 2 hr. The cooled reaction mixture was subjected to chromatographic purification (silica gel, methanol/methylene chloride) to give the title compound.

### Example 14

### Procedure for preparation of 2-((S)-2-amino-3-phenylpropoxy)-4-(3-methylphenyl)-5-(4-pyridyl)pyridine

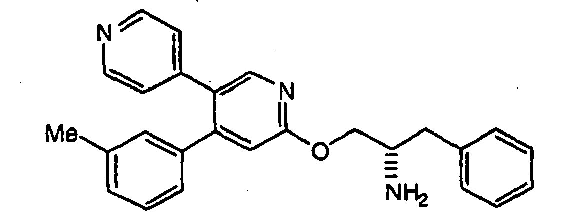

To a stirred mixture of 4-(3-methylphenyl)-5-(4-pyridyl)-1H-pyrid-2-one (12 mg, 0.05 mmole), (S)-2-tert-butoxycarbonylamino-3-phenylpropanol (15 mg, 0.06 mmole), triphenylphosphine (18 mg, 0.07 mmole), in methylene chloride (1 mL) at room temperature was added diethyl azodicarboxylate (12 mg, 0.07 mmol). When the reaction was complete (monitored by TLC), methanol was added (1 mL) and the reaction was concentrated and treated with 1 mL of 1:1 TFA/methanol for 30 minutes. The mixture was concentrated, neutralized with 1 drop conc NH₄OH, and purified (SiO₂, 10% methanol/methylene chloride) to give the title compound: MS (m/z): Calcd. C₂₆M₂₅N₃O (M⁺): 395, found (M+H)⁺ : 396.

### Example 15

### Procedure for preparation of 1- ((S) -2-amino-3-phenylpropyl)-4- (3-methylphenyl)-5- (9-pyridyl)-1H-pyrid-2-one

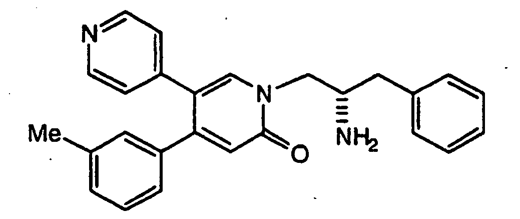

The title compound was obtained as a slower eluting byproduct from Example 14: MS (m/z): Calcd. C₂₆H₂₅N₃O (M⁺) : 395, found (M+H)⁺ : 396.

### Example 16

### Procedure for preparation of 2-(benzyloxy)-4-(3-methylphenyl)-5-(4-pyridyl)pyridine

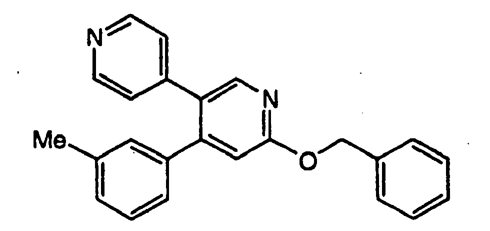

The title compound was obtained according to the procedure outlined in Example 14 using benzyl alcohol and was obtained as the faster eluting regio-isomer: MS (m/z): Calcd. C₂₄H₂₀N₂O (M⁺) : 352, found (M+H)⁺ : 353.

### Example 17

### Procedure for preparation of 1-benzyl-4-(3-methylphenyl)-5-(4-pyridyl)-1H-pyrid-2-one

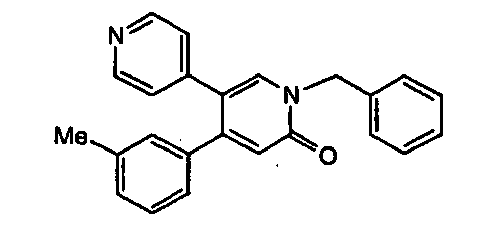

The title compound was obtained from the reaction outlined in Example 16 and was obtained as the faster eluting regio-isomer: MS (m/z): Calcd. C₂₄H₂₀N₂O (M⁺) : 352, found (M+H)⁺: 353.

### Example 18

### Procedure for preparation of 2-(3-phenylpropoxy)-4-(3-methylphenyl)-5-(4-pyridyl)pyridine

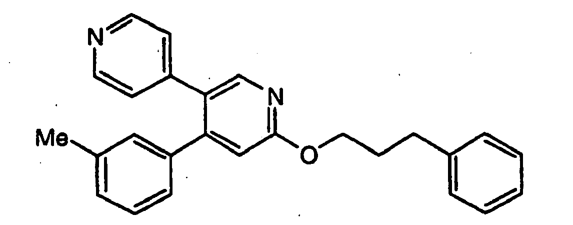

The title compound was obtained according to the procedure outlined in Example 14 using 3-phenylpropanol and was obtained as the faster eluting regio-isomer: MS *(m*/*z):* Calcd. C₂₆H₂₄N₂O (M⁺) : 380, found (M+H)⁺: 381.

### Example 19

### Procedure for preparation of 1-(3-phenylpropyl)-4-(3-methylphenyl)-5-(4-pyridyl)-1H-pyrid-2-one

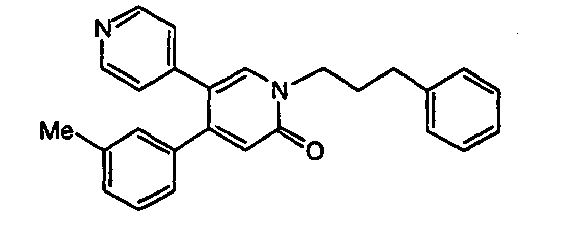

The title compound was obtained from the reaction outlined in Example 18, and was obtained as the slower eluting regio-isomer: MS *(m*/*z):* Calcd. C₂₆H₂₄N₂O (M⁺) : 380, found (M+H)⁺ : 381.

### Example 20

### Procedure for preparation of 2-(4-pyridylmethoxy)-4-(4-fluorophenyl)-5-(4-pyridyl)pyridine

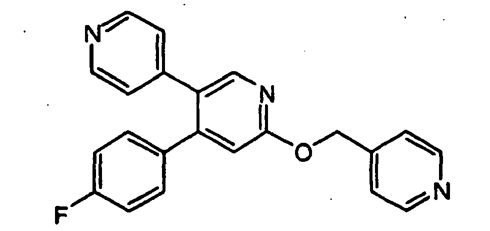

The title compound was obtained from the reaction outlined in Example 14 using 4-(4-fluorophenyl)-5-(4-pyridyl)-1H-pyrid-2-one and 4-pyridylcarbinol, and was obtained as the faster eluting isomer: MS (m/z): Calcd. C₂₃H₁₇N₂FO (M⁺) : 356, found (M+H)⁺ : 357.

### Example 21

### Procedure for preparation of 1-(4-pyridylmethoxy)-4-(4-fluorophenyl)-5-(4-pyridyl)-1H-pyrid-2-one

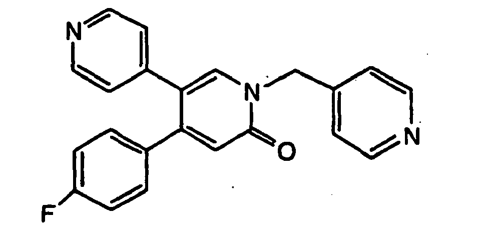

The title compound was obtained from the reaction outlined in Example 20, and was obtained as the slower eluting regio-isomer: MS (m/z): Calcd. C₂₃H₁₇N₂FO (M⁺) : 356, found (M+H)⁺ : 357.

### Example 22

### Biological Assays

The following assays were used to characterize the ability of compounds of the invention to inhibit the production of TNF-α and IL-1-β. The second assay measured the inhibition of TNF-α and/or IL-1-β in mice after oral administration of the test compounds. The third assay, a glucagon binding inhibition in vitro assay, can be used to characterize the ability of compounds of the invention to inhibit glucagon binding. The fourth assay, a Cyclooxygenase enzyme (COX-1 and COX-2) inhibition activity in vitro assay, can be used to characterize the ability of compounds of the invention to inhibit COX-1 and/or COX-2. The fifth assay, a Raf-kinase inhibition assay, can be used to characterize the compounds of the invention to inhibit phosphorylation of MEK by activated Raf-kinase.

### Lipopolysaccharide-activated monocyte TNF production assay

### Isolation of monocytes

Test compounds were evaluated *in vitro* for the ability to inhibit the production of TNF by monocytes activated with bacterial lipopolysaccharide (LPS). Fresh residual source leukocytes (a byproduct of plateletpheresis) were obtained from a local blood bank, and peripheral blood mononuclear cells (PBMCs) were isolated by density gradient centrifugation on Ficol-Paque Plus (Pharmacia). PBMCs were suspended at 2 x 10'/ml in DMEM supplemented to contain 2% FCS, 10 mM, 0.3 mg/ml glutamate, 100 U/ml penicillin G and 100 mg/ml streptomycin sulfate (complete media). Cells were plated into Falcon flat bottom, 96 well culture plates (200 µl/well) and cultured overnight at 37°C and 6% CO₂. Non-adherent cells were removed by washing with 200 µl/well of fresh medium. Wells containing adherent cells (~70% monocytes) were replenished with 100 µl of fresh medium.

### Preparation of test compound stock solutions

Test compounds were dissolved in DMZ. Compound stock solutions were prepared to an initial concentration of 10 - 50 µM. Stocks were diluted initially to 20 - 200 µM in complete media. Nine twofold serial dilutions of each compound were then prepared in complete medium.

### Treatment of cells with test compounds and activation of TNF production with lipopolysaccharide

One hundred microliters of each test compound dilution were added to microtiter wells containing adherent monocytes and 100 µl complete medium. Monocytes were cultured with test compounds for 60 min at which time 25 µl of complete medium containing 30 ng/ml lipopolysaccharide from E. coli K532 were added to each well. Cells were cultured an additional 4 hrs. Culture supernatants were then removed and TNF presence in the supernatants was quantified using an ELISA.

### TNF ELISA

Flat bottom, 96 well Corning High Binding ELISA plates were coated overnight (4°C) with 150 µL/well of 3 µg/ml murine anti-human TNF-α MAb (R&D Systems #MAB210). Wells were then blocked for 1 hr at room temperature with 200 µL/well of CaCl₂-free ELISA buffer supplemented to contain 20 mg/ml BSA (standard ELISA buffer: 20 mM, 150 mM NaCl, 2 mM CaCl₂, 0.15 mM thimerosal, pH 7.4). Plates were washed and replenished with 100 µl of test supernatants (diluted 1:3) or standards. Standards consisted of eleven 1.5-fold serial dilutions from a stock of 1 ng/ml recombinant human TNF (R&D Systems). Plates were incubated at room temperature for 1 hr on orbital shaker (300 rpm), washed and replenished with 100 µl/well of 0.5 µg/ml goat anti-human TNF-α (R&D systems #AB-210-NA) biotinylated at a 4:1 ratio. Plates were incubated for 40 min, washed and replenished with 100 µl/well of alkaline phosphatase-conjugated streptavidin (Jackson ImmunoResearch #016-050-084) at 0.02 µg/ml. Plates were incubated 30 min, washed and replenished with 200 µl/well of 1 mg/ml of p-nitrophenyl phosphate. After 30 min, plates were read at 405 nm on a Vₘₐₓ plate reader.

### Data analysis

Standard curve data were fit to a second order polynomial and unknown TNF-α concentrations determined from their OD by solving this equation for concentration. TNF concentrations were then plotted vs. test compound concentration using a second order polynomial. This equation was then used to calculate the concentration of test compounds causing a 50% reduction in TNF production.

Compounds of the invention can also be shown to inhibit LPS-induced release of IL-1β, IL-6 and/or IL-8 from monocytes by measuring concentrations of IL-1β, IL-6 and/or IL-8 by methods well known to those skilled in the art. In a similar manner to the above described assay involving the LPS induced release of TNF-α from monocytes, compounds of this invention can also be shown to inhibit LPS induced release of IL-1β, IL-6 and/or IL-8 from monocytes by measuring concentrations of IL-1β, IL-6 and/or IL-8 by methods well known to those skilled in the art. Thus, the compounds of the invention may lower elevated levels of TNF-α, IL-1, IL-6, and IL-8 levels. Reducing elevated levels of these inflammatory cytokines to basal levels or below is favorable in controlling, slowing progression, and alleviating many disease states. All of the compounds are useful in the methods of treating disease states in which TNF-α, IL-1β, IL-6, and IL-8 play a role to the full extent of the definition of TNF-α-mediated diseases described herein.

### Inhibition of LPS-Induced TNF-α production in mice

Male DBA/1LACJ mice were dosed with vehicle or test compounds in a vehicle (the vehicle consisting of 0.5% tragacanth in 0.03 N HCl) 30 minutes prior to lipopolysaccharide (2 mg/kg, I.V.) injection. Ninety minutes after LPS injection, blood was collected and the serum was analyzed by ELISA for TNF levels.
The following compounds exhibit activities in the monocyte assay (LPS induced TNF release) with IC₅₀ values of 20 µM or less:
1-(3-phenylpropyl)-4-(3-methylphenyl)-5-(4-pyridyl)-1H-pyrid-2-one
2-(3-phenylpropoxy)-4-(3-methylphenyl)-5-(4-pyridyl)pyridine
1-((S)-2-amino-3-phenylpropyl)-4-(3-methylphenyl)-5-(4-pyridyl)-1*H*-pyrid-2-one
2-((S)-2-amino-3-phenylpropoxy)-4-(3-methylphenyl)-5-(4-pyridyl)pyridine
2-(3-amino-3-phenylpropylamino)-5-(4-fluorophenyl)-4-(4-pyridinyl)pyridine
2-(3-amino-3-phenylpropylamino)-5-(4-chlorolphenyl)-4-(4-pyridinyl)pyridine
2-(3-amino-3-phenylpropylamino)-5-(3-fluorophenyl)-4-(9-pyridinyl)pyridine
2-(3-amino-3-phenylpropylamino)-5-(3-trifluoromethylphenyl)-4-(4-pyridinyl)pyridine
2-(3-amino-3-phenylpropylamino)-5-(3-isopropylphenyl)-4-(4-pyridinyl)pyridine
2-(3-amino-3-phenylpropylamino)-5-(3-methylphenyl)-4-(4-pyridinyl)pyridine
2-((S)-2-amino-3-phenylpropylamino)-5-(4-fluorophenyl)-4-(4-pyridinyl)pyridine
2-((S)-2-amino-3-phenylpropylamino)-5-(4-chlorophenyl)-4-(4-pyridinyl)pyridine
2-((S)-2-amino-3-phenylpropylamino)-5-(3-fluorophenyl-4-(4-pyridinyl)pyridine
2-((S)-2-amino-3-phenylpropylamino)-5-(3-trifluoromethylphenyl-4-(4-pyridinyl)pyridine
2-((S)-2-amino-3-phenylpropylamino)-5-(3-isopropylphenyl)-4-(4-pyridinyl)pyridine
6-[((S)-2-amino-3-phenylpropyl)-amino]-3-(4-fluorophenyl)-4-(4-pyridyl)-pyridazine
6-((S)-2-Amino-3-phenylpropylamino)-3-(2-benzothiophenyl)-2-(4-pyridyl)pyridine
6-((S)-2-Amino-3-phenylpropylamino)-3-(4-chloro-3-fluorophenyl)-2-(4-pyridyl)pyridine
6-((S)-2-Amino-3-phenylpropylamino)-3-(4-methoxyphenyl)-2-(4-pyridyl)pyridine
6-((S)-2-Amino-3-phenylpropylamino)-3-(3-isopropylphenyl)-2-(4-pyridyl)pyridine
6-((S)-2-Amino-3-phenylpropylamino)-3-(4-chlorophenyl)-2-(4-pyridyl)pyridine
6-((S)-2-Amino-3-phenylpropylamino)-3-(2-napthyl)-2-(4-pyridyl)pyridine
6-((S)-2-Amino-3-phenylpropylamino)-3-(3-trifluoromethylphenyl)-2-(4-pyridyl)pyridine
6-((S)-2-Amino-3-phenylpropylamino)-3-(3-methylphenyll-2-(4-pyridyl)pyridine
The following compounds exhibit activities in the monocyte assay (LPS induced TNF release) with IC₅₀ values of 5 µM or less:
1-((S)-2-amino-3-phenylpropyl)-4-(3-methylphenyl)-5-(4-pyridyl)-*1H*-pyrid-2-one
2-(3-amino-3-phenylpropylamino)-5-(4-fluorophenyl)-4-(4-pyridinyl)pyridine
2-(3-amino-3-phenylpropylamino)-5-(4-chlorolphenyl)-4-(4-pyridinyl)pyridine
2-(3-amino-3-phenylpropylamino)-5-(3-fluorophenyl)-4-(4-pyridinyl)pyridine
2-(3-amino-3-phenylpropylamino)-5-(3-trifluoromethylphenyl)-4-(4-pyridinyl)pyridine
2-(3-amino-3-phenylpropylamino)-5-(3-isopropylphenyl)-4-(4-pyridinyl)pyridine
2-(3-amino-3-phenylpropylamino)-5-(3-methylphenyl)-4-(4-pyridinyl)pyridine
2-((S)-2-amino-3-phenylpropylamino)-5-(4-fluorophenyl)-4-(4-pyridinyl)pyridine
2-((S)-2-amino-3-phenylpropylamino)-5-(4-chlorophenyl)-4-(4-pyridinyl)pyridine
2-((S)-2-amino-3-phenylpropylamino)-5-(3-fluorophenyl-4-(4-pyridinyl)pyridine
2-((S)-2-amino-3-phenylpropylamino)-5-(3-trifluoromethylphenyl-4-(4-pyridinyl)pyridine
2-((S)-2-amino-3-phenylpropylamino)-5-(3-isopropylphenyl)-4-(4-pyridinyl)pyridine
6-[((S)-2-amino-3-phenylpropyl)-amino]-3-(4-fluorophenyl)-4-(4-pyridyl)-pyridazine
6-((S)-2-Amino-3-phenylpropylamino)-3-(2-benzothiophenyl)-2-(4-pyridyl)pyridine
6-((S)-2-Amino-3-phenylpropylamino)-3-(4-chloro-3-fluorophenyl)-2-(4-pyridyl)pyridine
6-((S)-2-Amino-3-phenylpropylamino)-3-(4-methoxyphenyl)-2-(4-pyridyl)pyridine
6-((S)-2-Amino-3-phenylpropylamino)-3-(3-isopropylphenyl)-2-(4-pyridyl)pyridine
6-((S)-2-Amino-3-phenylpropylamino)-3-(4-chlorophenyl)-2-(4-pyridyl)pyridine
6-((S)-2-Amino-3-phenylpropylamino)-3-(2-napthyl)-2-(4-pyridyl)pyridine
6-((S)-2-Amino-3-phenylpropylamino)-3-(3-trifluoromethylphenyl)-2-(4-pyridyl)pyridine
6-((S)-2-Amino-3-phenylpropylamino)-3-(3-methylphenyl)-2-(4-pyridyl)pyridine.

Compounds of the invention may be shown to have anti-inflammatory properties in animal models of inflammation, including carageenan paw edema, collagen induced arthritis and adjuvant arthritis, such as the carageenan paw edema model (C. A. Winter et al Proc. Soc. Exp. Biol. Med. (1962) vol 111, p 544; K. F. Swingle, in R. A. Scherrer and M. W. Whitehouse, Eds., Antiinflammatory Agents, Chemistry and Pharmacology, Vol. 13-II, Academic, New York, 1974, p. 33) and collagen induced arthritis (D. E. Trentham et al J. Exp. Med. (1977) vol. 146, p 857; J. S. Courtenay, Nature (New Biol.) (1980), Vol 283, p 666).

### ¹²³I-Glucagon Binding Screen with CHO/hGLUR Cells

The assay is described in WO 97/16442, which is incorporated herein by reference in its entirety.

### Reagents

The reagents can be prepared as follows: (a) prepare fresh 1M o-Phenanthroline (Aldrich) (198.2 mg/ml ethanol); (b) prepare fresh 0.5M DTT (Sigma); (c) Protease Inhibitor Mix (1000X): 5 mg leupeptin, 10 mg benzamidine, 40 mg bacitracin and 5 mg soybean trypsin inhibitor per ml DMSO and store aliquots at -20°C; (d) 250 µM human glucagon (Peninsula): solubilize 0.5 mg vial in 575 µl 0.1N acetic acid (1 µl yields 1 µM final concentration in assay for non-specific binding) and store in aliquots at -20°C; (e) Assay Buffer: 20mM Tris (pH 7.8), 1 mM DTT and 3 mM o-phenanthroline; (f) Assay Buffer with 0.1% BSA (for dilution of label only; 0.01% final in assay): 10 µl 10% BSA (heat-inactivated) and 990 µl Assay Buffer; (g) ¹²⁵I-Glucagon (NEN, receptor-grade, 2200 Ci/mmol): dilute to 50,000 cpm/25 µl in assay buffer with BSA (about 50pM final concentration in assay).

### Harvesting of CHO/hGLUR Cells for Assay

1. Remove media from confluent flask then rinse once each with PBS (Ca, Mg-free) and Enzyme-free Dissociation Fluid (Specialty Media, Inc.).
2. Add 10 ml Enzyme-free Dissoc. Fluid and hold for about 4 min. at 37°C.
3. Gently tap cells free, triturate, take aliquot for counting and centrifuge remainder for 5 min. at 1000 rpm .
4. Resuspend pellet in Assay Buffer at 75000 cells per 100 µl.

Membrane preparations of CHO/hGLUR cells can be used in place of whole cells at the same assay volume. Final protein concentration of a membrane preparation is determined on a per batch basis.

### Assay

The determination of inhibition of glucagon binding can be carried out by measuring the reduction of I¹²⁵-glucagon binding in the presence of compounds of Formula I. The reagents are combined as follows:

| | Compound/ Vehicle | 250 µM Glucagon | ¹²⁵I-Glucagon | CHO/hGLUR Cells |
|---|---|---|---|---|
| Total Binding | --/5 µl | -- | 25 µl | 100 µl |
| + Compound | 5 µl/-- | -- | 25 µl | 100 µl |
| Nonspecif ic Binding | --/5 µl | 1 µl | 25 µl | 100 µl |

The mixture is incubated for 60 min. at 22°C on a shaker at 275 rpm. The mixture is filtered over pre-soaked (0.5% polyethylimine (PEI)) GF/C filtermat using an Innotech Harvester or Tomtec Harvester with four washes of ice-cold 20mM Tris buffer (pH 7.8). The radioactivity in the filters is determined by a gamma-scintillation counter.

Thus, compounds of the invention may also be shown to inhibit the binding of glucagon to glucagon receptors.

### Cyclooxygenase Enzyme Activity Assay

The human monocytic leukemia cell line, THP-1, differentiated by exposure to phorbol esters expresses only COX-1; the human osteosarcoma cell line 143B expresses predominantly COX-2. THP-1 cells are routinely cultured in RPMI complete media supplemented with 10% FBS and human osteosarcoma cells (HOSC) are cultured in minimal essential media supplemented with 10% fetal bovine serum (MEM-10%FBS); all cell incubations are at 37°C in a humidified environment containing 5% CO₂.

### COX-1 Assay

In preparation for the COX-1 assay, THP-1 cells are grown to confluency, split 1:3 into RPMI containing 2% FBS and 10 mM phorbol 12-myristate 13-acetate (TPA), and incubated for 48 hours on a shaker to prevent attachment. Cells are pelleted and resuspended in Hank's Buffered Saline (HBS) at a concentration of 2.5 x 10⁶ cells/mL and plated in 96-well culture plates at a density of 5 x 10⁵ cells/mL. Test compounds are diluted in HBS and added to the desired final concentration and the cells are incubated for an additional 4 hours. Arachidonic acid is added to a final concentration of 30 mM, the cells incubated for 20 minutes at 37°C, and enzyme activity determined as described below.

### COX-2 Assay

For the COX-2 assay, subconfluent HOSC are trypsinized and resuspended at 3 x 10⁶ cells/mL in MEM-FBS containing 1 ng human IL-1b/mL, plated in 96-well tissue culture plates at a density of 3 x 10⁴ cells per well, incubated on a shaker for 1 hour to evenly distribute cells, followed by an additional 2 hour static incubation to allow attachment. The media is then replaced with MEM containing 2% FBS (MEM-2%FBS) and 1 ng human IL-1b/mL, and the cells incubated for 18-22 hours. Following replacement of media with 190 mL MEM, 10 mL of test compound diluted in HBS is added to achieve the desired concentration and the cells incubated for 4 hours. The supernatants are removed and replaced with MEM containing 30 mM arachidonic acid, the cells incubated for 20 minutes at 37°C, and enzyme activity determined as described below.

### COX Activity Determined

After incubation with arachidonic acid, the reactions are stopped by the addition of 1 N HCl, followed by neutralization with 1 N NaOH and centrifugation to pellet cell debris. Cyclooxygenase enzyme activity in both HOSC and THP-1 cell supernatants is determined by measuring the concentration of PGE₂ using a commercially available ELISA (Neogen #404110). A standard curve of PGE₂ is used for calibration, and commercially available COX-1 and COX-2 inhibitors are included as standard controls.

### Raf Kinase assay

*In vitro* Raf kinase activity is measured by the extent of phosphorylation of the substrate MEK (Map kinase/ERK kinase) by activated Raf kinase, as described in GB 1,238,959 (incorporated herein by reference in its entirety). Phosphorylated MEK is trapped on a filter and incorporation of radiolabeled phosphate is quantified by scintillation counting.

### MATERIALS:

Activated Raf is produced by triple transfection of Sf9 cells with baculoviruses expressing "Glu-Glu"-epitope tagged Raf,val¹²-H-Ras, and Lck. The "Glu-Glu"-epitope, Glu-Try-Met-Pro-Met-Glu, was fused to the carboxy-terminus of full length c-Raf.
Catalytically inactive MEK (K97A mutation) is produced in Sf9 cells transfected with a baculovirus expressing c-terminus "Glu-Glu" epitope-tagged K97A MEK1.
Anti "Glu-Glu" antibody was purified from cells grown as described in: Grussenmeyer, et al., Proceedings of the National Academy of Science, U.S.A. pp 7952-7954, 1985.
Column buffer: 20 mM Tris pH=8, 100 mM NaCl, 1 mM EDTA, 2.5 mM EGTA, 10 mM MgCl₂, 2 mM DTT, 0.4 mM AEBSF, 0.1% n-octylglucopyranoside, 1 nM okadeic acid, and 10 µg/mL each of benzamidine, leupeptin, pepstatin, and aprotinin.
5x Reaction buffer: 125 mM HEPES pH=8, 25 mM MgCl₂, 5 mM EDTA, 5 mM Na₃VO₄, 100 µg/mL BSA.
Enzyme dilution buffer: 25 mM HEPES pH=8, 1 mM EDTA, 1 mM Na₃VO₄, 400 µg/mL BSA.
Stop solution: 100 mM EDTA, 80 mM sodium pyrophosphate. Filter plates: Milipore multiscreen # SE3MO78E3, Immobilon-P (PVDF).

### METHODS:

Protein purification: Sf9 cells were infected with baculovirus and grown as described in Williams, et al., Proceedings of the National Academy of Science, U.S.A. pp 2922-2926, 1992. All subsequent steps were preformed on ice or at 4°C. Cells were pelleted and lysed by sonication in column buffer. Lysates were spun at 17,000xg for 20 min, followed by 0.22 µm filtration. Epitope tagged proteins were purified by chromatography over GammaBind Plus affinity column to which the "Glu-Glu" antibody was coupled. Proteins were loaded on the column followed by sequential washes with two column volumes of column buffer, and eluted with 50 µg/mL Glu-Tyr-Met-Pro-Met-Glu in column buffer.
Raf kinase assay: Test compounds were evaluated using ten 3-fold serial dilutions starting at 10 - 100 µM. 10 µL of the test inhibitor or control, dissolved in 10% DMSO, was added to the assay plate followed by the addition of 30 µL of the a mixture containing 10 µL 5x reaction buffer, 1mM ³³P-γ-ATP (20 µCi/mL), 0.5 µL MEK (2.5 mg/mL), 1 µL 50 mM β-mercaptoethanol The reaction was started by the addition of 10 µL of enzyme dilution buffer containing 1 mM DTT and an amount of activated Raf that produces linear kinetics over the reaction time course. The reaction was mixed and incubated at room temperature for 90 min. and stopped by the addition of 50 µL stop solution. 90 µL aliquots of this stopped solution were transferred onto GFP-30 cellulose microtiter filter plates (Polyfiltronics), the filter plates washed in four well volumes of 5% phosphoric acid, allowed to dry, and then replenished with 25 µl scintillation cocktail. The plates were counted for ³³P gamma emission using a TopCount Scintillation Reader.

Accordingly, the compounds of the invention or a pharmaceutical composition thereof are useful for prophylaxis and treatment of rheumatoid arthritis; Pagets disease; osteophorosis; multiple myeloma; uveititis; acute and chronic myelogenous leukemia; pancreatic β cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; Reiter's syndrome; type I and type II diabetes; bone resorption diseases; graft vs. host reaction; ischemia reperfusion injury; atherosclerosis; brain trauma; Alzheimer's disease; stroke; myocardial infarction; multiple sclerosis; cerebral malaria; sepsis; septic shock; toxic shock syndrome; fever, and myalgias due to infection. HIV-1, HIV-2, HIV-3, cytomegalovirus (CMV), influenza, adenovirus, the herpes viruses (including HSV-1, HSV-2), and herpes zoster, all of which are sensitive to TNF-α and/or IL-1 inhibition or glucagon antagonism, will also be positively effected by the compounds and methods of the invention.

The compounds of the present invention may also possess oncolytic characteristics and may be useful for the treatment of cancer. The compounds of the present invention may also block signal transduction by extracellular mitogenic stimuli and oncoproteins through inhibition of Raf kinase. Thus the compounds of the present invention, a pharmaceutical salt thereof, or a pharmaceutical composition of either, may also be useful in the prophylaxis and/or treatment of cancers which are mediated by Raf and Raf-inducable proteins, such as cancers where Raf kinase is implicated by overexpression and cancers involving overexpression of upstream activators of Raf or Raf-activating oncogenes. Examples of cancers where Raf kinase is implicated by overexpression include cancers of the brain, larynx, lung, lymphatic system, urinary tract and stomach, including hystocytic lymphoma, lung adenocarcinoma, small cell lung cancers and the like. Examples of cancers involving overexpression of upstream activators of Raf or Raf-activating oncogenes, include pancreatic carcinoma, breast carcinoma and the like.

The compounds of the present invention also may possess analgesic properties and may be useful for the treatment of pain disorders, such as hyperalgesia due to excessive IL-1. The compounds of the present invention may also prevent the production of prostaglandins by inhibition of enzymes in the human arachidonic acid/prostaglandin pathway, including cyclooxygenase (WO 96/03387, incorporated herein by reference in its entirety).

Because of their ability to lower TNF-α and IL-1 concentrations or inhibit glucagon binding to its receptor, the compounds of the invention are also useful research tools for studying the physiology associated with blocking these effects.

The methods of the invention comprise administering an effective dose of a compound of the invention, a pharmaceutical salt thereof, or a pharmaceutical composition of either, to a subject (i.e., an animal, preferably a mammal, most preferably a human) in need of a reduction in the level of TNF-α, IL-1, IL-6, and/or IL-8 levels and/or reduction in plasma glucose levels and/or which subject may be suffering from rheumatoid arthritis; Pagets disease; osteophorosis; multiple myeloma; uveititis; acute and chronic myelogenous leukemia; pancreatic ß cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; Reiter's syndrome; type I and type II diabetes; cancer; bone resorption diseases; graft vs. host reaction; Alzheimer's disease; stroke; myocardial infarction; ischemia reperfusion injury; atherosclerosis; brain trauma; multiple sclerosis; cerebral malaria; sepsis; septic shock; toxic shock syndrome; fever, and myalgias due to infection, or which subject is infected by HIV-1, HIV-2, HIV-3, cytomegalovirus (CMV), influenza, adenovirus, the herpes viruses (including HSV-1, HSV-2), or herpes zoster.

In another aspect, this invention comprises the use of a compound of the invention, or pharmaceutically acceptable salts thereof, in the manufacture of a medicament for the treatment either acutely or chronically of a TNF-α, IL-1β, IL-6, and/or IL-8 mediated disease state, including those described previously. The compounds of the present are also useful in the manufacture of an anti-cancer medicant. The compounds of the present invention are also useful in the manufacture of a medicant to attenuate or prevent signal transduction by extracellular mitogenic stimuli and oncoproteins through inhibition of Raf kinase. Also, the compounds of this invention are useful in the manufacture of a analgesic medicament and a medicament for treating pain disorders, such as hyperalgesia. The compounds of the present invention also are useful in the manufacture of a medicament to prevent the production of prostaglandins by inhibition of enzymes in the human arachidonic acid/prostaglandin pathway.

A further method of the invention comprises administering an effective dose of a compound of the invention, a pharmaceutical salt thereof, or a pharmaceutical composition of either, to a subject (i.e., an animal, preferably a mammal, most preferably a human) in need of prophylaxis and/or treatment of a cancer(s) which is mediated by Raf, Raf-inducable proteins and/or activators of Raf or Raf-activating oncogenes, and/or which subject may be suffering from cancers of the brain, larynx, lung, lymphatic system, urinary tract and stomach, including hystocytic lymphoma, lung adenocarcinoma, small cell lung cancers, pancreatic carcinoma, breast carcinoma and the like. Further, the compounds of this invention may be useful in the manufacture of a medicament for treating cancers, such as cancers of the brain, larynx, lung, lymphatic system, urinary tract and stomach, including hystocytic lymphoma, lung adenocarcinoma, small cell lung cancers, pancreatic carcinoma, breast carcinoma and the like.

In still another aspect, this invention provides a pharmaceutical composition comprising an effective TNF-α, IL-1β, IL-6, and/or IL-8 lowering amount and/or effective plasma glucose level lowering amount and/or effective tumor supressing amount of a compound of the invention and a pharmaceutically acceptable carrier or diluent, and if desired other active ingredients. The compounds of the invention are administered by any suitable route, preferably in the form of a pharmaceutical composition adapted to such a route, and in a dose effective for the treatment intended. Therapeutically effective doses of the compounds of the present invention required to arrest the progress or prevent tissue damage associated with the disease are readily ascertained by one of ordinary skill in the art using standard methods.

For the treatment of TNF-α, IL-1β, IL-6, and IL-8 mediated diseases, cancer, and/or hyperglycemia, the compounds of the present invention may be administered orally, parentally, by inhalation spray, rectally, or topically in dosage unit formulations containing conventional pharmaceutically acceptable carriers, adjuvants, and vehicles. The term parenteral as used herein includes, subcutaneous, intravenous, intramuscular, intrasternal, infusion techniques or intraperitoneally.

The dosage regimen for treating a TNF-α, IL-1, IL-6, and IL-8 mediated diseases, cancer, and/or hyperglycemia with the compounds of this invention and/or compositions of this invention is based on a variety of factors, including the type of disease, the age, weight, sex, medical condition of the patient, the severity of the condition, the route of administration, and the particular compound employed. Thus, the dosage regimen may vary widely, but can be determined routinely using standard methods. Dosage levels of the order from about 0.01 mg to 30 mg per kilogram of body weight per day, preferably from about 0.1 mg to 10 mg/kg, more preferably from about 0.25 mg to 1 mg/kg are useful for all methods of use disclosed herein.

The pharmaceutically active compounds of this invention can be processed in accordance with conventional methods of pharmacy to produce medicinal agents for administration to patients, including humans and other mammals.

For oral administration, the pharmaceutical composition may be in the form of, for example, a capsule, a tablet, a suspension, or liquid. The pharmaceutical composition is preferably made in the form of a dosage unit containing a given amount of the active ingredient. For example, these may contain an amount of active ingredient from about 1 to 2000 mg, preferably from about 1 to 500 mg, more preferably from about 5 to 150 mg. A suitable daily dose for a human or other mammal may vary widely depending on the condition of the patient and other factors, but, once again, can be determined using routine methods.

The active ingredient may also be administered by injection as a composition with suitable carriers including saline, dextrose, or water. The daily parenteral dosage regimen will be from about 0.1 to about 30 mg/kg of total body weight, preferably from about 0.1 to about 10 mg/kg, and more preferably from about 0.25 mg to 1 mg/kg.

Injectable preparations, such as sterile injectable aqueous or oleaginous suspensions, may be formulated according to the known are using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at ordinary temperatures but liquid at the rectal temperature and will therefore melt in the rectum and release the drug.

A suitable topical dose of active ingredient of a compound of the invention is 0.1 mg to 150 mg administered one to four, preferably one or two times daily. For topical administration, the active ingredient may comprise from 0.001% to 10% w/w, e.g., from 1% to 2% by weight of the formulation, although it may comprise as much as 10% w/w, but preferably not more than 5% w/w, and more preferably from 0.1% to 1% of the formulation.

Formulations suitable for topical administration include liquid or semi-liquid preparations suitable for penetration through the skin (e.g., liniments, lotions, ointments, creams, or pastes) and drops suitable for administration to the eye, ear, or nose.

For administration, the compounds of this invention are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinyl-pyrrolidine, and/or polyvinyl alcohol, and tableted or encapsulated for conventional administration. Alternatively, the compounds of this invention may be dissolved in saline, water, polyethylene glycol, propylene glycol, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The pharmaceutical compositions may be made up in a solid form (including granules, powders or suppositories) or in a liquid form (e.g., solutions, suspensions, or emulsions). The pharmaceutical compositions may be subjected to conventional pharmaceutical operations such as sterilization and/or may contain conventional adjuvants, such as preservatives, stabilizers, wetting agents, emulsifiers, buffers etc.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting, sweetening, flavoring, and perfuming agents.

Compounds of the present invention can possess one or more asymmetric carbon atoms and are thus capable of existing in the form of optical isomers as well as in the form of racemic or non-racemic mixtures thereof. The optical isomers can be obtained by resolution of the racemic mixtures according to conventional processes, *e.g.,* by formation of diastereoisomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereoisomers by crystallization followed by liberation of the optically active bases from these salts. A different process for separation of optical isomers involves the use of a chiral chromatography column optimally chosen to maximize the separation of the enantiomers. Still another available method involves synthesis of covalent diastereoisomeric molecules by reacting compounds of the invention with an optically pure acid in an activated form or an optically pure isocyanate. The synthesized diastereoisomers can be separated by conventional means such as chromatography, distillation, crystallization or sublimation, and then hydrolyzed to deliver the enantiomerically pure compound. The optically active compounds of the invention can likewise be obtained by using active starting materials. These isomers may be in the form of a free acid, a free base, an ester or a salt.

The compounds of the present invention can be used in the form of salts derived from inorganic or organic acids. The salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hyroxy-ethanesulfonate, lactate, maleate, methansulfonate, nicotinate, 2-naphthalenesulfonate, oxalate, palmoate, pectinate, persulfate, 2-phenylpropionate, picrate, pivalate, propionate, succinate, tartrate, thiocyanate, tosylate, mesylate, and undecanoate. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids that may be employed to from pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

While the compounds of the invention can be administered as the sole active pharmaceutical agent, they can also be used in combination with one or more compounds of the invention or other agents. When administered as a combination, the therapeutic agents can be formulated as separate compositions that are given at the same time or different times, or the therapeutic agents can be given as a single composition.

The foregoing is merely illustrative of the invention and is not intended to limit the invention to the disclosed compounds. Variations and changes which are obvious to one skilled in the art are intended to be within the scope and nature of the invention which are defined in the appended claims.

From the foregoing description, one skilled in the art can easily ascertain the essential characteristics of this invention, and without departing from the spirit and scope thereof, can make various changes and modifications of the invention to adapt it to various usages and conditions.

## Claims

1. A compound of formula 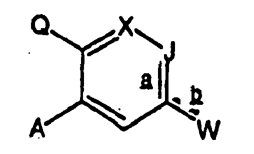 or a pharmaceutically acceptable salt thereof, wherein
W is R₁, R₂ or O;
A is R₁₁ and Q is R₁₂ or vice versa;
X is N or C-H;
J is N-R, N, C-R₁ or C-R₂, provided at least one of X or J is N or N-R₃; and
when W is R₃, then a is a double bond, b is a single bond and J is other than N-R₃ or C-R₁; when W is R₂, then a is a double bond, b is a single bond and J is other than N-R₃ or C-R₂; and when W is O, then a is a single bond, b is a double bond and J is N-R₃;
R₁ is -Z-Y or -Y; and each R₃ is independently a hydrogen radical or -Z-Y; provided that the total number of aryl, heteroaryl, cycloalkyl and heterocyclyl radicals in R₁, R₂ and R₃ is 0-2;
R₂ is (1) hydrogen, halo, trifluoromethyl or cyano radical; or
(2) C₁-C₄ alkyl radical optionally substituted by (a) 1-2 radicals of amino, C₁-C₄ alkylamino or di-(C₁-C₄ alkyl) amino;
each Z is independently a
(1) C₁-C₄ alkyl radical optionally substituted by (a) 1-2 radicals of amino, di-(C₁-C₂ alkyl) amino, hydroxy, C₁-C₂ alkylthio, and (b) an aryl radical or
(2) a heterocyclyl radical optionally substituted by 1-2 radicals of C₁-C₂ alkyl or aryl-C₁-C₂ alkyl radicals; wherein the aryl radicals are optionally substituted by 1-2 radicals of amino, di- (C₁-C₂ alkyl) amino, hydroxy, C₁-C₂ alkoxy, C₁-C₂ alkylthio, cyano, halo, C₁-C₂ alkyl or trifluoromethyl radicals;
each Y is independently a (1) hydrogen radical;
(2) -C(O)-R₂₀ or -C(O)-NR₅R₂₁ radical;
(3) -OR₂₁, -SR₂₁, -S(O)-R₂₀, -S(O)₂-R₂₀ or -S(O)₂-NR₅R₂₁ radical; or
(4) -NR₅R₂₁ or -NR₂₂-C(O)-R₂₁ radical;
each R₅ is independently
(1) hydrogen radical;
(2) C₁-C₄ alkyl radical optionally substituted by 1-3 radicals of amino, di-(C₁-C₂-alkyl)amino, hydroxy, C₁-C₂ alkoxy, C₁-C₂ alkylthio or halo; or
(3) phenyl-C₁-C₂-alkyl, heteroaryl-C₁-C₂-alkyl, heterocyclyl-C₁-C₂-alkyl or C₁-C₆-cycloalkyl-C₁-C₂-alkyl radicals optionally substituted by 1-3 radicals of amino, di-(C₁-C₂-alkyl)amino, hydroxy, C₁-C₂ alkoxy, C₁-C₂ alkylthio, methoxy, methylthio, C₁-C₄ alkyl or trifluoromethyl radicals;
each R₂₀ is independently
(1) C₁-C₈ alkyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino,-.hydro,xy, C₁-C₄ alkoxy, C₁-C₄ alkylthio, C₁-C₄ alkylsulfinyl, C₁-C₄ alkylsulfonyl, halo or C₃-C₆ cycloalkyl, heterocyclyl, aryl or heteroaryl radicals optionally substituted by 1-2 radicals of amino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of hydroxy, C₁-C₄ alkoxy, C₁-C₄ alkylthio or C₁-C₄ alkyl; or
(3) aryl or heteroaryl radicals optionally substituted by 1-2 radicals of (C₁-C₄ alkoxy)carbonyl, amino, C₁-C₄ alkylamino, di-(C₁-C₄ alkyl)amino, hydroxy, C₁-C₄ alkoxy, C₁-C₄ alkylthio, cyano, halo, azido, C₁-C₄ alkyl or trifluoromethyl radicals;
each R₂₁ is independently hydrogen radical or R₂₀;
each R₂₂ is independently hydrogen or C₁-C₄ alkyl radical;
R₁₁ is an aryl or heteroaryl radical other than an "N°-heteroaryl radical, and R₁₂ is a "N°-heteroaryl radical, wherein the aryl, heteroayyl and "N"-heteroaryl radicals are optionally substituted by 1-2 radicals of
(1) R₃₀;
(2) halo or cyano radicals; or
(3) -C(O)-NR₃₁R₃₂, -OR₂₉, -SR₂₉, -S(O)-R₃₀, -S(O)₂-R₃₀,-S(O)₂-NR₃₁R₃₂, -NR₃₁R₃₂ or -NR₃₃-C(O)-R₂₉ radicals;
each R₃₀ is independently
(1) C₁-C₄ alkyl radical optionally substituted by a phenyl or heteroaryl radical optionally substituted by 1-3 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀; and
each R₃₁ is independently
(1) hydrogen radicals; or
(2) C₁-C₄ alkyl radical optionally substituted by an phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyl or-trifluoromethyl radicals;
wherein heterocyclyl is a radical of a monocyclic saturated heterocyclic ring system having 5-6 ring members, wherein 1-3 ring members are oxygen, sulfur or nitrogen heteroatoms, which is optionally benzo-fused and optionally substituted by 1-2 oxo or thioxo radicals; aryl is a phenyl or naphthyl radical; and heteroaryl is radical of a monocyclic aromatic heterocyclic ring system having 5-6 ring members, wherein 1-3 ring members are oxygen, sulfur or nitrogen heteroatoms, which is optionally benzo-fused or saturated C₃-C₄-carbocyclic-fused.
each R₃₂ is independently
(1) hydrogen radicals;
(2) C₁-C₄ alkyl radical or C₁-C₂ alkyl radical substituted by phenyl or heteroaryl radical optionally substituted by 1-3 radicals of amino, dimethylamino, acetamido, hydroxy, methoxy, methyl or trifluoromethyl radicals; or
(3) phenyl or heteroaryl radical optionally substituted by 1-3 radicals of amino, dimethylamino, acetamido, hydroxy, methoxy, methyl or trifluoromethyl radicals; and each R₃₃ is independently hydrogen or C₁-C₄ alkyl radical, provided that when X is C-H and Q is other than a phenyl radical; and when X is N and J is C-H, A is other than a 4-(methylsulfonyl) phenyl, 4-(aminosulfonyl) -phenyl, 4-(trifluoromethylcarbonyl-aminosulfonyl) phenyl or 4-(methylaminosulfonyl) phenyl radical; with the further proviso that the compound is not selected from the group of compounds of general formula (I) 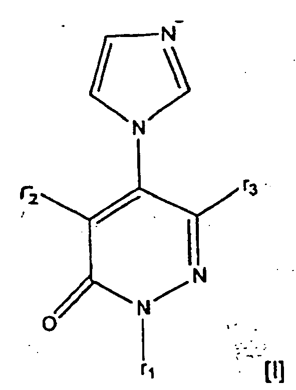
where r₁ is a hydrogen atom, methyl group, ethyl group, propyl group or butyl group, r₂ is a hydrogen atom and r₃ is an optionally-halogen-substituted phenyl group selected from a 4-chlorophenyl group and 2, 4-dichlorophenyl group.

2. The compound of Claim 1 or a pharmaceutically acceptable salt thereof, wherein
W is R₁ or R₂;
J is N, C-R₁ or C-R₂, provided at least one of X or J is N;
a is a double bond and b is a single bond; and
when W is R₁, then J is other than C-R₁; when W is R₂, then J is other than C-R₂;
each R₅ is independently
(1) hydrogen radical;
(2) C₁-C₄ alkyl radical optionally substituted by 1-3 halo radicals; or
(3) phenyl-C₁-C₂-alkyl or heteroaryl-C₁-C₂-alkyl, radicals optionally substituted by 1-3 radicals of amino, dimethylamino, hydroxy, methoxy, methylthio, methyl or trifluoromethyl radicals;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical optionally substituted by 1-2 radicals of hydroxy or C₁-C₄ alkyl ; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an aryl or heteroaryl radical other than an "N"-heteroaryl radical, optionally substituted by 1-2 radicals of (1) R₃₀; (2) halo or cyano radicals; or (3) -C(O)-NR₃₁R₃₂, -OR₂₉, -SR₂₉, -S(O)-R₃₀, -S(O)₂-R₃₀, - S(O)₂-NR₃₁R₃₂, -NR₃₁R₃₂ or -NR₃₃-C(O)-R₂₉ radicals;
R₁₂ is an "N"-heteroaryl radical optionally substituted by 1-2 radicals of (1) R₃₀; (2) halo or cyano radicals; or (3) -C(O)-NR₃₁R₃₂, -OR₂₉, -SR₂₉, -NR₃₁R₃₂ or -NR₃₃-C(O)-R₂₉ radicals;
R₃₀ is independently
(1) C₁-C₄ alkyl radical optionally substituted by a phenyl or heteroaryl radical optionally substituted by 1-2 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀;
each R₃₁ is independently hydrogen or C₁-C₄ alkyl radicals;
R₃₂ is independently
(1) hydrogen or C₁-C₄ alkyl radical; or
(2) phenyl or heteroaryl radical optionally substituted by 1-2 radicals of amino, dimethylamino, acetamido, hydroxy, methoxy, methyl or trifluoromethyl radicals; and
each R₃₃ is independently hydrogen or C₁-C₄ alkyl radical; and
wherein heterocyclyl is a radical of a monocyclic saturated heterocyclic ring system having 5-6 ring members, wherein 1-2 ring members are oxygen, sulfur or nitrogen heteroatoms, which is optionally benzo-fused and optionally substituted by 1-2 oxo or thioxo radicals; aryl is a phenyl or naphthyl radical; and heteroaryl is radical of a monocyclic aromatic heterocyclic ring system having 5-6 ring members, wherein 1-2 ring members are oxygen, sulfur or nitrogen heteroatoms, which is optionally benzo-fused.

3. The compound of Claim 2 or a pharmaceutically acceptable salt thereof, wherein
each Y is independently a -OR₂₁, -SR₂₁ or -NR₅R₂₁ radical;
each R₅ is independently hydrogen or C₁-C₄ alkyl radical;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl or naphthyl radical or a phenyl radical substituted by 1-2 radicals of methyl, amino, dimethylamino, acetamido, hydroxy, halo, cyano, methoxy, methylthio, methylsulfinyl, methylsulfonyl, aminocarbonyl, methyl or trifluoromethyl radicals; and
R₁₂ is a 4-pyridyl, 4-pyrimidyl, 4-quinolinyl, 7-imidazo[4,5-b]pyridinyl, 8-quinazolinyl, 6-(1H)-purinyl, or a 4-imidazolyl radical optionally substituted by a radical of amino, dimethylamino, acetamido, hydroxy, halo, cyano, methoxy, methyl or trifluoromethyl radicals.

4. The compound of Claim 3 or a pharmaceutically acceptable salt thereof, wherein
W is. R₁ ;
A is R₁₂ and Q is R₁₁ ;
X is N and J is C-R₂, or X is C-H and J is N, or X and
J are both N; and
a is a double bond and b is a single bond;
R₂ is a hydrogen, halo, trifluoromethyl, cyano or C₁-C₄ alkyl radical;
each Z is independently a
(1) C₁-C₄ alkyl radical optionally substituted by 1-2 radicals of amino, dimethylamino or phenyl radical; or
(2) a heterocyclyl radical optionally substituted by 1-2 radicals of methyl or phenylmethyl;
wherein the phenyl radicals are optionally substituted by 1-2 radicals of amino, di-(C₁-C₂ alkyl)amino, hydroxy, C₁-C₂ alkoxy, C₁-C₂ alkylthio, cyano, halo, C₁-C₂ alkyl or trifluoromethyl radicals;
each R₅ is a hydrogen or methyl radical;
each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl radical or a phenyl radical substituted by 1-2 radicals of methyl, amino, dimethylamino, acetamido, hydroxy, halo, cyano, methoxy, methylthio, methylsulfonyl, methyl or trifluoromethyl radicals; and
R₁₂ is a 4-pyridyl or 4-pyrimidyl radical optionally substituted by a radical of amino, dimethylamino, acetamido, hydroxy, halo, cyano, methoxy, methyl or trifluoromethyl radicals.

5. The compound of Claim 2 or a pharmaceutically acceptable salt thereof, wherein
W is R₂;
A is R₁₁ and Q is R₁₂;
X is N and J is C-R₁; and
a is a double bond and b is a single bond;
R₂ is a hydrogen, halo, trifluoromethyl, cyano or C₁-C₄ alkyl radical;
each Z is independently a
(1) C₁-C₄ alkyl radical optionally substituted by 1-2 radicals of amino, dimethylamino or phenyl radical; or
(2) a heterocyclyl radical optionally substituted by 1-2 radicals of methyl or phenylmethyl;
wherein the phenyl radicals are optionally substituted by 1-2 radicals of amino, di-(C₁-C₂ alkyl)amino, hydroxy, C₁-C₂ alkoxy, C₁-C₂ alkylthio, cyano, halo, C₁-C₂ alkyl or trifluoromethyl radicals;
each R₅ is a hydrogen or methyl radical;
each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl radical or a phenyl radical substituted by 1-2 radicals of methyl, amino, dimethylamino, acetamido, hydroxy, halo, cyano, methoxy, methylthio, methylsulfonyl, methyl or trifluoromethyl radicals; and
R₁₂ is a 4-pyridyl or 4-pyrimidyl radical optionally substituted by a radical of amino, dimethylamino, acetamido, hydroxy, halo, cyano, methoxy, methyl or trifluoromethyl radicals.

6. The compound of Claim 1 or a pharmaceutically acceptable salt thereof, wherein
W is O ;
A is R₁₁ and Q is R₁₂, or A is R₁₂ and Q is R₁₁;
X is N or C-H;
J is N-R₃; and
a is a single bond and b is a double bond;
each Z is independently a
(1) C₁-C₄ alkyl or C₂-C₅ alkenyl radical optionally substituted by (a) 1-3 radicals of amino, di-(C₁-C₂ alkyl)amino, (C₁-C₄ alkoxy)carbonylamino, hydroxy, C₁-C₂ alkoxy, C₁-C₂ alkylthio or halo, and (b) 1-2 radicals of aryl or heteroaryl; or
(2) heterocyclyl, aryl or heteroaryl radical;
wherein the heterocyclyl radicals are optionally substituted by 1-2 radicals of C₁-C₄ alkyl or aryl-C₁-C₂ alkyl radicals; and the aryl and heteroaryl radicals are optionally substituted by 1-3 radicals of amino, di- (C₁-C₂ alkyl)amino, acetamido, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
each Y is independently a hydrogen, -OR₂₁, -SR₂₁, -S(O)-R₂₀, -S(O)₂-R₂₀ or -NR₅R₂₁ radical;
each R₅ is independently
(1) hydrogen radical;
(2) C₁-C₄ alkyl radical optionally substituted by 1-3 halo radicals; or
(3) phenyl-C₁-C₂-alkyl or heteroaryl-C₁-C₂-alkyl, radicals optionally substituted by 1-3 radicals of amino, dimethylamino, hydroxy, methoxy, methylthio, methyl or trifluoromethyl radicals;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical optionally substituted by 1-2 radicals of hydroxy or C₁-C₄ alkyl; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an aryl or heteroaryl radical other than an "N"-heteroaryl radical, optionally substituted by 1-2 radicals of (1) R₃₀; (2) halo or cyano radicals; or (3) -C(O)-NR₃₁R₃₂, -OR₂₉, -SR₂₉, -S(O)-R₃₀, -S(O)₂-R₃₀, - S(O)₂-NR₃₁P₃₂, -NP₃₁R₃₂ or -NR₃₃-C(O)-R₂₉ radicals;
R₁₂ is an "N"-heteroaryl radical optionally substituted by 1-2 radicals of (1) R₃₀; (2) halo or cyano radicals; or (3)-C(O)-NR₃₁R₃₂, -OR₂₉, -SR₂₉, -NR₃₁R₃₂ or -NR₃₃-C (O) -R₂₉ radicals;
R₃₀ is independently
(1) C₁-C₄ alkyl radical optionally substituted by a phenyl or heteroaryl radical optionally substituted by 1-2 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀;
each R₃₁ is independently hydrogen or C₁-C₄ alkyl radicals;
R₃₂ is independently
(1) hydrogen or C₁-C₄ alkyl radical; or
(2) phenyl or heteroaryl radical optionally substituted by 1-2 radicals of amino, dimethylamino, acetamido, hydroxy, methoxy, methyl or trifluoromethyl radicals; and
each R₃₃ is independently hydrogen or C₁-C₄ alkyl radical; and
wherein heterocyclyl is a radical of a monocyclic saturated heterocyclic ring system having 5-6 ring members, wherein 1-2 ring members are oxygen, sulfur or nitrogen heteroatoms, which is optionally benzo-fused and optionally substituted by 1-2 oxo or thioxo radicals; aryl is a phenyl or naphthyl radical; and heteroaryl is radical of a monocyclic aromatic heterocyclic ring system having 5-6 ring members, wherein 1-2 ring members are oxygen, sulfur or nitrogen heteroatoms, which is optionally benzo-fused.

7. The compound of Claim 6 or a pharmaceutically acceptable salt thereof, wherein
W is O;
A is R₁₁ and Q is R₁₂;
X is N or C-H;
J is N-R₁; and
a is a single bond and b is a double bond;
each Z is independently a
(1) C₁-C₄ alkyl radical optionally substituted by (a) 1-2 radicals of amino, di-(C₁-C₂ alkyl)amino, hydroxy, C₁-C₂ alkoxy or C₁-C₂ alkylthio, and (b) an aryl radical; or
(2) a heterocyclyl radical optionally substituted by 1-2 radicals of C₁-C₂ alkyl or aryl-C₁-C₂ alkyl radicals,
wherein the aryl radicals are optionally substituted by 1-2 radicals of amino, di-(C₁-C₂ alkyl)amino, hydroxy, C₁-C₂ alkoxy, C₁-C₂ alkylthio, cyano, halo, C₁-C₂ alkyl or trifluoromethyl radicals;
each Y is independently a -OR₂₁, -SR₂₁ or -NR₅R₂₁ radical ;
each R₅ is independently hydrogen or C₁-C₄ alkyl radical ;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl or naphthyl radical or a phenyl radical substituted by 1-2 radicals of methyl, amino, dimethylamino, acetamido, hydroxy, halo, cyano, methoxy, methylthio, methylsulfinyl, methylsulfonyl, aminocarbonyl, methyl or trifluoromethyl radicals; and R₁₂ is a 4-pyridyl, 4-pyrimidyl, 4-quinolinyl, 7-imidazo [4,5-b] pyridinyl, 8-quinazolinyl, 6-(1*H*)-purinyl, or a 4-imidazolyl radical optionally substituted by a radical of amino, dimethylamino, acetamido, hydroxy, halo, cyano, methoxy, methyl or trifluoromethyl radicals.

8. The compound of Claim 7 or a pharmaceutically acceptable salt thereof, wherein
W is o;
A is R₁₁ and Q is R₁₂;
X is C-H;
J is N-R₃; and
a is a single bond and b is a double bond;
each Z is independently a
(1) C₁-C₄ alkyl radical optionally substituted by 1-2 radicals of amino, dimethylamino or phenyl radical; or
(2) a heterocyclyl radical optionally substituted by 1-2 radicals of methyl or phenylmethyl;
wherein the phenyl radicals are optionally substituted by 1-2 radicals of amino, di-(C₁-C₂-alkyl) amino, hydroxy, C₁-C₂ alkoxy, C₁-C₂ alkylthio, cyano, halo, C₁-C₂ alkyl or trifluoromethyl radicals;
each R₅ is a hydrogen or methyl radical;
each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl radical or a phenyl radical substituted by 1-2 radicals of methyl, amino, dimethylamino, acetamido, hydroxy, halo, cyano, methoxy, methylthio, methylsulfonyl, methyl or trifluoromethyl radicals; and
R₁₂ is a 4-pyridyl or 4-pyrimidyl radical optionally substituted by a radical of amino, dimethylamino, acetamido, hydroxy, halo, cyano, methoxy, methyl or trifluoromethyl radicals.

9. The compound of Claim 1 which is:
1-(3-phenylpropyl)-4-(3-methylphenyl)-5-(4-pyridyl)-1*H-*pyrid-2-one;
2-(3-phenylpropoxy)-4-(3-methylphenyl)-5-(4-pyridyl)pyridine;
1-((S)-2-amino-3-phenylpropyl)-4-(3-methylphenyl)-5-(4-pyridyl)-1*H*-pyrid=2-one;
2-((S)-2-amino-3-phenylpropoxy)-4-(3-methylphenyl)-5-(4-pyridyl)pyridine;
2-(3-amino-3-phenylpropylamino)-5-(4-fluorophenyl)-4-(4-pyridinyl)pyridine;
2-(3-amino-3-phenylpropylamino)-5-(4-chlorolphenyl)-4-(4-pyridinyl)pyridine;
2-(3-amino-3-phenylpropylamino)-5-(3-fluorophenyl)-4-(4-pyridinyl)pyridine;
2-(3-amino-3-phenylpropylamino)-5-(3-trifluoromethylphenyl)-4-(4-pyridinyl)pyridine;
2-(3-amino-3-phenylpropylamino)-5-(3-isopropylphenyl)-4-(4-pyridinyl)pyridine;
2-(3-amino-3-phenylpropylamino)-5-(3-methylphenyl)-4-(4-pyridinyl)pyridine;
2 - ( (S) -2-amino-3-phenylpropylamino)- 5 - (4-fluorophenyl)-4-(4-pyridinyl)pyridine;
2-((S)-2-amino-3-phenylpropylamino)-5-(4-chlorophenyl)-4-(4-pyridinyl)pyridine;
2- ((S) -2-amino-3-phenylpropylamino)-5- (3-fluorophenyl-4-(4-pyridinyl)pyridine;
2-((S)-2-amino-3-phenylpropylamino)-5-(3-trifluoromethylphenyl-4-(4-pyridinyl)pyridine;
2-((S)-2-amino-3-phenylpropylamino)-5- (3-isopropylphenyl)-4-(4-pyridinyl)pyridine;
6-[((S)-2-amino-3-phenylpropyl)-amino]-3-(4-fluorophenyl)-4-(4-pyridyl)-pyridazine;
6-((S) -2-Amino-3-phenylpropylamino)-3- (2-benzothiophenyl)-2-(4-pyridyl)pyridine;
6- ((S) -2-Amino-3-phenylpropylamino)-3- (4-chloro-3-fluorophenyl)-2-(4-pyridyl)pyridine;
6- ((S) -2-Amino-3-phenylpropylamino)-3- (4-methoxyphenyl)-2- (4-pyridyl) pyridine;
6-((S)-2-Amino-3-phenylpropylamino)-3-(3-isopropylphenyl)-2-(4-pyridyl)pyridine;
6-((S)-2-Amino-3-phenylpropylamino)-3-(4-chlorophenyl)-2-(4-pyridyl)pyridine;
*6-((S)*-2-Amino-3-phenylpropylamino)-3-(2-napthyl)-2-(4-pyridyl)pyridine;
6-((S)-2-Amino-3-phenylpropylamino)-3-(3-trifluoromethylphenyl)-2-(4-pyridyl)pyridine;
6-((S)-2-Amino-3-phenylpropylamino)-3-(3-methylphenyl)-2-(4-pyridyl)pyridine; or
a pharmaceutically acceptable salt thereof.

10. The compound of Claim 1, wherein R₃ is -Z-Y, each Z is a heterocyclyl radical optionally substituted by 1-2 radicals of C₁-C₂ alkyl or aryl-C₁-C₂ alkyl radicals; wherein the aryl radicals are optionally substituted by 1-2 radicals of amino, di-(C₁-C₂ alkyl) amino, hydroxy, C₁-C₂ alkoxy, C₁-C₂ alkylthio, cyano, halo, C₁-C₂ alkyl or trifluoromethyl radicals.

11. The compound of Claim 1, wherein W is R₁ or R₂.

12. The compound of Claim 1, wherein A is R₁ and Q is R₁₂.

13. The compound of Claim 1, wherein A is R₁₂ and Q is R₁₁ wherein R₁₁ is an aryl or heteroaryl radical other than an "N" - heteroaryl radical, and R₁₂ is a "N"-heteroaryl radical, wherein the aryl and heteroaryl radicals are optionally substituted and "N" - heteroaryl is substituted by 1-2 radicals of
(1) R₃₀
(2) halo or cyano radicals; or
(3) -C(O) -NR₃₁R₃₂, -OR₂₉, -SR₂₉, -S(O) -R₃₀, -S(O)₂-R_{30,}-S(O)₂-NR₃₁R₃₂, -NR₃₁R₃₂ or -NR₃₃-C(O) -R₂₉ radicals;

14. The compound of Claim 1, wherein A is R₁₂ and Q is R₁₁ wherein R₁₁ is a heteroaryl radical other than an "N"-heteroaryl radical, and R₁₂ is a "N"-heteroaryl radical, wherein the heteroaryl and "N"-heteroaryl radicals are optionally substituted by 1-2 radicals of
(1) R₃₀
(2) halo or cyano radicals; or
(3) -C(O) -NR₃₁R₃₂, -OR₂₉, -SR₂₉, -S(O) -R₃₀, -S(O)₂-R₃₀,-S(O)₂-NR₃₁R₃₂, -NR₃₁R₃₂ or -NR₃₃-C(O) -R₂₉ radicals; or
R₁₁ is an aryl radical substituted by 1-2 radicals of
(1) R₃₀
(2) cyano radicals; or
(3) -C(O) -NR₃₁R₃₂, -OR₂₉, -SR₂₉, -S(O) -R₃₀, -S(O)₂-R₃₀, S(O)₂-NR₃₁R₃₂, -NR-₃₁R₃₂ or -NR₃₃-C(O) -R₂₉ radicals;

15. The compound of Claim 1, wherein X is C-H.

16. A pharmaceutical composition comprising a compound of Claims 1 to 15 and a pharmaceutically acceptable carrier.

17. The compound of any one of Claims 1 to 15 for use in a method of prophylaxis or treatment of inflammation, the method comprising administering an effective amount of the compound.

18. The composition of Claim 16, for use in a method of prophylaxis or treatment of inflammation.

19. The compound of Claims 1 to 15, for use in a method of prophylaxis or treatment of rheumatoid arthritis, Pagets disease, osteophorosis, multiple myeloma, uveititis, acute or chronic myelogenous leukemia, pancreatic B cell destruction, osteoarthritis, rheumatoid spondylitis, gouty arthritis, inflammatory bowel disease, adult respiratory distress syndrome (ARDS), psoriasis, Crohn's disease, allergic rhinitis, ulcerative colitis, anaphylaxis, contact dermatitis, asthma, muscle degeneration, cachexia, Reiter's syndrome, type I diabetes, type II diabetes, bone resorption diseases, graft vs. host reaction, Alzheimer's disease, stroke, myocardial infarction, ischemia reperfusion injury, atherosclerosis, brain trauma, multiple sclerosis, cerebral malaria, sepsis, septic shock, toxic shock syndrome, fever, myalgias due to HIV-1, HIV-2, HIV-3, cytomegalovirus (CMV), influenza, adenovirus, the herpes viruses or herpes zoster infection in a mammal.

20. The composition of Claim 16, for use in a method of prophylaxis or treatment of rheumatoid arthritis, Pagets disease, osteophorosis, multiple myeloma, uveititis, acute or chronic myelogenous leukemia, pancreatic B cell destruction, osteoarthritis, rheumatoid spondylitis, gouty arthritis, inflammatory bowel disease, adult respiratory distress syndrome (ARDS), psoriasis, Crohn's disease, allergic rhinitis, ulcerative colitis, anaphylaxis, contact dermatitis, asthma, muscle degeneration, cachexia, Reiter's syndrome, type I diabetes, type II diabetes, bone resorption diseases, graft vs. host reaction, Alzheimer's disease, stroke, myocardial infarction, ischemia reperfusion injury, atherosclerosis, brain trauma, multiple sclerosis, cerebral malaria, sepsis, septic shock, toxic shock syndrome, fever, myalgias due to HIV-1, HIV-2, HIV-3, cytomegalovirus (CMV), influenza, adenovirus, the herpes viruses or herpes zoster infection in a mammal.

21. The compound of Claims 1 to 15, for use in a method of lowering plasma concentrations of either or both TNF-a and IL-1.

22. The composition of Claim 16, for use in a method of lowering plasma concentrations of either or both TNF-a and IL-1.

23. The compound of Claims 1 to 15, for use in a method of lowering plasma concentrations of either or both IL-6 and IL-8.

24. The composition of Claim 16, for use in a method of lowering plasma concentrations of either or both IL-6 and IL-8.

25. The compound of Claims 1 to 15, for use in a method of prophylaxis or treatment of diabetes disease in a mammal comprising administering an effective amount of the compound to produce a glucagon antagonist effect.

26. The composition of Claim 16, for use in a method of prophylaxis or treatment of diabetes disease in a mammal comprising administering an effective amount of the compound to produce a glucagon antagonist effect.

27. The compound according to Claims 1 to 15, for use in a method of prophylaxis or treatment of a pain disorder in a mammal.

28. The pharmaceutical composition according to Claim 16, for use in a method of prophylaxis or treatment of a pain disorder in a mammal.

29. The compound according to Claims 1 to 15, for use in a method of decreasing prostaglandins production in a mammal.

30. The pharmaceutical composition according to Claim 16, for use in a method of decreasing prostaglandins production in a mammal.

31. The compound according to Claims 1 to 15, for use in a method of decreasing cyclooxygenase enzyme activity in a mammal.

32. The compound of Claim 31, wherein the cyclooxygenase enzyme is COX-2.

33. The pharmaceutical composition according to Claim 16, for use in a method of decreasing cyclooxygenase enzyme activity in a mammal.

34. The composition of Claim 33, wherein the cyclooxygenase enzyme is COX-2.

35. The compound according to Claims 1 to 15, for use in a method of prophylaxis or treatment of cancer in a mammal.

36. The compound of Claim 35, wherein the cancer is mediated by Raf and Raf-inducable proteins.

37. The compound of Claim 35, wherein the cancer is pancreatic cancer, breast cancer, brain cancer, larynx cancer, lung cancer, lymphatic system cancer, urinary tract cancer or stomach cancer.

38. The pharmaceutical composition according to Claim 16, for use in a method of prophylaxis or treatment of cancer in a mammal.

39. The composition of Claim 38, wherein the cancer is mediated by Raf and Raf-inducable proteins.

40. The composition of Claim 38, wherein the cancer is pancreatic cancer, breast cancer, brain cancer, larynx cancer, lung cancer, lymphatic system cancer, urinary tract cancer or stomach cancer.

41. Use of a compound of Claims 1 to 15, for the preparation of a composition for use in the prophylaxis or treatment of diabetes disease.

42. Use of a compound of Claims 1 to 15, for the preparation of a composition for use in treating rheumatoid arthritis, osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; HIV infections; cytomegalovirus (CMV) infections; influenza; adenovirus infections; the herpesvirus infections; herpes zoster; muscle degeneration; cachexia; Reiter's syndrome; type II diabetes; bone resorption diseases; graft vs. host reaction; ischemia reperfusion injury; atherosclerosis; brain trauma; Alzheimer's disease; multiple sclerosis; cerebral malaria; sepsis; septic shock; toxic shock syndrome; or fever or mylagias due to infection.

43. Use of a compound of Claims 1 to 15, for the preparation of a composition for use in treating pancreatic cancer, breast cancer, brain cancer, larynx cancer, lung cancer, lymphatic system cancer, urinary tract cancer or stomach cancer.

44. Use of a compound of Claims 1 to 15, for the preparation of a composition for use in lowering plasma concentrations of TNF-α or IL-1.

45. Use of a compound of Claims 1 to 15, for the preparation of a composition for use in decreasing prostaglandins production in a mammal.

46. Use of a compound of Claims 1 to 15, for the preparation of a composition for use in decreasing cyclooxygenase enzyme activity in a mammal.

## Patentansprüche

1. Verbindung von Formel 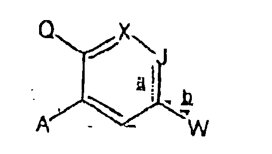 oder ein pharmazeutisch annehmbares Salz derselben, wobei
W R₁, R₂ oder O ist;
A R₁₁ ist und Q R₁₂ ist oder umgekehrt;
X N oder C-H ist;
J N-R₃, N, C-R₁ oder C-R₂ ist, vorausgesetzt, daß wenigstens eines von X oder J N oder N-R₃ ist; und,
wenn W R₁ ist, a eine Doppelbindung ist, b eine Einfachbindung ist und J von N-R₃ oder C-R₁ verschieden ist; wenn W R₂ ist, a eine Doppelbindung ist, b eine Einfachbindung ist und J von N-R₃ oder C-R₂ verschieden ist; und wenn W O ist, a eine Einfachbindung ist, b eine Doppelbindung ist und J N-R₃ ist;
R₁ -Z-Y oder -Y ist; und jedes R₃ unabhängig ein Wasserstoffrest oder -Z-Y ist; vorausgesetzt, daß die Gesamtzahl von Aryl-, Heteroaryl-, Cycloalkyl- und Heterocyclylresten in R₁, R₂ und R₃ 0 bis 2 beträgt;
R₂ (1) ein Wasserstoff-, Halo-, Trifluormethyl- oder Cyanorest; oder (2) ein C₁-C₄-Alkylrest, fakultativ substituiert mit (a) 1 bis 2 Resten von Amino, C₁-C₄-Alkylamino oder Di-(C₁-C₄-alkyl)amino, ist;
jedes Z unabhängig ein
(1) C₁-C₄-Alkylrest, fakultativ substituiert mit (a) 1 bis 2 Resten von Amino, Di-(C₁-C₂-alkyl)amino, Hydroxy, C₁-C₂-Alkoxy oder C₁-C₂-Alkylthio und (b) einem Arylrest; oder
(2) ein Heterocyclylrest, fakultativ substituiert mit 1 bis 2 Resten von C₁-C₂-Alkyl- oder Aryl-C₁-C₂-alkylresten, ist; wobei die Arylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio-, Cyano-, Halo-, C₁-C₂-Alkyl- oder Trifluormethylresten;
jedes Y unabhängig ein
(1) Wasserstoffrest;
(2) -C(O)-R₂₀- oder -C(O)-NR₅R₂₁-Rest,
(3) -OR₂₁-, -SR₂₁-, -S(O)-R₂₀-, -S(O)₂-R₂₀- oder -S(O)₂-NR₅R₂₁-Rest; oder
(4) -NR₅R₂₁- oder -NR₂₂-C(O)-R₂₁-Rest ist;
jedes R₅ unabhängig
(1) Wasserstoffrest;
(2) C₁-C₄-Alkylrest, fakultativ substituiert mit 1 bis 3 Resten von Amino, Di-(C₁-C₂-alkyl)amino, Hydroxy, C₁-C₂-Alkoxy, C₁-C₂-Alkylthio oder Halo; oder
(3) Phenyl-C₁-C₂-alkyl-, Heteroaryl-C₁-C₂-alkyl-, Heterocyclyl-C₁-C₂-alkyl- oder C₃-C₆-Cycloalkyl-C₁-C₂-alkylreste, fakultativ substituiert mit 1 bis 3 Resten von Amino-, Di-(C₁-C₂-alkyl)amino-, Hydroxy-, C₁-C₂-Alkoxy-, C₁-C₂-Alkylthio-, Methoxy-, Methylthio-, C₁-C₄-Alkyl- oder Trifluormethylresten, ist;
jedes R₂₀ unabhängig
(1) C₁-C₈-Alkylreste, fakultativ substituiert mit 1 bis 3 Resten von Amino-, C₁-C₄-Alkylamino-, Di-(C₁-C₄-alkyl)amino-, C₁-C₅-Alkanoylamino-, (C₁-C₄-Alkoxy)carbonylamino-, N-((C₁-C₄-Alkoxy)carbonyl)-N-(C₁-C₄-alkyl)amino-, Aminocarbonylamino-, Hydroxy-, C₁-C₄-Alkoxy-, C₁-C₄-Alkylthio-, C₁-C₄-Alkylsulfinyl-, C₁-C₄-Alkylsulfonyl-, Halo- oder C₃-C₆-Cycloalkyl-, Heterocyclyl-, Aryl- oder Heteroarylresten, fakultativ substituiert mit 1 bis 2 Resten von Amino-, Di-(C₁-C₄-alkyl)amino-, C₁-C₅-Alkanoylamino-, (C₁-C₄-Alkoxy)carbonylamino-, C₁-C₄-Alkylsulfonylamino-, Hydroxy-, C₁-C₄-Alkoxy-, C₁-C₄-Alkylthio-, Halo-, C₁-C₄-Alkyl-oder Trifluormethylresten;
(2) Heterocyclylrest, fakultativ substituiert mit 1 bis 2 Resten von Hydroxy, C₁-C₄-Alkoxy, C₁-C₄-Alkylthio oder C₁-C₄-Alkyl; oder
(3) Aryl- oder Heteroarylreste, fakultativ substituiert mit 1 bis 2 Resten von (C₁-C₄-Alkoxy)carbonyl-, Amino-, C₁-C₄-Alkylamino-, Di-(C₁-C₄-alkyl)amino-, Hydroxy-, C₁-C₄-Alkoxy-, C₁-C₄-Alkylthio-, Cyano-, Halo-, Azido-, C₁-C₄-Alkyl- oder Trifluormethylresten, ist;
jedes R₂₁ unabhängig Wasserstoffrest oder R₂₀ ist;
jedes R₂₂ unabhängig Wasserstoff- oder C₁-C₄-Alkylrest ist;
R₁₁ ein Arylrest oder ein von einem "N"-Heteroarylrest verschiedener Heteroarylrest ist und R₁₂ ein "N"-Heteroarylrest ist, wobei die Aryl-, Heteroaryl- und "N"-Heteroarylreste fakultativ substituiert sind mit 1 bis 2 Resten von
(1) R₃₀;
(2) Halo- oder Cyanoresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂- oder -NR₃₃-C(O)-R₂₉-Resten;
jedes R₃₀ unabhängig
(1) C₁-C₄-Alkylrest, fakultativ substituiert mit einem Phenyl- oder Heteroarylrest, fakultativ substituiert mit 1 bis 3 Resten von Amino-, Dimethylamino-, Acetamido-, Hydroxy-, Halo-, Methoxy-, Methyl- oder Trifluormethylresten;
(2) Trifluormethylrest; oder
(3) Aryl- oder Heteroarylreste, fakultativ substituiert mit 1 bis 3 Resten von Amino-, Dimethylamino-, Acetamido-, Hydroxy-, Halo-, Methoxy-, Methyl- oder Trifluormethylresten, ist;
jedes R₂₉ unabhängig Wasserstoffrest oder R₃₀ ist; und
jedes R₃₁ unabhängig
(1) Wasserstoffreste; oder
(2) C₁-C₄-Alkylrest, fakultativ substituiert mit einem Phenyl- oder Heteroarylrest, fakultativ substituiert mit 1 bis 3 Resten von Amino-, C₁-C₄-Alkylamino-, Di-(C₁-C₄-alkyl)amino-, C₁-C₅-Alkanoylamino-, (C₁-C₄-Alkoxy)carbonylamino-, Hydroxy-, C₁-C₄-Alkoxy-, C₁-C₄-Alkylthio-, Cyano-, C₁-C₄-Alkyl- oder Trifluormethylresten, ist;
wobei Heterocyclyl ein Rest eines monocyclischen gesättigten heterocyclischen Ringsystems mit 5 bis 6 Ringgliedern ist, wobei 1 bis 3 Ringglieder Sauerstoff-, Schwefel- oder Stickstoff-Heteroatome sind, das fakultativ benzo-kondensiert und fakultativ mit 1 bis 2 Oxo- oder Thioxoresten substituiert ist; Aryl ein Phenyl- oder Naphthylrest ist; und Heteroaryl ein Rest eines monocyclischen aromatischen heterocyclischen Ringsystems mit 5 bis 6 Ringgliedern ist, wobei 1 bis 3 Ringglieder Sauerstoff-, Schwefel- oder Stickstoff-Heteroatome sind, das fakultativ-benzo-kondensiert oder mit einem gesättigten C₃-C₄-Carbocyclus kondensiert ist;
jedes R₃₂ unabhängig
(1) Wasserstoffreste;
(2) C₁-C₄-Alkylrest oder C₁-C₂-Alkylrest, substituiert mit Phenyl- oder Heteroarylrest, fakultativ substituiert mit 1 bis 3 Resten von Amino-, Dimethylamino-, Acetamido-, Hydroxy-, Methoxy-, Methyl- oder Trifluormethylresten; oder
(3) Phenyl- oder Heteroarylrest, fakultativ substituiert mit 1 bis 3 Resten von Amino-, Dimethylamino-, Acetamido-, Hydroxy-, Methoxy-, Methyl- oder Trifluormethylresten, ist; und
jedes R₃₃ unabhängig Wasserstoff- oder C₁-C₄-Alkylrest ist;
vorausgesetzt, daß, wenn X C-H ist und Q von einem Phenylrest verschieden ist; und wenn X N ist und J C-H ist, A verschieden ist von einem 4-(Methylsulfonyl)phenyl-, 4-(Aminosulfonyl)phenyl-, 4-(Trifluormethylcarbonylaminosulfonyl)phenyl- oder 4-(Methylaminosulfonyl)phenylrest;
mit der weiteren Maßgabe, daß die Verbindung nicht ausgewählt ist aus der Gruppe von Verbindungen der allgemeinen Formel [I] 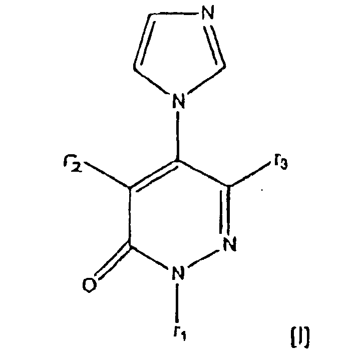 worin r₁ ein Wasserstoffatom, eine Methylgruppe, eine Ethylgrupe, eine Propylgruppe oder eine Butylgruppe ist, r₂ ein Wasserstoffatom ist und r₃ eine fakultativ Halogensubstituierte Phenylgruppe ist, ausgewählt aus einer 4-Chlorphenylgruppe und 2,4-Dichlorphenylgruppe.

2. Verbindung nach Anspruch 1 oder ein pharmazeutisch annehmbares Salz derselben, **dadurch gekennzeichnet, daß**
W R₁ oder R₂ ist;
J N, C-R₁ oder C-R₂ ist, vorausgesetzt, daß wenigstens eines von X oder J N ist;
a eine Doppelbindung ist und b eine Einfachbindung ist; und,
wenn W R₁ ist, J von C-R₁ verschieden ist; wenn W R₂ ist, J von C-R₂ verschieden ist;
jedes Y unabhängig ein Wasserstoff-, -OR₂₁-, -SR₂,-, -S(O)-R₂₀-, -S(O)-R₂₀- oder -NR₅R₂₁-Rest ist;
jedes R₅ unabhängig
(1) Wasserstoffrest;
(2) C₁-C₄-Alkylrest, fakultativ substituiert mit 1 bis 3 Haloresten; oder
(3) Phenyl-C₁-C₂-alkyl- oder Heteroaryl-C₁-C₂-alkylreste, fakultativ substituiert mit 1 bis 3 Resten von Amino-, Dimethylamino-, Hydroxy-, Methoxy-, Methylthio-, Methyl- oder Trifluormethylresten, ist;
jedes R₂₀ unabhängig
(1) C₁-C₆-Alkylreste, fakultativ substituiert mit 1 bis 3 Resten von Amino-, Methylamino-, Dimethylamino-, t-Butoxycarbonylamino-, N-((t-Butoxy)carbonyl)-N-(methyl)amino-, Aminocarbonylamino-, Hydroxy-, Butoxy-, Methoxy-, Butylthio-, Methylthio-, Methylsulfinyl-, Methylsulfonyl-, Halo- oder C₅-C₆-Cycloalkyl-, Heterocyclyl-, Phenyl- oder Heteroarylresten, fakultativ substituiert mit 1 bis 2 Resten von Amino-, Dimethylamino-, Acetamino-, Hydroxy-, Methoxy-, Methylthio-, Halo-, Methyl- oder Trifluormethylresten;
(2) Heterocyclylrest, fakultativ substituiert mit 1 bis 2 Resten von Hydroxy oder C₁-C₄-Alkyl; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein Arylrest oder ein von einem "N"-Heteroarylrest verschiedener Heteroarylrest ist, fakultativ substituiert mit 1 bis 2 Resten von (1) R₃₀; (2) Halo- oder Cyanoresten; oder (3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂- oder -NR₃₃-C(O)-R₂₉-Resten;
R₁₂ ein "N"-Heteroarylrest ist, fakultativ substituiert mit 1 bis 2 Resten von (1) R₃₀; (2) Halo- oder Cyanoresten; oder (3) -C(O)-NR₃₁R₃₂-, -OR₂₉-, -SR₂₉-, -NR₃₁R₃₂- oder -NR₃₃-C(O)-R₂₉-Resten;
R₃₀ unabhängig
(1) C₁-C₄-Alkylrest, fakultativ substituiert mit einem Phenyl- oder Heteroarylrest, fakultativ substituiert mit 1 bis 2 Resten von Amino-, Dimethylamino-, Acetamido-, Hydroxy-, Halo-, Methoxy-, Methyl- oder Trifluormethylresten;
(2) Trifluormethylrest; oder
(3) Aryl- oder Heteroarylreste, fakultativ substituiert mit 1 bis 3 Resten von Amino-, Dimethylamino-, Acetamido-, Hydroxy-, Halo-, Methoxy-, Methyl- oder Trifluormethylresten, ist;
jedes R₂₉ unabhängig Wasserstoffrest oder R₃₀ ist;
jedes R₃₁ unabhängig Wasserstoff- oder C₁-C₄-Alkylreste ist;
R₃₂ unabhängig
(1) Wasserstoff- oder C₁-C₄-Alkylrest; oder
(2) Phenyl- oder Heteroarylrest, fakultativ substituiert mit 1 bis 2 Resten von Amino-, Dimethylamino-, Acetamido-, Hydroxy-, Methoxy-, Methyl- oder Trifluormethylresten, ist; und
jedes R₃₃ unabhängig Wasserstoff- oder C₁-C₄-Alkylrest ist; und
wobei Heterocyclyl ein Rest eines monocyclischen gesättigten heterocyclischen Ringsystems mit 5 bis 6 Ringgliedern ist, wobei 1 bis 2 Ringglieder Sauerstoff-, Schwefel- oder Stickstoff-Heteroatome sind, das fakultativ benzo-kondensiert und fakultativ mit 1 bis 2 Oxo- oder Thioxoresten substituiert ist; Aryl ein Phenyl- oder Naphthylrest ist; und Heteroaryl ein Rest eines monocyclischen aromatischen heterocyclischen Ringsystems mit 5 bis 6 Ringgliedern ist, wobei 1 bis 2 Ringglieder Sauerstoff-, Schwefel- oder Stickstoff-Heteroatome sind, das fakultativ benzo-kondensiert ist.

3. Verbindung nach Anspruch 2 oder ein pharmazeutisch annehmbares Salz derselben, **dadurch gekennzeichnet, daß**
jedes Y unabhängig ein -OR₂₁-, -SR₂₁- oder -NR₅R₂₁-Rest ist;
jedes R₅ unabhängig Wasserstoff- oder C₁-C₄-Alkylrest ist;
jedes R₂₀ unabhängig
(1) C₁-C₆-Alkylreste, fakultativ substituiert mit 1 bis 3 Resten von Amino-, Methylamino-, Dimethylamino-, t-Butoxycarbonylamino-, N-((t-Butoxy)carbonyl)-N-(methyl)amino-, Aminocarbonylamino-, Hydroxy-, Butoxy-, Methoxy-, Butylthio-, Methylthio-, Methylsulfinyl-, Methylsulfonyl-, Halo- oder C₅-C₆-Cycloalkyl-, Heterocyclyl-, Phenyl- oder Heteroarylresten, fakultativ substituiert mit 1 bis 2 Resten von Amino-, Dimethylamino-, Acetamino-, Hydroxy-, Methoxy-, Methylthio-, Halo-, Methyl- oder Trifluormethylresten;
(2) Heterocyclylrest; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein unsubstituierter Phenyl- oder Naphthylrest ist oder ein Phenylrest, der substituiert ist mit 1 bis 2 Resten von Methyl-, Amino-, Dimethylamino-, Acetamido-, Hydroxy-, Halo-, Cyano-, Methoxy-, Methylthio-, Methylsulfinyl-, Methylsulfonyl-, Aminocarbonyl-, Methyl- oder Trifluormethylresten; und
R₁₂ ein 4-Pyridyl-, 4-Pyrimidyl-, 4-Chinolinyl-, 7-Imidazo[4,5-b]pyridinyl-, 8-Chinazolinyl-, 6-(1H)-Purinyl- oder ein 4-Imidazolylrest ist, fakultativ substituiert mit einem Rest von Amino-, Dimethylamino-, Acetamido-, Hydroxy-, Halo-, Cyano-, Methoxy-, Methyl- oder Trifluormethylresten.

4. Verbindung nach Anspruch 3 oder ein pharmazeutisch annehmbares Salz derselben, **dadurch gekennzeichnet, daß**
W R₁, ist;
A R₁₂ ist und Q R₁₁ ist;
X N ist und J C-R₂ ist oder X C-H ist und J N ist oder X und J beide N sind; und
a eine Doppelbindung ist und b eine Einfachbindung ist;
R₂ ein Wasserstoff-, Halo-, Trifluormethyl-, Cyano- oder C₁-C₄-Alkylrest ist;
jedes Z unabhängig ein
(1) C₁-C₄-Alkylrest, fakultativ substituiert mit 1 bis 2 Resten von Amino-, Dimethylamino- oder Phenylrest; oder
(2) ein Heterocyclylrest, fakultativ substituiert mit 1 bis 2 Resten von Methyl oder Phenylmethyl, ist; wobei die Phenylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio-, Cyano-, Halo-, C₁-C₂-Alkyl- oder Trifluormethylresten;
jedes R₅ ein Wasserstoff- oder Methylrest ist;
jedes R₂₀ unabhängig
(1) C₁-C₆-Alkylreste, fakultativ substituiert mit 1 bis 3 Resten von Amino-, Methylamino-, Dimethylamino-, Hydroxy- oder Phenyl- oder Heteroarylresten, fakultativ substituiert mit 1 bis 2 Resten von Amino-, Dimethylamino-, Hydroxy-, Methoxy-, Methylthio-, Halo-, Methyl- oder Trifluormethylresten;
(2) Heterocyclylrest; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein unsubstituierter Phenylrest ist oder ein Phenylrest, der substituiert ist mit 1 bis 2 Resten von Methyl-, Amino-, Dimethylamino-, Acetamido-, Hydroxy-, Halo-, Cyano-, Methoxy-, Methylthio-, Methylsulfonyl-, Methyl- oder Trifluormethylresten; und
R₁₂ ein 4-Pyridyl- oder 4-Pyrimidylrest ist, fakultativ substituiert mit einem Rest von Amino-, Dimethylamino-, Acetamido-, Hydroxy-, Halo-, Cyano-, Methoxy-, Methyl- oder Trifluormethylresten.

5. Verbindung nach Anspruch 2 oder ein pharmazeutisch annehmbares Salz derselben, **dadurch gekennzeichnet, daß**
W R₂ ist;
A R₁₁ ist und Q R₁₂ ist;
X N ist und J C-R₁ ist; und
a eine Doppelbindung ist und b eine Einfachbindung ist;
R₂ ein Wasserstoff-, Halo-, Trifluormethyl-, Cyano- oder C₁-C₄-Alkylrest ist;
jedes Z unabhängig ein
(1) C₁-C₄-Alkylrest, fakultativ substituiert mit 1 bis 2 Resten von Amino-, Dimethylamino- oder Phenylrest; oder
(2) ein Heterocyclylrest, fakultativ substituiert mit 1 bis 2 Resten von Methyl oder Phenylmethyl, ist; wobei die Phenylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio-, Cyano-, Halo-, C₁-C₂-Alkyl- oder Trifluormethylresten;
jedes R₅ ein Wasserstoff- oder Methylrest ist;
jedes R₂₀ unabhängig
(1) C₁-C₆-Alkylreste, fakultativ substituiert mit 1 bis 3 Resten von Amino-, Methylamino-, Dimethylamino-, Hydroxy- oder Phenyl- oder Heteroarylresten, fakultativ substituiert mit 1 bis 2 Resten von Amino-, Dimethylamino-, Hydroxy-, Methoxy-, Methylthio-, Halo-, Methyl- oder Trifluormethylresten;
(2) Heterocyclylrest; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein unsubstituierter Phenylrest ist oder ein Phenylrest, der substituiert ist mit 1 bis 2 Resten von Methyl-, Amino-, Dimethylamino-, Acetamido-, Hydroxy-, Halo-, Cyano-, Methoxy-, Methylthio-, Methylsulfonyl-, Methyl- oder Trifluormethylresten; und
R₁₂ ein 4-Pyridyl- oder 4-Pyrimidylrest ist, fakultativ substituiert mit einem Rest von Amino-, Dimethylamino-, Acetamido-, Hydroxy-, Halo-, Cyano-, Methoxy-, Methyl- oder Trifluormethylresten.

6. Verbindung nach Anspruch 1 oder ein pharmazeutisch annehmbares Salz derselben, **dadurch gekennzeichnet, daß**
W O ist;
A R₁₁ ist und Q R₁₂ ist oder A R₁₂ ist und Q R₁₁ ist;
X N oder C-H ist;
J N-R₃; und
a eine Einfachbindung ist und b eine Doppelbindung ist;
jedes Z unabhängig ein
(1) C₁-C₄-Alkyl- oder C₂-C₅-Alkenylrest, fakultativ substituiert mit (a) 1 bis 3 Resten von Amino, Di-(C₁-C₂-alkyl)amino, (C₁-C₄-Alkoxy)carbonylamino, Hydroxy, C₁-C₂-Alkoxy, C₁-C₂-Alkylthio oder Halo und (b) 1 bis 2 Resten von Aryl oder Heteroaryl; oder
(2) Heterocyclyl-, Aryl- oder Heteroarylrest, ist; wobei die Heterocyclylreste fakultativ substituiert sind mit 1 bis 2 Resten von C₁-C₄-Alkyl- oder Aryl-C₁-C₂-alkylresten; und die Aryl- und Heteroarylreste fakultativ substituiert sind mit 1 bis 3 Resten von Amino-, Di-(C₁-C₂-alkyl)amino-, Acetamido-, (C₁-C₄-Alkoxy)-carbonylamino-, Hydroxy-, C₁-C₂-Alkoxy-, C₁-C₂-Alkylthio-, Cyano-, Halo-, C₁-C₄-Alkyl- oder Trifluormethylresten;
jedes Y unabhängig ein Wasserstoff-, -OR₂₁-, -SR₂₁-, -S(O)-R₂₀-, -S(O)₂-R₂₀- oder -NR₅R₂₁-Rest ist;
jedes R₅ unabhängig
(1) Wasserstoffrest;
(2) C₁-C₄-Alkylrest, fakultativ substituiert mit 1 bis 3 Haloresten; oder
(3) Phenyl-C₁-C₂-alkyl- oder Heteroaryl-C₁-C₂-alkylreste, fakultativ substituiert mit 1 bis 3 Resten von Amino-, Dimethylamino-, Hxdroxy-, Methoxy-, Methylthio-, Methyl- oder Trifluormethylresten, ist;
jedes R₂₀ unabhängig
(1) C₁-C₆-Alkylreste, fakultativ substituiert mit 1 bis 3 Resten von Amino-, Methylamino-, Dimethylamino-, t-Butoxycarbonylamino-, N-((t-Butoxy)carbonyl)-N-(methyl)amino-, Aminocarbonylamino-, Hydroxy-, Butoxy-, Methoxy-, Butylthio-, Methylthio-, Methylsulfinyl-, Methylsulfonyl-, Halo- oder C₅-C₆-Cycloalkyl-, Heterocyclyl-, Phenyl- oder Heteroarylresten, fakultativ substitutiert mit 1 bis 2 Resten von Amino-, Dimethylamino-, Acetamino-, Hydroxy-, Methoxy-, Methylthio-, Halo-, Methyl- oder Trifluormethylresten;
(2) Heterocyclylrest, fakultativ substituiert mit 1 bis 2 Resten von Hydroxy oder C₁-C₄-Alkyl; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein Arylrest oder ein von einem "N"-Heteroarylrest veschiedener Heteroarylrest ist, fakultativ substituiert mit 1 bis 2 Resten von (1) R₃₀; (2) Halo- oder Cyanoresten; oder (3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂- oder -NR₃₃-C(O)-R₂₉-Resten;
R₁₂ ein "N"-Heteroarylrest ist, fakultativ substituiert mit 1 bis 2 Resten von (1) R₃₀; (2) Halo- oder Cyanoresten; oder (3) -C(O)-NR₃₁R₃₂-, -OR₂₉-, -SR₂₉-, -NR₃₁R₃₂- oder -NR₃₃-C(O)-R₂₉-Resten;
R₃₀ unabhängig
(1) C₁-C₄-Alkylrest, fakultativ substituiert mit einem Phenyl- oder Heteroarylrest, fakultativ substituiert mit 1 bis 2 Resten von Amino-, Dimethylamino-, Acetamido-, Hydroxy-, Halo-, Methoxy-, Methyl- oder Trifluormethylresten,
(2) Trifluormethylrest; oder
(3) Aryl- oder Heteroarylreste, fakultativ substituiert mit 1 bis 3 Resten von Amino-, Dimethylamino-, Acetamido-, Hydroxy-, Halo-, Methoxy-, Methyl- oder Trifluormethylresten, ist;
jedes R₂₉ unabhängig Wasserstoffrest oder R₃₀ ist;
jedes R₃₁ unabhängig Wasserstoff- oder C₁-C₄-Alkylrest ist;
R₃₂ unabhängig
(1) Wasserstoff- oder C₁-C₄-Alkylrest; oder
(2) Phenyl- oder Heteroarylrest, fakultativ substituiert mit 1 bis 2 Resten von Amino-, Dimethylamino-, Acetamido-, Hydroxy-, Methoxy-, Methyl- oder Trifluormethylresten, ist; und
jedes R₃₃ unabhängig Wasserstoff- oder C₁-C₄-Alkylrest ist; und
wobei Heterocyclyl ein Rest eines monocyclischen gesättigten heterocyclischen Ringsystems mit 5 bis 6 Ringgliedern ist, wobei 1 bis 2 Ringglieder Sauerstoff-, Schwefel- oder Stickstoff-Heteroatome sind, das fakultativ benzo-kondensiert und fakultativ mit 1 bis 2 Oxo- oder Thioxoresten substituiert ist; Aryl ein Phenyl- oder Naphthylrest ist; und Heteroaryl ein Rest eines monocyclischen aromatischen heterocyclischen Ringsystems mit 5 bis 6 Ringgliedern ist, wobei 1 bis 2 Ringglieder Sauerstoff-, Schwefel- oder Stickstoff-Heteroatome sind, das fakultativ benzo-kondensiert ist.

7. Verbindung nach Anspruch 6 oder ein pharmazeutisch annehmbares Salz derselben, **dadurch gekennzeichnet, daß**
W O ist;
A R₁₁ ist und Q R₁₂ ist;
X N oder C-H ist;
J N-R₃ ist; und
a eine Einfachbindung ist und b eine Doppelbindung ist;
jedes Z unabhängig ein .
(1) C₁-C₄-Alkylrest, fakultativ substituiert mit (a) 1 bis 2 Resten von Amino, Di-(C₁-C₂-alkyl)amino, Hydroxy, C₁-C₂-Alkoxy oder C₁-C₂-Alkylthio und (b) einem Arylrest; oder
(2) ein Heterocyclylrest, fakultativ substituiert mit 1 bis 2 Resten von C₁-C₂-Alkyl- oder Aryl-C₁-C₂-alkylresten, ist; wobei die Arylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio-, Cyano-, Halo-, C₁-C₂-Alkyl- oder Trifluormethylresten,
jedes Y unabhängig ein -OR₂₁-, -SR₂₁- oder -NR₅R₂₁-Rest ist;
jedes R₅ unabhängig Wasserstoff- oder C₁-C₄-Alkylrest ist;
jedes R₂₀ unabhängig
(1) C₁-C₆-Alkylreste, fakultativ substituiert mit 1 bis 3 Resten von Amino-, Methylamino-, Dimethylamino-, t-Butoxycarbonylamino-, N-((t-Butoxy)carbonyl)-N-(methyl)-amino-, Aminocarbonylamino-, Hydroxy-, Butoxy-, Methoxy-, Butylthio-, Methylthio-, Methylsulfinyl-, Methylsulfonyl-, Halo- oder C₅-C₆-Cycloalkyl-, Heterocyclyl-, Phenyl- oder Heteroarylresten, fakultativ substitutiert mit 1 bis 2 Resten von Amino-, Dimethylamino-, Acetamino-, Hydroxy-, Methoxy-, Methylthio-, Halo-, Methyl- oder Trifluormethylresten;
(2) Heterocyclylrest; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein unsubstituierter Phenyl- oder Naphthylrest ist oder ein Phenylrest, der substituiert ist mit 1 bis 2 Resten von Methyl-, Amino-, Dimethylamino-, Acetamido-, Hydroxy-, Halo-, Cyano-, Methoxy-, Methylthio-, Methylsulfinyl-, Methylsulfonyl-, Aminocarbonyl-, Methyl- oder Trifluormethylresten; und
R₁₂ ein 4-Pyridyl-, 4-Pyrimidyl-, 4-Chinolinyl-, 7-Imidazo[4,5-b]pyridinyl-, 8-Chinazolinyl-, 6-(1H)-Purinyl- oder ein 4-Imidazolylrest ist, fakultativ substituiert mit einem Rest von Amino-, Dimethylamino-, Acetamido-, Hydroxy-, Halo-, Cyano-, Methoxy-, Methyl- oder Trifluormethylresten.

8. Verbindung nach Anspruch 7 oder ein pharmazeutisch annehmbares Salz derselben, **dadurch gekennzeichnet, daß**
W O ist;
A R₁₁ ist und Q R₁₂ ist;
X C-H ist;
J N-R₃ ist; und
a eine Einfachbindung ist und b eine Doppelbindung ist;
jedes Z unabhängig ein
(1) C₁-C₄-Alkylrest, fakultativ substituiert mit 1 bis 2 Resten von Amino-, Dimethylamino- oder Phenylrest; oder
(2) ein Heterocyclylrest, fakultativ substituiert mit 1 bis 2 Resten von Methyl oder Phenylmethyl, ist;
wobei die Phenylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio-, Cyano-, Halo-, C₁-C₂-Alkyl- oder Trifluormethylresten,
jedes R₅ ein Wasserstoff- oder Methylrest ist;
jedes R₂₀ unabhängig
(1) C₁-C₆-Alkylreste, fakultativ substituiert mit 1 bis 3 Resten von Amino-, Methylamino-, Dimethylamino-, Hydroxy- oder Phenyl- oder Heteroarylresten, fakultativ substituiert mit 1 bis 2 Resten von Amino-, Dimethylamino-, Hydroxy-, Methoxy-, Methylthio-, Halo-, Methyl- oder Trifluormethylresten;
(2) Heterocyclylrest; oder
(3) Aryl- oder Heteroarylreste, fakultativ substituiert mit 1 bis 2 Resten von Amino-, Dimethylamino-, Hydroxy-, Methoxy-, Methylthio-, Halo-, Methyl- oder Trifluormethylresten, ist;
jedes R₂₁ unabhängig Wasserstoffrest oder R₂₀ ist;
R₁₁ ein unsubstituierter Phenylrest ist oder ein Phenylrest, der substituiert ist mit 1 bis 2 Resten von Methyl-, Amino-, Dimethylamino-, Acetamido-, Hydroxy-, Halo-, Cyano-, Methoxy-, Methylthio-, Methylsulfonyl-, Methyl- oder Trifluormethylresten; und
R₁₂ ein 4-Pyridyl- oder 4-Pyrimidylrest ist, fakultativ substituiert mit einem Rest von Amino-, Dimethylamino-, Acetamido-, Hydroxy-, Halo-, Cyano-, Methoxy-, Methyl- oder Trifluormethylresten.

9. Verbindung nach Anspruch 1, **dadurch gekennzeichnet, daß** sie
1-(3-Phenylpropyl)-4-(3-methylphenyl)-5-(4-pyridyl)-1H-pyrid-2-on;
2-(3-Phenylpropoxy)-4-(3-methylphenyl)-5-(4-pyridyl)pyridin;
1-((S)-2-Amino-3-phenylpropyl)-4-(3-methylphenyl)-5-(4-pyridyl)-1H-pyrid-2-on;
2-((S)-2-Amino-3-phenylpropoxy)-4-(3-methylphenyl)-5-(4-pyridyl)pyridin;
2-(3-Amino-3-phenylpropylamino)-5-(4-fluorphenyl)-4-(4-pyridinyl)pyridin;
2-(3-Amino-3-phenylpropylamino)-5-(4-chlorphenyl)-4-(4-pyridinyl)pyridin;
2-(3-Amino-3-phenylpropylamino)-5-(3-fluorphenyl)-4-(4-pyridinyl)pyridin;
2-(3-Amino-3-phenylpropylamino)-5-(3-trifluormethylphenyl)-4-(4-pyridinyl)pyridin;
2-(3-Amino-3-phenylpropylamino)-5-(3-isopropylphenyl)-4-(4-pyridinyl)pyridin;
2-(3-Amino-3-phenylpropylamino)-5-(3-methylphenyl)-4-(4-pyridinyl)pyridin;
2-((S)-2-Amino-3-phenylpropylamino)-5-(4-fluorphenyl)-4-(4-pyridinyl)pyridin;
2-((S)-2-Amino-3-phenylpropylamino)-5-(4-chlorphenyl)-4-(4-pyridinyl)pyridin;
2-((S)-2-Amino-3-phenylpropylamino)-5-(3-fluorphenyl)-4-(4-pyridinyl)pyridin;
2-((S)-2-Amino-3-phenylpropylamino)-5-(3-trifluormethylphenyl-4-(4-pyridinyl)pyridin;
2-((S)-2-Amino-3-phenylpropylamino)-5-(3-isopropylphenyl)-4-(4-pyridinyl)pyridin;
6-[((S)-2-Amino-3-phenylpropyl)-amino]-3-(4-fluorphenyl)-4-(4-pyridyl)-pyridazin;
6-((S)-2-Amino-3-phenylpropylamino)-3-(2-benzothiophenyl)-2-(4-pyridyl)pyridin;
6-((S)-2-Amino-3-phenylpropylamino)-3-(4-chlor-3-fluorphenyl)-2-(4-pyridyl)pyridin;
6-((S)-2-Amino-3-phenylpropylamino)-3-(4-methoxyphenyl)-2-(4-pyridyl)pyridin;
6-((S)-2-Amino-3-phenylpropylamino)-3-(3-isopropylphenyl)-2-(4-pyridyl)pyridin;
6-((S)-2-Amino-3-phenylpropylamino)-3-(4-chlorphenyl)-2-(4-pyridyl)pyridin;
6-((S)-2-Amino-3-phenylpropylamino)-3-(2-naphthyl)-2-(4-pyridyl)pyridin;
6-((S)-2-Amino-3-phenylpropylamino)-3-(3-trifluormethylphenyl)-2-(4-pyridyl)pyridin;
6-((S)-2-Amino-3-phenylpropylamino)-3-(3-methylphenyl)-2-(4-pyridyl)pyridin ist; oder ein pharmazeutisch annehmbares Salz derselben.

10. Verbindung nach Anspruch 1, **dadurch gekennzeichnet, daß** R₃ -Z-Y ist, jedes Z ein Heterocyclylrest ist, fakultativ substituiert mit 1 bis 2 Resten von C₁-C₂-Alkyl- oder Aryl-C₁-C₂-alkylresten; wobei die Arylreste fakultativ substituiert sind mit 1 bis 2 Resten von Amino-, Di-(C₁-C₂-alkyl)amino-, Hydroxy-, C₁-C₂-Alkoxy-, C₁-C₂-Alkylthio, Cyano-, Halo-, C₁-C₂-Alkyl- oder Trifluormethylresten.

11. Verbindung nach Anspruch 1, **dadurch gekennzeichnet, daß** W R₁ oder R₂ ist.

12. Verbindung nach Anspruch 1, **dadurch gekennzeichnet, daß** A R₁₁ ist und Q R₁₂ ist.

13. Verbindung nach Anspruch 1, **dadurch gekennzeichnet, daß** A R₁₂ ist und Q R₁₁ ist, wobei R₁₁ ein Arylrest oder ein von einem "N"-Heteroarylrest verschiedener Heteroarylrest ist, und R₁₂ ein "N"-Heteroarylrest ist, wobei die Aryl- und Heteroarylreste fakultativ substituiert sind und "N"-Heteroaryl substituiert ist mit 1 bis 2 Resten von
(1) R₃₀;
(2) Halo- oder Cyanoresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂,-, -NR₃₁R₃₂- oder -NR₃₃-C(O)-R₂₉-Resten.

14. Verbindung nach Anspruch 1, **dadurch gekennzeichnet, daß** A R₁₂ ist und Q R₁₁ ist, wobei R₁₁ ein von einem "N"-Heteroarylrest verschiedener Heteroarylrest ist und R₁₂ ein "N"-Heteroarylrest ist, wobei die Heteroaryl- und "N"-Heteroarylreste fakultativ substituiert sind mit 1 bis 2 Resten von
(1)R₃₀;
(2) Halo- oder Cyanoresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂- oder -NR₃₃-C(O)-R₂₉-Resten; oder
R₁₁ ein Arylrest ist, der substituiert ist mit 1 bis 2 Resten von
(1) R₃₀;
(2) Cyanoresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂- oder -NR₃₃-C(O)-R₂₉-Resten.

15. Verbindung nach Anspruch 1, **dadurch gekennzeichnet, daß** X C-H ist.

16. Pharmazeutische Zusammensetzung, die eine Verbindung nach den Ansprüchen 1 bis 15 und einen pharmazeutisch annehmbaren Trägerstoff umfaßt.

17. Verbindung nach einem der Ansprüche 1 bis 15 zur Verwendung in einem Verfahren zur Prophylaxe oder Behandlung von Entzündungen, wobei das Verfahren die Verabreichung einer wirksamen Menge der Verbindung umfaßt.

18. Zusammensetzung nach Anspruch 16 zur Verwendung in einem Verfahren zur Prophylaxe oder Behandlung von Entzündungen.

19. Verbindung nach den Ansprüchen 1 bis 15 zur Verwendung in einem Verfahren zur Prophylaxe oder Behandlung von rheumatoider Arthritis, Paget-Krankheit, Osteoporose, multiplem Myelom, Uveititis, akuter oder chronischer myelogener Leukämie, Bauchspeicheldrüsen-β-Zellzerstörung, Osteoarthritis, rheumatoider Spondylitis, Gichtarthritis, entzündlicher Darmerkrankung, posttraumatischer Lungeninsuffizienz (ARDS), Psoriasis, Crohn-Krankheit, allergischer Rhinitis, Colitis ulcerosa, Anaphylaxie, Kontaktdermatitis, Asthma, Muskeldegeneration, Kachexie, Reiter-Syndrom, Typ-I-Diabetes, Typ-II-Diabetes, Knochenresorptionserkrankungen, Reaktion eines Wirtes gegen ein Transplantat, Alzheimer-Krankheit, Schlaganfall, Myokardinfarkt, Ischämie-Reperfusions-Verletzung, Atherosklerose, Hirntrauma, multipler Sklerose, cerebraler Malaria, Sepsis, septischem Schock, toxischem Schocksyndrom, Fieber, Myalgien aufgrund von HIV-1, HIV-2, HIV-3, Cytomegalovirus (CMV), Grippe, Adenovirus, den Herpesviren oder Herpes-Zoster-Infektion in einem Säuger.

20. Zusammensetzung nach Anspruch 16 zur Verwendung in einem Verfahren zur Prophylaxe oder Behandlung von rheumatoider Arthritis, Paget-Krankheit, Osteoporose, multiplem Myelom, Uveititis, akuter oder chronischer myelogener Leukämie, Bauchspeicheldrüsen-β-Zellzerstörung, Osteoarthritis, rheumatoider Spondylitis, Gichtarthritis, entzündlicher Darmerkrankung, posttraumatischer Lungeninsuffizienz (ARDS), Psoriasis, Crohn-Krankheit, allergischer Rhinitis, Colitis ulcerosa, Anaphylaxie, Kontaktdermatitis, Asthma, Muskeldegeneration, Kachexie, Reiter-Syndrom, Typ-I-Diabetes, Typ-II-Diabetes, Knochenresorptionserkrankungen, Reaktion eines Wirtes gegen ein Transplantat, Alzheimer-Krankheit, Schlaganfall, Myokardinfarkt, Ischämie-Reperfusions-Verletzung, Atherosklerose, Hirntrauma, multipler Sklerose, cerebraler Malaria, Sepsis, septischem Schock, toxischem Schocksyndrom, Fieber, Myalgien aufgrund von HIV-1, HIV-2, HIV-3, Cytomegalovirus (CMV), Grippe, Adenovirus, den Herpesviren oder Herpes-Zoster-Infektion in einem Säuger.

21. Verbindung nach den Ansprüchen 1 bis 15 zur Verwendung in einem Verfahren zur Senkung von Plasmakonzentrationen von TNF-a und/oder IL-1.

22. Zusammensetzung nach Anspruch 16 zur Verwendung in einem Verfahren zur Senkung von Plasmakonzentrationen von TNF-a und/oder IL-1.

23. Verbindung nach den Ansprüchen 1 bis 15 zur Verwendung in einem Verfahren zur Senkung von Plasmakonzentrationen von IL-6 und/oder IL-8.

24. Zusammensetzung nach Anspruch 16 zur Verwendung in einem Verfahren zur Senkung von Plasmakonzentrationen von IL-6 und/oder IL-8.

25. Verbindung nach den Ansprüchen 1 bis 15 zur Verwendung in einem Verfahren zur Prophylaxe oder Behandlung von Diabetes-Erkrankung in einem Säuger, welches die Verabreichung einer wirksamen Menge der Verbindung umfaßt, um einen Glucagon-Antagonisten-Effekt zu erzeugen.

26. Zusammensetzung nach Anspruch 16 zur Verwendung in einem Verfahren zur Prophylaxe oder Behandlung von Diabetes-Erkrankung in einem Säuger, welches die Verabreichung einer wirksamen Menge der pharmazeutischen Zusammensetzung umfaßt, um einen Glucagon-Antagonisten-Effekt zu erzeugen.

27. Verbindung nach den Ansprüchen 1 bis 15 zur Verwendung in einem Verfahren zur Prophylaxe oder Behandlung einer Schmerzerkrankung in einem Säuger.

28. Pharmazeutische Zusammensetzung nach Anspruch 16 zur Verwendung in einem Verfahren zur Prophylaxe oder Behandlung einer Schmerzerkrankung in einem Säuger.

29. Verbindung nach den Ansprüchen 1 bis 15 zur Verwendung in einem Verfahrem zur Verminderung der Produktion von Prostaglandinen in einem Säuger.

30. Pharmazeutische Zusammensetzung nach Anspruch 16 zur Verwendung in einem Verfahren zur Verminderung der Produktion von Prostaglandinen in einem Säuger.

31. Verbindung nach den Ansprüchen 1 bis 15 zur Verwendung in einem Verfahren zur Verminderung der Cyclooxygenase-Enzymaktivität in einem Säuger.

32. Verbindung nach Anspruch 31, **dadurch gekennzeichnet, daß** das Cyclooxygenase-Enzym COX-2 ist.

33. Pharmazeutische Zusammensetzung nach Anspruch 16 zur Verwendung in einem Verfahren zur Verminderung der Cyclooxygenase-Enzymaktivität in einem Säuger.

34. Zusammensetzung nach Anspruch 33, **dadurch gekennzeichnet, daß** das Cyclooxygenase-Enzym COX-2 ist.

35. Verbindung nach den Ansprüchen 1 bis 15 zur Verwendung in einem Verfahren zur Prophylaxe oder Behandlung von Krebs in einem Säuger.

36. Verbindung nach Anspruch 35, **dadurch gekennzeichnet, daß** der Krebs durch Raf und Raf-induzierbare Proteine vermittelt ist.

37. Verbindung nach Anspruch 35, **dadurch gekennzeichnet, daß** der Krebs Bauchspeicheldrüsenkrebs, Brustkrebs, Hirnkrebs, Kehlkopfkrebs, Lungenkrebs, Krebs des lymphatischen Systems, Harntraktkrebs oder Magenkrebs ist.

38. Pharmazeutische Zusammensetzung nach Anspruch 16 zur Verwendung in einem Verfahren zur Prophylaxe oder Behandlung von Krebs in einem Säuger.

39. Zusammensetzung nach Anspruch 38, **dadurch gekennzeichnet, daß** der Krebs durch Raf und Raf-induzierbare Proteine vermittelt ist.

40. Zusammensetzung nach Anspruch 38, **dadurch gekennzeichnet, daß** der Krebs Bauchspeicheldrüsenkrebs, Brustkrebs, Hirnkrebs, Kehlkopfkrebs, Lungenkrebs, Krebs des lymphatischen Systems, Harntraktkrebs oder Magenkrebs ist.

41. Verwendung einer Verbindung nach den Ansprüchen 1 bis 15 zur Herstellung einer Zusammensetzung zur Verwendung in der Prophylaxe oder Behandlung von Diabetes-Erkrankung.

42. Verwendung einer Verbindung nach den Ansprüchen 1 bis 15 zur Herstellung einer Zusammensetzung zur Verwendung bei der Behandlung von rheumatoider Arthritis; Osteoarthritis; rheumatoider Spondylitis; Gichtarthritis; entzündlicher Darmerkrankung; posttraumatischer Lungeninsuffizienz (ARDS); Psoriasis; Crohn-Krankheit; allergischer Rhinitis; Colitis ulcerosa; Anaphylaxie; Kontaktdermatitis; Asthma; HIV-Infektionen; Cytomegalovirus(CMV)-Infektionen; Grippe; Adenovirus-Infektionen; den Herpesviren-Infektionen; Herpes zoster; Muskeldegeneration; Kachexie; Reiter-Syndrom; Typ-II- Diabetes; Knochenresorptionserkrankungen; Reaktion eines Wirtes gegen ein Transplantat; Ischämie-Reperfusions-Verletzung; Atherosklerose; Hirntrauma; Alzheimer-Krankheit; Multipler Sklerose; cerebraler Malaria; Sepsis; septischem Schock; toxischem Schocksyndrom; oder Fieber oder Myalgien aufgrund von Infektion.

43. Verwendung einer Verbindung nach den Ansprüchen 1 bis 15 zur Herstellung einer Zusammensetzung zur Verwendung bei der Behandlung von Bauchspeicheldrüsenkrebs, Brustkrebs, Hirnkrebs, Kehlkopfkrebs, Lungenkrebs, Krebs des lymphatischen Systems, Harntraktkrebs oder Magenkrebs.

44. Verwendung einer Verbindung nach den Ansprüchen 1 bis 15 zur Herstellung einer Zusammensetzung zur Verwendung bei der Senkung von Plasmakonzentrationen von TNF-α oder IL-1.

45. Verwendung einer Verbindung nach den Ansprüchen 1 bis 15 zur Herstellung einer Zusammensetzung zur Verwendung bei der Verminderung der Produktion von Prostaglandinen in einem Säuger.

46. Verwendung einer Verbindung nach den Ansprüchen 1 bis 15 zur Herstellung einer Zusammensetzung zur Verwendung bei der Verminderung der Cyclooxygenase-Enzymaktivität in einem Säuger.

## Revendications

1. Composé de formule 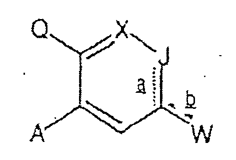 ou un sel pharmaceutiquement acceptable de celui-ci, dans lequel
W est R₁, R₂, ou O ;
A est R₁₁, et Q est R₁₂, ou vice versa ;
X est N ou C-H ;
J est N-R₃, N, C-R₁ ou C-R₂, pourvu qu'au moins un de X ou J soit N ou N-R₃ ; et
quand W est R₁, alors a est une double liaison, b est une liaison simple et J est autre que N-R₃ ou C-R₁ ; quand W est R₂, alors a est une double liaison, b est une liaison simple et J est autre que N-R₃ ou C-R₂ ; et quand W est O, alors a est une liaison simple, b est une double liaison et J est N-R₃;
R₁ est -Z-Y ou -Y ; et chaque R₃ est indépendamment un radical hydrogène ou -Z-Y ; pourvu que le nombre total de radicaux aryle, hétéroaryle, cycloalkyle et hétérocyclyle dans R₁, R₂ et R₃ soit O-2 ;
R₂ est (1) un radical hydrogène, halo, trifluorométhyle ou cyano ; ou
(2) un radical C₁-C₄ alkyle éventuellement substitué par (a) 1-2 radicaux amino, C₁-C₄ alkylamino ou di-(C₁-C₄ alkyl)amino ;
chaque Z est indépendamment
(1) un radical C₁-C₄ alkyle éventuellement substitué par (a) 1-2 radicaux amino, di-(C₁-C₂ alkyl)amino, hydroxy, C₁-C₂ alkoxy ou C₁-C₂ alkylthio, et (b) un radical aryle ; ou
(2) un radical hétérocyclyle éventuellement substitué par 1-2 radicaux de radicaux C₁-C₂ alkyle ou aryl-C₁-C₂ alkyle, où les radicaux aryle sont éventuellement substitués par 1-2 radicaux de radicaux amino, di-(C₁-C₂ alkyl)amino, hydroxy, C₁-C₂ alkoxy, C₁-C₂ alkylthio, cyano, halo, C₁-C₂ alkyle
ou trifluorométhyle ;
chaque Y est indépendamment
(1) un radical hydrogène ;
(2) un radical -C(O)-R₂₀ ou -C(O)-NR₅R₂₁ ;
(3) un radical -OR₂₁, -SR₂₁, -S(O)-R₂₀, -S(O)₂-R₂₀ ou -S(O)₂-NR₅R₂₁ ; ou
(4) un radical -NR₅R₂₁ ou -NR₂₂-C(O)-R₂₁ ;
chaque R₅ est indépendamment
(1) un radical hydrogène ;
(2) un radical C₁-C₄ alkyle éventuellement substitué par 1-3 radicaux amino, di-(C₁-C₂-alkyl)amino, hydroxy, C₁-C₂ alkoxy, C₁-C₂ alkylthio ou halo ;
ou
(3) des radicaux phényl-C₁-C₂-alkyle, hétéroaryl-C₁-C₂-alkyle, hétérocyclyl-C₁-C₂-alkyle ou C₃-C₆-cycloalkyl-C₁-C₂-alkyle éventuellement substitués par 1-3 radicaux de radicaux amino-, di-(C₁-C₂-alkyl)amino, hydroxy, C₁-C₂ alkoxy, C₁-C₂ alkylthio, méthoxy; méthylthio, C₁-C₄ alkyle ou trifluorométhyle ;
chaque R₂₀ est indépendamment
(1) des radicaux C₁-C₈ alkyle éventuellement substitués par 1-3 radicaux amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkyl-sulfinyle, C₁-C₄ alkylsulfonyle, halo ou des radicaux C₃-C₆ cycloalkyle, hétérocyclyle, aryle ou hétéroaryle éventuellement substitués par 1-2 radicaux de radicaux amino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyle ou trifluorométhyle ;
(2) un radical hétérocyclyle éventuellement substitué par 1-2 radicaux d'hydroxy, C₁-C₄ alkoxy, C₁-C₄ alkylthio ou C₁-C₄ alkyle ; ou
(3) des radicaux aryle ou hétéroaryle éventuellement substitués par 1-2 radicaux de radicaux (C₁-C₄-alkoxy)carbonyle, amino, C₁-C₄ alkylamino, di-(C₁-C₄ alkyl)amino, hydroxy, C₁-C₄ alkoxy, C₁-C₄ alkylthio, cyano, halo, azido, C₁-C₄ alkyle ou trifluorométhyle ;
chaque R₂₁ est indépendamment un radical hydrogène ou R₂₀ ;
chaque R₂₂ est indépendamment un radical hydrogène ou C₁-C₄ alkyle ;
R₁₁ est un radical aryle ou hétéroaryle autre qu'un radical "N"-hétéroaryle, et R₁₂ est un radical "N"-hétéroaryle, où les radicaux aryle, hétéroaryle et "N"-hétéroaryle sont éventuellement substitués par 1-2 radicaux de
(1) R₃₀ ;
(2) radicaux halo ou cyano ; ou
(3) radicaux -C(O)-NR₃₁R₃₂, -OR₂₉, -SR₂₉, -S(O)-R₃₀, -S(O)₂-R₃₀, -S(O)₂-NR₃₁ R₃₂, -NR₃₁R₃₂, ou -NR₃₃-C(O)-R₂₉ ;
chaque R₃₀ est indépendamment
(1) un radical C₁-C₄ alkyle éventuellement substitué par un radical phényle ou hétéroaryle éventuellement substitué par 1-3 radicaux de radicaux amino, diméthylamino, acétamido, hydroxy, halo, méthoxy, méthyle ou trifluorométhyle ;
(2) un radical trifluorométhyle ; ou
(3) des radicaux aryle ou hétéroaryle éventuellement substitués par 1-3 radicaux de radicaux amino, diméthylamino, acétamido, hydroxy, halo, méthoxy, méthyle ou trifluorométhyle ;
chaque R₂₉ est indépendamment un radical hydrogène ou R₃₀ ; et
chaque R₃₁ est indépendamment
(1) des radicaux hydrogène ; ou
(2) un radical C₁-C₄ alkyle éventuellement substitué par un radical phényle ou hétéroaryle éventuellement substitué par 1-3 radicaux de radicaux amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyle ou trifluorométhyle ;
dans lequel l'hétérocyclyle est un radical d'un noyau hétérocyclique saturé monocyclique ayant 5-6 chaînons de cycle, où 1-3 chaînons de cycle sont des hétéroatomes d'oxygène, de soufre ou d'azote, qui est éventuellement benzo-condensé et éventuellement substitué par 1-2 radicaux oxo ou thioxo ; aryle est un radical phényle ou naphtyle ; et hétéroaryle est un radical d'un noyau hétérocyclique aromatique monocyclique ayant 5-6 chaînons de cycle, où 1-3 chaînons de cycle sont des hétéroatomes d'oxygène, de soufre ou d'azote, qui est éventuellement benzo-condensé ou saturé C₃-C₄-carbocyclique-condensé ;
chaque R₃₂ est indépendamment
(1) des radicaux hydrogène ;
(2) un radical C₁-C₄ alkyle ou un radical C₁-C₂ alkyle substitué par un radical phényle ou hétéroaryle éventuellement substitué par 1-3 radicaux de radicaux amino, diméthylamino, acétamido, hydroxy, méthoxy, méthyle ou trifluorométhyle ; ou
(3) un radical phényle ou hétéroaryle éventuellement substitué par 1-3 radicaux de radicaux amino, diméthylamino, acétamido, hydroxy, méthoxy, méthyle ou trifluorométhyle ; et
chaque R₃₃ est indépendamment un radical hydrogène ou C₁-C₄ alkyle,
pourvu que, quand X est C-H et Q est autre qu'un radical phényle ; et quand X est N et J est C-H, A soit autre qu'un radical 4-(méthylsulfonyl)phényle, 4-(aminosulfonyl)phényle, 4-(trifluorométhylcarbonylaminosulfonyl)phényle ou 4-(méthylaminosulfonyl)phényle à la condition suivante que le composé ne soit pas choisi parmi le groupe de composés de la formule générale [1] 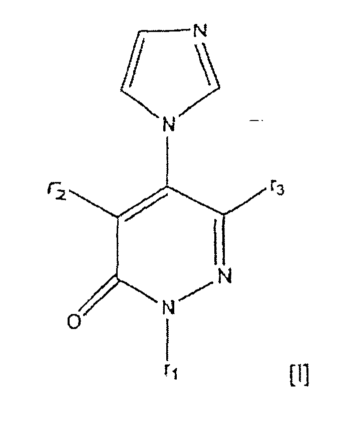 où n est un atome d'hydrogène, un groupe méthyle, un groupe éthyle, un groupe propyle ou un groupe butyle, r₂ est un atome d'hydrogène et r₃ est un groupe phényle substitué par éventuellement un halogène, choisi à partir d'un groupe 4-chlorophényle et un groupe 2,4-dichlorophényle.

2. Composé de la revendication 1 ou un sel pharmaceutiquement acceptable de celui-ci, dans lequel
W est R₁ ou R₂ ;
J est N, C-R₁ ou C-R₂, pourvu qu'au moins un de X ou J soit N ;
a est une double liaison et b est une liaison simple ; et quand W est R₁, alors J est autre que C-R₁ ; quand W est R₂, alors J est autre que C-R₂ ;
chaque Y est indépendamment un radical hydrogène, -OR₂₁, -SR₂₁, -S(O)-R_{20'} =S(O)₂-H₂₀ ou -NR₅R₂₁ ;
chaque R₅ est indépendamment
(1) un radical hydrogène ;
(2) un radical C₁-C₄ alkyle éventuellement substitué par 1-3 halo radicaux ;
ou
(3) des radicaux phényl-C₁-C₂-alkyle ou hétéroaryl-C₁-C₂-alkyle, éventuellement substitués par 1-3 radicaux de radicaux amino, diméthylamino, hydroxy, méthoxy, méthylthio, méthyle ou trifluorométhyle ;
chaque R₂₀ est indépendamment
(1) des radicaux C₁-C₆ alkyle éventuellement substitués par 1-3 radicaux amino, méthylamino, diméthylamino, t-butoxycarbonylamino, N-((t-butoxy)carbonyl)-N-(méthyl)amino, aminocarbonylamino, hydroxy, butoxy, méthoxy, butylthio, méthylthio, méthylsulfinyle, méthylsulfonyle, halo ou des radicaux C₅-C₆ cycloalkyle, hétérocyclyle, phényle ou hétéroaryle éventuellement substitués par 1-2 radicaux de radicaux amino, diméthylamino, acétamino, hydroxy, méthoxy, méthylthio, halo, méthyle ou trifluorométhyle ;
(2) un radical hétérocyclyle éventuellement substitué par 1-2 radicaux hydroxy ou C₁-C₄ alkyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀ ;
R₁₁ est un radical aryle ou hétéroaryle autre qu'un radical "N"-hétéroaryle, éventuellement substitué par 1-2 radicaux de (1) R₃₀ ; (2) radicaux halo ou cyano ; ou (3) radicaux -C(O)-NR₃₁R₃₂, -OR₂₉, -SR₂₉, -S(O)-R₃₀, -S(O)₂-R₃₀, - S(O)₂-NR₃₁R₃₂, -NR₃₁R₃₂ ou-NR₃₃-C(O)-R₂₉;
R₁₂ est un radical "N"-hétéroaryle éventuellement substitué par 1-2 radicaux de (1) R₃₀ ; (2) des radicaux halo ou cyano ; ou (3) des radicaux -C(O)-NR₃₁R₃₂, -OR₂₉, -SR₂₉, -NR₃₁R₃₂ ou NR₃₃-C(O)-R₂₉;
R₃₀ est indépendamment
(1) un radical C₁-C₄ alkyle éventuellement substitué par un radical phényle ou hétéroaryle éventuellement substitué par 1-2 radicaux de radicaux amino, diméthylamino, acétamido, hydroxy, halo, méthoxy, méthyle ou trifluorométhyle ;
(2) un radical trifluorométhyle ; ou
(3) des radicaux aryle ou hétéroaryle éventuellement substitués par 1-3 radicaux de radicaux amino, diméthylamino, acétamido, hydroxy, halo, méthoxy, méthyle ou trifluorométhyle ;
chaque R₂₉ est indépendamment un radical hydrogène ou R₃₀ ;
chaque R₃₁ est indépendamment des radicaux hydrogène ou C₁-C₄ alkyle ;
R₃₂ est indépendamment
(1) un radical hydrogène ou C₁-C₄ alkyle ; ou
(2) un radical phényle ou hétéroaryle éventuellement substitué par 1-2 radicaux de radicaux amino, diméthylamino, acétamido, hydroxy, méthoxy, méthyle ou trifluorométhyle ; et
chaque R₃₃ est indépendamment un radical hydrogène ou C₁-C₄ alkyle ; et
dans lequel l'hétérocyclyle est un radical d'un noyau hétérocyclique saturé monocyclique ayant 5-6 chaînons de cycle, où 1-2 chaînons de cycle sont des hétéroatomes d'oxygène, de soufre ou d'azote, qui est éventuellement benzo-condensé et éventuellement substitué par 1-2 radicaux oxo ou thioxo ; aryle est un radical phényle ou naphtyle ; et hétéroaryle est un radical d'un noyau hétérocyclique aromatique monocyclique ayant 5-6 chaînons de cycle, où 1-2 chaînons de cycle sont des hétéroatomes d'oxygène, de soufre ou d'azote, qui est éventuellement benzo-condensé.

3. Composé de la revendication 2 ou un sel pharmaceutiquement acceptable de celui-ci, dans lequel
chaque Y est indépendamment un radical -OR₂₁, -SR₂₁ ou -NR₅R₂₁ ;
chaque R₅ est indépendamment un radical hydrogène ou C₁-C₄ alkyle ;
chaque R₂₀ est indépendamment
(1) des radicaux C₁-C₆ alkyle éventuellement substitués par 1-3 radicaux amino, méthylamino, diméthylamino, t-butoxycarbonylamino, N-((t-butoxy)carbonyl)-N-(méthyl)amino, aminocarbonylamino, hydroxy, butoxy, méthoxy, butylthio, méthylthio, méthylsulfinyle, méthylsulfonyle, halo ou des radicaux C₅-C₆ cycloalkyle, hétérocyclyle, phényle ou hétéroaryle éventuellement substitués par 1-2 radicaux de radicaux amino, diméthylamino, acétamino, hydroxy, méthoxy, méthylthio, halo, méthyle ou trifluorométhyle ;
(2) un radical hétérocyclyle ; ou
(3) des radicaux aryle ou héléroaryle éventuellement substitués par 1-2 radicaux de radicaux amino, diméthylamino, hydroxy, méthoxy, méthylthio, halo, méthyle ou trifluorométhyle ;
chaque R₂₁, est indépendamment un radical hydrogène ou R₂₀ ;
R₁₁ est un radical phényle ou naphtyle non substitué ou un radical phényle substitué par 1-2 radicaux de radicaux méthyle, amino, diméthylamino, acétamido, hydroxy, halo, cyano, méthoxy, méthylthio, méthylsulfinyle, méthylsulfonyle, aminocarbonyle, méthyle ou trifluorométhyle ; et
R₁₂ est un radical 4-pyridyle, 4-pyrimidyle, 4-quinolinyle, 7-îmid-azo[4.5-b]pyridinyle, 8-quinazolinyle, 6-(1H)-purinyle, ou 4-imidazolyle éventuellement substitué par un radical de radicaux amino, diméthylamino, acétamido, hydroxy, halo, cyano, méthoxy, méthyle ou trifluorométhyle.

4. Composé de la revendication 3 ou un sel pharmaceutiquement acceptable de celui-ci, dans lequel
W est R₁ ;
A est R₁₂ et Q est R₁₁ ;
X est N et J est C-R₂, ou X est C-H et J est N, ou X et J sont les deux N ; et
a est une double liaison et b est une liaison simple;
R₂ est un radical hydrogène, halo, trifluorométhyle, cyano ou C₁-C₄ alkyle;
chaque Z est indépendamment
(1) un radical C₁-C₄ alkyle éventuellement substitué par 1-2 radicaux de radical amino, diméthylamino ou phényle ; ou
(2) un radical hétérocyclyle éventuellement substitué par 1-2 radicaux de méthyle eu phénylméthyle ;
dans lequel les radicaux phényle sont éventuellement substitués par 1-2 radicaux de radicaux amino, di-(C₁-C₂ alkyl)amino, hydroxy, C₁-C₂ alkoxy, C₁-C₂ alkylthio, cyano, halo, C₁-C₂ alkyle ou trifluorométhyle ;
chaque R₅ est un radical hydrogène ou méthyle ;
chaque R₂₀ est indépendamment
(1) des radicaux C₁-C₆ alkyle éventuellement substitués par 1-3 radicaux de radicaux amino, méthylamino, diméthylamino, hydroxy ou phényle ou hétéroaryle éventuellement substitués par 1-2 radicaux de radicaux amino, diméthylamino, hydroxy, méthoxy, méthylthio, halo, méthyle ou trifluorométhyle ;
(2) un radical hétérocyclyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀ ;
R₁₁ est un radical phényle non substitué ou un radical phényle substitué par 1-2 radicaux de radicaux méthyle, amino, diméthylamino, acétamido, hydroxy, halo, cyano, méthoxy, méthylthio, méthylsulfonyle, méthyle ou trifluorométhyle ; et
R₁₂ est un radical 4-pyridyle ou 4-pyrimidyle, éventuellement substitué par un radical de radicaux amino, diméthylamino, acétamido, hydroxy, halo, cyano, méthoxy, méthyle ou trifluorométhyle.

5. Composé de la revendication 2 ou un sel pharmaceutiquement acceptable de celui-ci, dans lequel
W est R₂ ;
A est R₁₁ et Q est R₁₂ ;
X est N et J est C-R₁ ; et
a est une double liaison et b est une liaison simple ;
R₂ est un radical hydrogène, halo, trifluorométhyle, cyano ou C₁-C₄ alkyle ;
chaque Z est indépendamment
(1) un radical C₁-C₄ alkyle éventuellement substitué par 1-2 radicaux de radical amino, diméthylamino ou phényle ; ou
(2) un radical hétérocyclyle éventuellement substitué par 1-2 radicaux de méthyle ou phénylméthyle ;
où les radicaux phényle sont éventuellement substitués par 1-2 radicaux de radicaux amino, di-(C₁-C₂ alkyl)amino, hydroxy, C₁-C₂ alkoxy, C₁-C₂ alkylthio, cyano, halo, C₁-C₂ alkyle ou trifluorométhyle ;
chaque R₅ est un radical hydrogène ou méthyle ;
chaque R₂₀ est indépendamment
(1) des radicaux C₁-C₆ alkyle éventuellement substitués par 1-3 radicaux de radicaux amino, méthylamino, diméthylamino, hydroxy ou phényle ou hétéroaryle éventuellement substitués par 1-2 radicaux de radicaux amino, diméthylamino, hydroxy, méthoxy, méthylthio, halo, méthyle ou trifluorométhyle ;
(2) un radical hétérocyclyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀ ;
R₁₁ est un radical phényle non substitué ou un radical phényle substitué par 1-2 radicaux de radicaux méthyle, amino, diméthylamino, acétamido, hydroxy, halo, cyano, méthoxy, méthylthio, méthylsulfonyle, méthyle ou trifluorométhyle ; et
R₁₂ est un radical 4-pyridyle ou 4-pyrimidyle, éventuellement substitué par un radical de radicaux amino, diméthylamino, acétamido, hydroxy, halo, cyano, méthoxy, méthyle ou trifluorométhyle.

6. Composé de la revendication 1 ou un sel pharmaceutiquement acceptable de celui-ci dans lequel
W est O ;
A est R₁₁ et Q est R₁₂, ou A est R₁₂ et Q est R₁₁ ;
X est N ou C-H ;
J est N-R₃ ; et
a est une liaison simple et b est une double liaison ;
chaque Z est indépendamment
(1) un radical C₁-C₄ alkyle ou C₂-C₅ alkényle éventuellement substitué par (a) 1-3 radicaux amino, di-(C₁-C₂ alkyl)amino, (C₁-C₄ alkoxy)carbonylamino, hydroxy, C₁-C₂ alkoxy, C₁-C₂ alkylthio ou halo, et (b) 1-2 radicaux aryle ou hétéroaryle ; ou
(2) un radical hétérocyclyle, aryle ou hétéroaryle ; dans lequel les radicaux hétérocyclyle sont éventuellement substitués par 1-2 radicaux de radicaux C₁-C₄ alkyle ou aryl-C₁-C₂ alkyle ; et les radicaux aryle et hétéroaryle sont éventuellement substitués par 1-3 radicaux de radicaux amino, di-(C₁-C₂ alkyl)amino, acétamido (C₁-C₄ alkoxy)carbonylamino, hydroxy, C₁-C₂ alkoxy, C₁-C₂ alkylthio, cyano, halo, C₁-C₄ alkyle ou trifluorométhyle ;
chaque Y est indépendamment un radical hydrogène, -OR₂₁, -SR₂₁ -S(O) - R₂₀, -S(O)₂-R₂₀ ou -NR₅R₂₁ ;
chaque R₅ est indépendamment
(1) un radical hydrogène ;
(2) un radical C₁-C₄ alkyle éventuellement substitué par 1-3 radicaux halo ; ou
(3) des radicaux phényl-C₁-C₂-alkyle ou hétéroaryl-C₁-C₂-alkyle, éventuellement substitués par 1-3 radicaux de radicaux amino, diméthylamino, hydroxy, méthoxy, méthylthio, méthyle ou trifluorométhyle ;
chaque R₂₀ est indépendamment
(1) des radicaux C₁-C₆ alkyle éventuellement substitués par 1-3 radicaux amino, méthylamino, diméthylamino, t-butoxycarbonylamino, N-((t-butoxy)carbonyl)-N-(méthyl)amino, aminocarbonylamino, hydroxy, butoxy, méthoxy, butylthio, méthylthio, méthylsulfinyle, méthylsulfonyle, halo, ou des radicaux C₅-C₆ cycloalkyle, hétérocyclyle, phényle ou hétéroaryle éventuellement substitués par 1-2 radicaux de radicaux amino, diméthylamino, acétamino, hydroxy, méthoxy, méthylthio, halo, méthyle ou trifluorométhyle ;
(2) un radical hétérocyclyle éventuellement substitué par 1-2 radicaux d'hydroxy ou C₁-C₄ alkyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀ ;
R₁₁ est un radical aryle ou hétéroaryle autre qu'un radical "-N"-hétéroaryle, éventuellement substitué par 1-2 radicaux de (1) R₃₀ ; (2) radicaux halo ou cyano ; ou (3) radicaux -C(O)-NR₃₁R₃₂, -OR₂₉, -SR₂₉,-S(O)-R₃₀, -S(O)₂-R₃₀, -S(O)₂-R₃₀, -S(O)₂-NR₃₁R₃₂, -NR₃₁R₃₂ ou -NR₃₃-C(O)-R₂₉;
R₁₂ est un radical "N"-hétéroaryle éventuellement substitué par 1-2 radicaux de (1) R₃₀; (2) radicaux halo ou cyano ; ou (3) radicaux -C(O)-NR₃₁R₃₂, -OR₂₉, -SR₂₉, , -NR₃₁R₃₂ ou -NR₃₃-C(O)-R₂₉ ;
R₃₀ est indépendamment
(1) un radical C₁-C₄ alkyle éventuellement substitué par un radical phényle ou hétéroaryle éventuellement substitué par 1-2 radicaux de radicaux amino, diméthylamino, acétamido, hydroxy, halo, méthoxy, méthyle ou trifluorométhyle ;
(2) un radical trifluorométhyle ; ou
(3) des radicaux aryle ou hétéroaryle éventuellement substitués par 1-3 radicaux de radicaux amino, diméthylamino, acétamido, hydroxy, halo, méthoxy, méthyle ou trifluorométhyle.
chaque R₂₉ est indépendamment un radical hydrogène ou R₃₀ ;
chaque R₃, est indépendamment des radicaux hydrogène ou C₁-C₄ alkyle;
R₃₂ est indépendamment
(1) un radical hydrogène ou C₁-C₄ alkyle ;
(2) un radical phényle ou hétéroaryle éventuellement substitué par 1-2 radicaux de radicaux amino, diméthylamino, acétamido, hydroxy, méthoxy, méthyle ou trifluorométhyle ; et
chaque R₃₃ est indépendamment un radical hydrogène ou C₁-C₄ alkyle ; et
dans lequel l'hétérocyclyle est un radical d'un noyau hétérocyclique saturé monocyclique ayant 5-6 chaînons de cycle, où 1-2 chaînons de cycle sont des hétéroatomes d'oxygène, de soufre ou d'azote, qui est éventuellement benzo-condensé et éventuellement substitué par 1-2 radicaux oxo ou thioxo ; aryle est un radical phényle ou naphtyle ; et hétéroaryle est un radical d'un noyau hétérocyclique aromatique monocyclique ayant 5-6 chaînons de cycle, où 1-2 chaînons de cycles sont des hétéroatomes d'oxygène, de soufre ou d'azote, qui est éventuellement benzo-condensé.

7. Composé de la revendication 6 ou un sel pharmaceutiquement acceptable de celui-ci, dans lequel
W est O ;
A est R₁₁ et Q est R₁₂ ;
X est N ou C-H ;
J est N-R₃ ; et
a est une liaison simple et b est une double liaison ;
chaque Z est indépendamment
(1) un radical C₁-C₄ alkyle éventuellement substitué par (a) 1-2 radicaux amino, di-(C₁-C₂ alkyl)amino, hydroxy, C₁-C₂ alkoxy ou C₁-C₂ alkylthio, et (b) un radical aryle ; ou
(2) un radical hétérocyclyle, éventuellement substitué par 1-2 radicaux de radicaux C₁-C₂ alkyle ou aryl-C₁-C₂ alkyle ; où les radicaux aryle sont éventuellement substitués par 1-2 radicaux de radicaux amino, di-(C₁-C₂ alkyl)amino, hydroxy, C₁-C₂ alkoxy, C₁-C₂ alkylthio, cyano, halo, C₁-C₂ alkyle ou trifluorométhyle ;
chaque Y est indépendamment un radical -OR₂₁, -SR₂₁, -NR₅R₂₁;
chaque R₅ est indépendamment un radical hydrogène ou C₁-C₄ alkyle ;
chaque R₂₀ est indépendamment
(1) des radicaux C₁-C₆ alkyle éventuellement substitués par 1-3 radicaux amino, méthylamino, diméthylamino, t-butoxycarbonylamino, N-((t-butoxy)carbonyl)-N-(méthyl)amino, aminocarbonylamino, hydroxy, butoxy, méthoxy, butylthio; méthylthio, méthylsulfinyle, méthylsulfonyle, halo, ou des radicaux C₅-C₆ cycloalkyle, hétérocyclyle, phényle ou hétéroaryle éventuellement substitués par 1-2 radicaux de radicaux amino, diméthylamino, acétamino, hydroxy, méthoxy, méthylthio, halo, méthyle ou trifluorométhyle ;
(2) un radical hétérocyclyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀;
R₁₁ est un radical phényle ou naphtyle non substitué ou un radical phényle substitué par 1-2 radicaux de radicaux méthyle, amino, diméthylamino, acétamino, hydroxy, halo, cyano, méthoxy, méthylthio, méthylsulfinyle, méthylsulfonyle, aminocarbonyle, méthyle ou trifluorométhyle ; et
R₁₂ est un radical 4-pyridyle, 4-pyrimidyle, 4-quinolinyle, 7-imidazo[4,5-b]pyridinyle, 8-quinazolinyle, 6-(1H)-purinyle, ou 4-imidazolyle éventuellement substitué par un radical de radicaux amino, diméthylamino, acétamido, hydroxy, halo, cyano, méthoxy, méthyle ou trifluorométhyle.

8. Composé de la revendication 7 ou un sel pharmaceutiquement acceptable de celui-ci, dans lequel
W est O ;
A est R₁₁ et Q est R₁₂ ;
X est C-H ;
J est N-R₃ ; et
a est une liaison simple et b est une double liaison ;
chaque Z est indépendamment
(1) un radical C₁-C₄ alkyle éventuellement substitué par (a) 1-2 radicaux de radical amino, diméthylamino ou phényle ; ou
(2) un radical hétérocyclyle, éventuellement substitué par 1-2 radicaux de méthyle ou phénylméthyle;
où les radicaux phényle sont éventuellement substitués par 1-2 radicaux des radicaux amino, di-(C₁-C₂ alkyl)amino, hydroxy, C₁-C₂ alkoxy, C₁-C₂ alkylthio, cyano, halo, C₁-C₂ alkyle ou trifluorométhyle ;
chaque R₅ est un radical hydrogène ou méthyle ;
chaque R₂₀ est indépendamment
(1) des radicaux C₁-C₆ alkyle éventuellement substitués par 1-3 radicaux amino, méthylamino, diméthylamino, hydroxy, ou des radicaux phényle ou hétéroaryle éventuellement substitués par 1-2 radicaux de radicaux amino, diméthylamino, hydroxy, méthoxy, méthylthio, halo, méthyle ou trifluorométhyle ;
(2) un radical hétérocyclyle ; ou
(3) des radicaux aryle ou hétéroaryle éventuellement substitués par 1-2 radicaux de radicaux amino, diméthylamino, hydroxy, méthoxy, méthylthio, halo, méthyle ou trifluorométhyle ;
chaque R₂₁ est indépendamment un radical hydrogène ou R₂₀ ;
R₁₁ est un radical phényle non substitué ou un radical phényle substitué par 1-2 radicaux de radicaux méthyle, amino, diméthylamino, acétamino, hydroxy, halo, cyano, méthoxy, méthylthio, méthylsulfonyle, méthyle ou trifluorométhyle ; et
R₁₂ est un radical 4-pyridyle ou 4-pyrimidyle, éventuellement substitué par un radical de radicaux amino, diméthylamino, acétamido, hydroxy, halo, cyano, méthoxy, méthyle ou trifluorométhyle.

9. Composé de la revendication 1 qui est :
1-(3-phénylpropyl)-4-(3-méthylphényl)-5-(4-pyridyl)-1*H-*pyrid-2-one;
2- (3-phénylpropoxy) -4- (3-méthylphényl)-5-(4-pyridyl)pyridine;
1-((S)-2-amino-3-phénylpropyl)-4-(3-méthylphényl)-5-(4-pyridyl)-1*H*-pyrid-2-one;
2- ((S)-2-amino-3-phénylpropoxy) -4- (3-méthylphényl) -5-(4-pyridyl)pyridine;
2- (3-amino-3-phénylpropylamino)-5- (4-fluorophényl) -4-(4-pyridinyl)pyridine;
2-(3-amino-3-phénylpropylamino)-5-(4-chlorolphényl)-4-(4-pyridinyl) pyridine;
2-(3-amino-3-phénylpropylamino)-5-(3-fluorophényl)-4-(4-pyridinyl) pyridine;
2-(3-amino-3-phénylpropylamino)-5-(3-trifluorométhylphenyl)-4-(4-pyridinyl)pyridine;
2-(3-amino-3-phénylpropylamino)-5-(3-isopropylphényl)-4-(4-pyridinyl)pyridine;
2-(3-amino-3-phénylpropylamino)-5-(3-méthylphényl)-4-(4-pyridinyl)pyridine;
2-((S)-2-amino-3-phénylpropylamino)-5-(4-fluorophényl)-4-(4-pyridinyl)pyridine;
2-((S)-2-amino-3-phénylpropylamino)-5-(4-chlorophényl)-4-(4-pyridinyl)pyridine;
2-((S)-2-amino-3-phénylpropylamino)-5-(3-fluorophényl-4-(4-pyridinyl)pyridine;
2-((S)-2-amino-3-phénylpropylamino)-5-(3-trifluorométhylphenyl-4-(4-pyridinyl)pyridine;
2-((S)-2-amino-3-phénylpropylamino)-5-(3-isopropylphényl)-4-(4-pyridinyl)pyridine;
6-[((S)-2-amino-3-phénylpropyl)-amino]-3-(4-fluorophényl)-4-(4-pyridyl)-pyridazine;
6- ((S)-2-Amino-3-phenylpropylamino)-3-(2-benzothiophényl)-2-(4-pyridyl)pyridine;
6- ((S)-2-Amino-3-phénylpropylamino)-3-(4-chloro-3-fluorophényl)-2-(4-pyridyl)pyridine;
6-((S)-2-Amino-3-phénylpropylamino)-3-(4-methoxyphényl) -2- (4-pyridyl) pyridine ;
6-((S)-2-Amino-3-phénylpropylamino)-3-(3-isopropylphényl) -2-(4-pyridyl)pyridine ;
6-((S)-2-Amino-3-phénylpropylamino)-3-(4-chlorophényl)-2-(4-pyridyl)pyridine ;
6-((S)-2-Amino-3-phénylpropylamino)-3-(2-napthyl)-2-(4-pyridyl)pyridine;
6-((S)-2-Amino-3-phénylpropylamino)-3-(3-trifluorométhylphenyl)-2-(4-pyridyl)pyridine;
6-((S)-2-Amino-3-phénylpropylamino)-3-(3-méthylphényl)-2-(4-pyridyl)pyridine;
ou un sel pharmaceutiquement acceptable de celui-ci.

10. Composé de la revendication 1, dans lequel R₃ est -Z-Y, chaque Z est un radical hétérocyclyle éventuellement substitué par 1-2 radicaux de radicaux C₁-C₂ alkyle ou aryl-C₁-C₂ alkyle; dans lesquels les radicaux aryle sont éventuellement substitués par 1-2 radicaux de radicaux amino, di-(C₁-C₂ alkyl)amino, hydroxy, C₁-C₂ alkoxy, C₁-C₂ alkylthio, cyano, halo, C₁-C₂ alkyle ou trifluorométhyle.

11. Composé de la revendication 1, dans lequel W est R₁ ou R₂.

12. Composé de la revendication 1, dans lequel A est R₁₁ et Q est R₁₂.

13. Composé de la revendication 1, dans lequel A est R₁₂ et Q est R₁₁ dans lequel R₁₁ est un radical aryle ou hétéroaryle autre qu'un radical « N »-hétéroaryle, et R₁₂ est un radical « N »-hétéroaryle, dans lequel les radicaux aryle et hétéroaryle sont éventuellement substitués par 1-2 radicaux de
(1)R₃₀
(2) radicaux halo ou cyano ; ou
(3) radicaux -C(O)-NR₃₁R₃₂,-OR₂₉,-SR₂₉,-S(O)-R₃₀, -S(O)₂-R₃₀, -S(O)₂-NR₃₁R₃₂, -NR₃₁R₃₂ ou -NR₃₃-C(O)-R₂₉.

14. Composé de la revendication 1, dans lequel A est R₁₂ et Q est R₁₁ dans lequel R₁₁ est un radical hétéroaryle autre qu'un radical « N »-hétéroaryle, et R₁₂ est un radical « N »-hétéroaryle, dans lequel les radicaux « N »-hétéroaryle sont éventuellement substitués par 1-2 radicaux de
(1) R₃₀
(2) radicaux halo ou cyano ; ou
(3) radicaux -C(O)-NR₃₁R₃₂,-OH₂₉, -SR_{29'}-S(O)-R₃₀,-S(O)₂-R_{30'}-S(O)₂-NR₃₁R₃₂, -NR₃₁R₃₂ ou -NR₃₃-C(O)-R₂₉; ou
R₁₁ est un radical aryle substitué par 1-2 radicaux de
(1) R₃₀
(2) radicaux cyano ; ou
(3) radicaux -C(O)-NR₃₁R₃₂,-OR₂₉,-SR₂₉,-S(O)-R₃₀,-S(O)₂-R₃₀,-S(O)₂-NR₃₁R₃₂, -NR₃₁R₃₂ ou -NR₃₃-C(O)-R₂₉.

15. Composé de la revendication 1, dans lequel X est C-H.

16. Composition pharmaceutique comprenant un composé des revendications 1 à 15 et un support pharmaceutiquement acceptable.

17. Composé selon l'une quelconque des revendications 1 à 15 pour une utilisation dans un procédé de prophylaxie ou de traitement d'inflammation, le procédé consistant à administrer une quantité efficace du composé.

18. Composition de la revendication 16 pour une utilisation dans un procédé de prophylaxie ou de traitement d'inflammation.

19. Composé des revendications 1 à 15 pour une utilisation dans un procédé de prophylaxie ou de traitement de rhumatisme articulaire, de la maladie de Pagets, d'ostéoporose, de myélome multiple, d'uvéite, de leucémie myélogène aiguë ou chronique, de destruction des cellules pancréatiques β, d'ostéoarthrite, de spondylarthrite ankylosante, d'arthrite goutteuse, de maladie intestinale inflammatoire, du syndrome de détresse respiratoire adulte (ARDS), de psoriasis, de la maladie de Crohn, de rhinite allergique, de colite ulcéreuse, d'anaphylaxie, de dermatite de contact, d'asthme, de dégénérescence musculaire, de cachexie, du syndrome de Reiter, du diabète du type I, du diabète du type II, de maladies de résorption osseuse, de réaction greffon/hôte, de la maladie d'Alzheimer, d'apoplexie, d'infarctus du myocarde, de lésion de reperfusion ischémique, d'athérosclérose, de trauma cérébral, de sclérose multiple, de malaria cérébrale, de septicémie, de choc septique, de syndrome de choc toxique, de fièvre, de myalgies dues à HIV-1, HIV-2, HIV-3, de cytomégalovirus (CMV), de la grippe, de l'adénovirus, des virus herpétiques ou du zona chez un mammifère.

20. Composition de la revendication 16 pour une utilisation dans un procédé de prophylaxie ou de traitement de rhumatisme articulaire, de la maladie de Pagets, d'ostéoporose, de myélome multiple, d'uvéite, de leucémie myélogène aiguë ou chronique, de destruction des cellules pancréatiques β, d'ostéoarthrite, de spondylarthrite ankylosante, d'arthrite goutteuse, de maladie intestinale inflammatoire, du syndrome de détresse respiratoire adulte (ARDS), de psoriasis, de la maladie de Crohn, de rhinite allergique, de colite ulcéreuse, d'anaphylaxie, de dermatite de contact, d'asthme, de dégénérescence musculaire, de cachexie, du syndrome de Reiter, du diabète du type I, du diabète du type II, de maladies de résorption osseuse, de réaction greffon/hôte, de la maladie d'Alzheimer, d'apoplexie, d'infarctus du myocarde, de lésion de reperfusion ischémique, d'athérosclérose, de trauma cérébral, de sclérose multiple, de malaria cérébrale, de septicémie, de choc septique, de syndrome de choc toxique, de fièvre, de myalgies dues à HIV-1, HIV-2, HIV-3, de cytomégalovirus (CMV), de la grippe, de l'adénovirus, des virus herpétiques ou du zona chez un mammifère.

21. Composé des revendications 1 à 15 pour une utilisation dans un procédé d'abaissement des concentrations de plasma de soit TNF-a ou IL-1 ou des deux.

22. Composition de la revendication 16 pour une utilisation dans un procédé d'abaissement des concentrations de plasma de soit TNF-a ou IL-1 ou des deux.

23. Composé des revendications 1 à 15 pour une utilisation dans un procédé d'abaissement des concentrations de plasma de soit IL-6 ou IL-8 ou des deux.

24. Composition de la revendication 16 pour une utilisation dans un procédé d'abaissement des concentrations de plasma de soit IL-6-ou IL-8 ou des deux.

25. Composé des revendications 1 à 15 pour une utilisation dans un procédé de prophylaxie ou de traitement de maladies diabétiques chez un mammifère, comprenant l'administration d'une quantité efficace du composé pour produire un effet antagoniste au glucagon.

26. Composition de la revendication 16 pour une utilisation dans un procédé de prophylaxie ou de traitement de maladies diabétiques chez un mammifère, comprenant l'administration d'une quantité efficace de la composition pharmaceutique pour produire un effet antagoniste au glucagon.

27. Composé selon les revendications 1 à 15 pour une utilisation dans un procédé de prophylaxie ou de traitement de trouble de la douleur chez un mammifère.

28. Composition pharmaceutique selon la revendication 16 pour une utilisation dans un procédé de prophylaxie ou de traitement de trouble de la douleur chez un mammifère.

29. Composé selon les revendications 1 à 15 pour une utilisation dans un procédé de diminution de la production de prostaglandine chez un mammifère.

30. Composition pharmaceutique selon la revendication 16 pour une utilisation dans un procédé de diminution de la production de prostaglandine chez un mammifère.

31. Composé selon les revendications 1 à 15 pour une utilisation dans un procédé de diminution de l'activité enzymatique de cyclo-oxygénase chez un mammifère.

32. Composé de la revendication 31 dans lequel l'enzyme cyclo-oxygénase est COX-2.

33. Composition pharmaceutique selon la revendication 16 pour une utilisation dans un procédé de diminution de l'activité enzymatique de cyclo-oxygénase chez un mammifère.

34. Composition selon la revendication 33, dans laquelle l'enzyme cyclo-oxygénase est COX-2.

35. Composé selon les revendications 1 à 15 pour une utilisation dans un procédé de prophylaxie ou de traitement du cancer chez un mammifère.

36. Composé selon la revendication 35, dans lequel le cancer a pour origine des protéines Raf et pouvant être induites par Raf.

37. Composé selon la revendication 35, dans lequel le cancer est le cancer du pancréas, le cancer du sein, le cancer du cerveau, le cancer du larynx, le cancer des poumons, le cancer du système lymphatique, le cancer de la voie urinaire ou le cancer de l'estomac.

38. Composition pharmaceutique selon la revendication 16 pour une utilisation dans un procédé de prophylaxie ou de traitement du cancer chez un mammifère.

39. Composition de la revendication 38, dans laquelle le cancer a pour origine des protéines Raf et pouvant être induites par Raf.

40. Composition de la revendication 38, dans laquelle le cancer est le cancer du pancréas, le cancer du sein, le cancer du cerveau, le cancer du larynx, le cancer des poumons, le cancer du système lymphatique, le cancer de la voie urinaire ou le cancer de l'estomac.

41. Utilisation d'un composé des revendications 1 à 15 pour la préparation d'une composition pour une utilisation dans le traitement ou la prophylaxie de maladies diabétiques.

42. Utilisation d'un composé des revendications 1 à 15 pour la préparation d'une composition pour une utilisation dans le traitement de rhumatisme articulaire, d'ostéoarthrite, de spondylarthrite ankylosante, d'arthrite goutteuse, de maladie intestinale inflammatoire, du syndrome de détresse respiratoire adulte (ARDS), de psoriasis, de la maladie de Crohn, de rhinite allergique, de colite ulcéreuse, d'anaphylaxie, de dermatite de contact, d'asthme, d'infections HIV, d'infections par cytomégalovirus (CMV), de grippe, d'infections par adénovirus, d'infections herpétiques, de zonas, de dégénérescence musculaire, de cachexie, du syndrome de Reiter, du diabète du type II, de maladies de résorption osseuse, de réaction greffon/hôte, de lésion de reperfusion ischémique, d'athérosclérose, de trauma cérébral, de la maladie d'Alzheimer, de sclérose multiple, de malaria cérébrale, de septicémie, de choc septique, de syndrome de choc toxique, ou de fièvre ou myalgies dues à l'infection.

43. Utilisation d'un composé des revendications 1 à 15 pour la préparation d'une composition pour une utilisation dans le traitement du cancer du pancréas, du cancer du sein, du cancer du cerveau, du cancer du larynx, du cancer- des poumons, du cancer du système lymphatique, du cancer de la voie urinaire ou du cancer de l'estomac.

44. Utilisation d'un -composé des revendications 1 à 15 pour la préparation d'une composition pour une utilisation dans l'abaissement des concentrations de plasma de TNF-α ou IL-1.

45. Utilisation d'un composé des revendications 1 à 15 pour la préparation d'une composition pour une utilisation dans la diminution de la production de prostaglandine chez un mammifère.

46. Utilisation d'un composé des revendications 1 à 15 pour la préparation d'une composition pour une utilisation dans la diminution de l'activité enzymatique de cyclo-oxygénase chez un mammifère.
